(19) **Europäisches Patentamt European Patent Office Office européen des brevets**

(11) **EP 1 567 540 B1**

(12) **EUROPEAN PATENT SPECIFICATION**

(45) Date of publication and mention of the grant of the patent:
**03.11.2010 Bulletin 2010/44**

(51) Int Cl.:
**C07H 21/04** *(2006.01)*   **C12Q 1/68** *(2006.01)*

(21) Application number: **03783563.4**

(22) Date of filing: **14.11.2003**

(86) International application number:
**PCT/US2003/036611**

(87) International publication number:
**WO 2004/046688 (03.06.2004 Gazette 2004/23)**

(54) **CFTR ALLELE DETECTION ASSAYS**

ASSAYS ZUM NACHWEIS VON CFTR-ALLELEN

DOSAGES DE DETECTION D'ALLELES DE CFTR

(84) Designated Contracting States:
**AT BE BG CH CY CZ DE DK EE ES FI FR GB GR HU IE IT LI LU MC NL PT RO SE SI SK TR**

(30) Priority: **14.11.2002 US 426144 P**
**21.02.2003 US 371913**
**26.06.2003 US 606577**
**21.07.2003 US 489095 P**
**25.08.2003 US 497644 P**
**28.10.2003 US 515175 P**
**14.11.2003 US 713653**

(43) Date of publication of application:
**31.08.2005 Bulletin 2005/35**

(73) Proprietor: **THIRD WAVE TECHNOLOGIES, INC.**
**Madison, WI 53719 (US)**

(72) Inventors:
• **ACCOLA, Molly A.**
**Madison, WI 53703-4502 (US)**
• **WIGDAL, Susan, S.**
**Madison, WI 53711 (US)**
• **MAST, Andrea, L.**
**Oregon, WI 53575 (US)**
• **BARTHOLOMAY, Christian, T.**
**Madison, WI 53711 (US)**
• **KWIATKOWSKI, Robert, W., Jr.**
**Verona, WI 53593 (US)**
• **TEVERE, Vincent**
**Flemington, NJ 08822 (US)**
• **IP, Hon, S.**
**Madison, WI 53711 (US)**
• **CARROLL, Kathleen**
**Verona, WI 53593 (US)**
• **PETERSON, Patrick**
**Madison, WI 53711 (US)**

• **AGARWAL, Poonam**
**Madison, WI 53717 (US)**
• **JARVIS, Nancy**
**Madison, WI 53704 (US)**
• **HALL, Jeff, G.**
**Madison, WI 53719 (US)**
• **HEISLER, Laura**
**Madison, WI 53703 (US)**

(74) Representative: **Glawe, Delfs, Moll**
**Patent- und Rechtsanwälte**
**Rothenbaumchaussee 58**
**20148 Hamburg (DE)**

(56) References cited:
**WO-A-02/44994      US-A- 5 849 483**
**US-A- 5 981 178**

• **LYAMICHEV V ET AL: "Polymorphism identification and quantitative detection of genomic DNA by invasive cleavage of oligonucleotide probes" NATURE BIOTECHNOLOGY, NATURE PUBLISHING GROUP, NEW YORK, NY, US, vol. 17, no. 3, March 1999 (1999-03), pages 292-296, XP002121762 ISSN: 1087-0156**
• **KWIATKOWSKI ET AL: "Clinical, genetic, and pharmacogenetic applications of the invader assay" MOLECULAR DIAGNOSIS, NAPERVILLE, IL, US, vol. 4, no. 4, December 1999 (1999-12), pages 353-364, XP005533505 ISSN: 1084-8592**
• **HIGHSMITH W EDWARD JR ET AL: "Identification of a splice site mutation (2789 + 5 G greater than A) associated with small amounts of normal CFTR mRNA and mild cystic fibrosis" HUMAN MUTATION, vol. 9, no. 4, 1997, pages 332-338, XP002464290 ISSN: 1059-7794**

EP 1 567 540 B1

- DATABASE BIOSIS [Online] BIOSCIENCES INFORMATION SERVICE, PHILADELPHIA, PA, US; 1993, FEREC CLAUDE ET AL: "Genotype analysis of adult cystic fibrosis patients" XP002464292 Database accession no. PREV199497327837 & HUMAN MOLECULAR GENETICS, vol. 2, no. 10, 1993, pages 1557-1560, ISSN: 0964-6906

- WALL JEFF ET AL: "A 31-mutation assay for cystic fibrosis testing in the clinical molecular diagnostics laboratory" HUMAN MUTATION, vol. 5, no. 4, 1995, pages 333-338, XP002464291 ISSN: 1059-7794

**Description**

**FIELD OF THE INVENTION**

The present invention relates to methods for the detection and characterization of mutations associated with cystic fibrosis. More particularly, the present invention relates to methods for using invasive cleavage structure assays (e.g. the INVADER assay) to screen nucleic acid samples, *e.g.*, from patients, for the presence of any one of a collection of mutations in the CFTR gene associated with cystic fibrosis. The present invention also relates to methods for screening sets of CFTR alleles in a single reaction container.

**BACKGROUND OF THE INVENTION**

Cystic fibrosis (CF) is the most predominant lethal autosomal recessive genetic disorder in Caucasians, with affected individuals occurring in approximately 1/3,000 live births; incidence is lower in other ethnic groups (Heim, et al., Genetics in Medicine 3(3):168-176 (2001)). CF disease is associated with high morbidity and reduced life span. Individuals carrying two defective CF chromosomes typically display a panoply of symptoms, including sinopulmonary disease, pancreatic insufficiency, and male infertility. Certain bacterial infections, e.g. Pseudomonas aeruginosa, are typically found only in individuals affected by CF (Raman, et al., Pediatrics 109(1): E13 (2002)). CFTR mutations are implicated in a broad spectrum of diseases such as congenital bilateral absence of the vas deference (CBAVD) (Dumur, et al., Hum Genet 97: 7-10 (1996)), allergic bronchopulmonary aspergillosis, and isolated chronic pancreatitis (Raman, *supra*). Moreover, disease manifestations may be exacerbated in some cases by additional environmental risk factors such as smoking, alcohol consumption, or allergy (Raman, *supra*).

Approximately one in 25 to 30 Caucasians is a CF carrier (Grody, Cutting, et al., Genetics in Medicine 3(2): 149-154 (2001)); however, no noticeable defects or biochemical or physiological alterations can be readily used to ascertain carrier status (Grody and Desnick, Genetics in Medicine 3(2):87-90 (2001)). Determination of carrier status, as well as confirmation of CF disease, may be of value in genetic counseling as well as in early diagnosis to determine treatment and disease management (Grody and Desnick, *supra*). There is currently no cure for the disease, although recent advances in palliative treatments have dramatically improved the quality of life and overall longevity of affected individuals.

Diagnosis of CF has been accomplished using various means since the 1950's and often requires positive results obtained using more than one clinical parameter (Rosenstein and Cutting, Journal of Pediatrics 132(4): 589-595 (1998)). In some cases, definitive diagnosis can remain elusive for years (Rosenstein and Cutting, *supra*). Sweat chloride testing, involving measurement of chloride in sweat following iontophoresis of pilocarpine is a widely used procedure, although there are reports of CF affected individuals with normal sweat chloride levels, even upon repeat testing (LeGrys, Laboratory Medicine 33(1): 55-57 (2002)). Nasal potential difference, involving bioelectrical measurements of the nasal epithelium, is another clinical method that has been used to detect CF in individuals with normal sweat chloride levels (Wilson, et al., Journal of Pediatrics 132 (4): 596-599 (1998)). Immunoreactive trypsinogen (IRT) levels have been used alone as well as in combination with mutational analysis for neonatal analysis (Gregg, et al., Pediatrics 99(6): 819-824 (1997)). Elevated IRT levels are suggestive of CF disease, although the IRT assay alone has low positive predictive value, often requires repeat testing (Gregg, *et al., supra*), and is complicated by age-related declines in IRT values beyond 30 days (Rock, et al., Pediatrics 85(6): 1001-1007 (1990)).

The CFTR gene was first identified in 1989. The gene is located on chromosome 7, includes 27 exons, and spans 250 kb (Kerem, et al., Science 245: 1073-1080 (1989); Riordan, et al., Science 245: 1066-1073 (1989); Rommens, et al., Science 245: 1059-1065 (1989)). The wild type gene and several key mutant variants are described in US Patent Nos: 6,001,588; 5,407,496; 5,981,178; 5,776, 677; as well as WO 01/21833 and EP 0677900 B1. CFTR encodes a chloride ion channel; defect-causing lesions in the gene result in abnormal intracellular chloride levels, leading to thickened mucosal secretions, which in turn affect multiple organ systems. More than 950 mutations have been identified in the cystic fibrosis transmembrane conductance regulator (CFTR) gene ((Cystic Fibrosis Genetic Analysis Consortium (CFGAC) 2002). One mutation, ΔF508, causes the loss of a phenylalanine residue at amino acid 508 in CFTR gene product and accounts for 66% of defective CF chromosomes worldwide (Bobadilla, et al., Human Mutation 19: 575-606 (2002)). The remaining alleles exhibit considerable ethnic and regional heterogeneity (Bobadilla, *et al., supra*) and, in many cases, exhibit poor genotype-phenotype correlations (Grody, Cutting *et al., supra*). Severity of CF disease in individuals affected by more rare mutations is highly variable. In some cases, atypical, moderate, or partial CF disease may be the result of a partially functional CFTR gene product (Noone and Knowles, Respiratory Research 2(6):328-332 (2001)).

The identification of the CFTR gene enabled significant advances in CF diagnosis and carrier screening. However, use of genetics to establish carrier status or the presence of CF disease remains challenging for several reasons. First, the number of exons and the overall size of the CFTR gene complicate analysis. Most methods applied to CF testing rely on PCR to amplify the more than 15 different exons and intronic regions found thus far to contain the most frequently encountered mutations; the amplicons are then tested individually to determine which mutations, if any, are present. Second, the number of mutations identified in the CFTR gene has increased steadily. As recently as 1994, 400 mutations had been identified; that number grew to more than 950 by 2002 ((Cystic Fibrosis Genetic Analysis Consortium (CFGAC)2002) and is likely to continue to increase. The existence of so many distinct alleles complicates the use of a number of standard mutation detection methods such as PCR-RFLP or AS-PCR. Third, many rarely encountered alleles appear to exhibit incomplete penetrance (Grody, Cutting *et al. supra*) and may be associated with heterologous genetic alterations (Raman, *et al., supra*; Rohlfs. et al., Genetics in Medicine 4(5):319-323 (2002)). Fourth, some alleles, such as R117H, produce different phenotypes depending on chromosomal background (Kiesewetter, et al., Nature Genetics 5(3): 274-278 (1993)). Another allele, 3199de16, which produces an in-frame 6-base deletion, appears to be important when present in *cis* with the I148T allele on some chromosomes (Rohlfs, EM., et al. Genetics in Medicine, 4: 319-323 (2002)). Despite these challenges, widespread genetic screening for CF has been recommended for Caucasian and Ashkenazi Jewish couples and made available to other ethnic groups in the U.S. considering pregnancy or already expecting (Grody, Cutting *et al. supra*). The American College of Obstetrics and Gynecology (ACOG), the American College of Medical Genetics (AMCG), and the National Center for Human Genomics Research (NCHGR) of the NIH have together agreed upon an initial panel of 25 mutations commonly found in North America, including AF508, to be used for prenatal and carrier screening in the US (Grody, Cutting *et al. supra*). This panel is more inclusive for mutations affecting certain ethnic groups than some others, particularly Ashkenazi Jews and Caucasians of North European, non-Jewish descent. Nonetheless, the joint committee concluded that all couples seeking to have a child could benefit from screening that would identify, at a minimum, 50-65% of CFTR mutations. Future recommendations will likely expand the core collection of alleles to be screened in order to encompass a greater percentage of the alleles found in other subpopulations.

The case of the most commonly encountered CF allele, ΔF508, presents a particular challenge to nucleic acid-based detection methods. This region contains three polymorphisms that do not cause CF but may interfere with hybridization of wild type probes (Grody, Cutting et al. 2001). These variations result in the following amino acid changes: F508C, I507V and I506V. This situation is complicated by the existence of the CF-causing mutation ΔI507. Many methods applied to CF genotyping rely on the use of reflex tests to distinguish these benign polymorphisms from the CF-causing mutations in codons 507 and 508. Assays that rely primarily on the stringency of annealing of an oligonucleotide to a target sequence, e.g. AS-PCR or SBH can yield false positive or negative results in the presence of such polymorphisms (Fujimura, Northrup et al. 1990).

LYAMICHEV V ET AL: "Polymorphism identification and quantitative detection of genomic DNA by invasive cleavage of oligonucleotide probes" NATURE BIOTECHNOLOGY, NATURE PUBLISHING GROUP, NEW YORK, NY, US, vol. 17, no. 3, March 1999 pages 292-296 discloses the detection of the ΔF508 allele of CFTR by an invader assay.

WO 02/44994 A (THIRD WAVE TECH INC [US]; BROWER AMY [US]; BROW MARY ANN [US]; CRACAUE) 6 June 2002 also relates to the detection of a polymorphism in the CFTR gene. In particular, WO 02/44994 A describes the use of the invader assay to detect the ΔF508 mutation in the CFTR gene in a pooled sample.

What is needed are detection assays that may be applied directly to the analysis of CTFR sequences (e.g. genomic sequences), as well as assays capable of detecting multiple CTFR alleles in a single reaction vessel.

## SUMMARY OF THE INVENTION

The present invention is defined in the claims and provides methods for the detection and characterization of mutations associated with cystic fibrosis. More particularly, the present invention provides methods for using invasive cleavage structure assays (e.g. the INVADER assay) to screen nucleic acid samples, *e.g.*, from patients, for the presence of any one of a collection of mutations in the CFTR gene associated with cystic fibrosis. The present invention also provides methods for screening sets of CFTR alleles in a single reaction container.

In other embodiments, synthetic DNA suitable for use with the methods of the present invention is made using a purified polymerase on multiply-primed genomic DNA, as provided, *e.g.*, in U.S. Patent Nos: 6,291,187, and 6,323,009, and in PCT applications WO 01/88190 and WO 02/00934. In these embodiments, amplification of DNA such as genomic DNA is accomplished using a DNA polymerase, such as the highly processive Φ 29 polymerase (as described, *e.g.*, in US Patent Nos. 5,198,543 and 5,001,050) in combination with exonuclease-resistant random primers, such as hexamers. The method is not limited by the nature of the target nucleic acid. In some embodiments, the target nucleic acid is single stranded or double stranded DNA or RNA. In some embodiments, double stranded nucleic acid is rendered single stranded (e.g., by heat) prior to formation of the cleavage structure. In some embodiments, the source of target nucleic acid comprises a sample containing genomic DNA. Sample include, but are not limited to, blood, saliva, cerebral spinal fluid, pleural fluid, milk, lymph, sputum and semen.

In some embodiments, the target nucleic acid comprises genomic DNA or mRNA. In other embodiments, the target nucleic acid comprises synthetic DNA or RNA. In some preferred embodiments, synthetic DNA or RNA within a sample is created using a purified polymerase. In some preferred embodiments, creation of synthetic DNA using a purified polymerase comprises the use ofPCR. In some preferred embodiments, creation of synthetic DNA comprises use of the methods and compositions for amplification using RNA-DNA composite primers (*e.g.*, as disclosed in U.S. Patent No. 6,251,639). In other preferred embodiments, creation of synthetic DNA using a purified DNA polymerase suitable for use with the methods of the present invention comprises use of rolling circle amplification, (*e.g.*, as in U.S. Pat. Nos. 6,210,884, 6,183,960 and 6,235,502). In other preferred embodiments, creation of synthetic DNA comprises amplification using nucleic acids comprising loop-forming sequences, *e.g.*, as described in U.S. Patent No. 6,410,278.

In still other embodiments, synthetic DNA suitable for use with the methods of the present invention is made by PCR. In some preferred embodiments, multiple PCR reactions are performed in the same reaction vessel to generate targets suitable for use with the methods and compositions of the present invention. In some particularly preferred embodiments, limited PCR cycles are carried out to generate small amounts of multiple targets in a single vessel (described in US Patent Appl. Ser. Nos. 10/321,039, filed December 17, 2002, and 60/511,955, filed October, 16, 2003, and in Example 10, below), either alone, or in combination with an additional assay, such as the INVADER assay. In other embodiments, alternative multiplex PCR approaches such as those described in Makowski, G.S. et al., Ann. Clin. Lab. Sci., (2003) 33: 243-250 are used to generate suitable targets.

In some preferred embodiments, creation of synthetic DNA comprises copying genomic DNA by priming from a plurality of sites on a genomic DNA sample. In some embodiments, priming from a plurality of sites on a genomic DNA sample comprises using short (*e.g.*, fewer than about 8 nucleotides) oligonucleotide primers. In other embodiments, priming from a plurality of sites on a genomic DNA comprises extension of 3' ends in nicked, double-stranded genomic DNA (*i.e.*, where a 3' hydroxyl group has been made available for extension by breakage or cleavage of one strand of a double stranded region of DNA). Some examples of making synthetic DNA using a purified polymerase on nicked genomic DNAs, suitable for use with the methods of the present invention, are provided in U.S. Patent Nos. 6,117,634, issued September 12, 2000, and 6,197,557, issued March 6, 2001, and in PCT application WO 98/39485.

The pooled detection assays for detection of mutations in the CFTR gene provided in the present invention may find use in detection assays that include, but are not limited to, enzyme mismatch cleavage methods (e.g., Variagenics, U.S. Pat. Nos. 6,110,684, 5,958,692, 5,851,770); polymerase chain reaction; branched hybridization methods (e.g., Chiron, U.S. Pat. Nos. 5,849,481, 5,710,264, 5,124,246, and 5,624,802); rolling circle replication (e.g., U.S. Pat. Nos. 6,210,884, 6,183,960 and 6,235,502); NASBA (e.g., U.S. Pat. No. 5,409,818); molecular beacon technology (e.g., U.S. Pat. No. 6,150,097); E-sensor technology (Motorola, U.S. Pat. Nos. 6,248,229,6,221,583, 6,013,170, and 6,063,573); cycling probe technology (e.g., U.S. Pat. Nos. 5,403,711, 5,011,769, and 5,660,988); Dade Behring signal amplification methods (e.g., U.S. Pat. Nos. 6,121,001, 6,110,677, 5,914,230, 5,882,867, and 5,792,614); ligase chain reaction (Barnay Proc. Natl. Acad. Sci USA 88, 189-93 (1991)); and sandwich hybridization methods (e.g., U.S. Pat. No. 5,288,609).

In some embodiments, the present invention provides a non-amplified oligonucleotide detection assay configured for detecting at least one CFTR allele. In other embodiments, the non-amplified oligonucleotide detection assay comprises first and second oligonucleotides configured to form an invasive cleavage structure (e.g. an INVADER assay) in combination with a target sequence comprising said at least one CFTR allele. In particular embodiments, the first oligonucleotide comprises a 5' portion and a 3' portion, wherein the 3' portion is configured to hybridize to the target sequence, and wherein the 5' portion is configured to not hybridize to the target sequence. In other embodiments, the second oligonucleotide comprises a 5' portion and a 3' portion, wherein the 5' portion is configured to hybridize to the target sequence, and wherein the 3' portion is configured to not hybridize to the target sequence.

In some preferred embodiments, the present invention provides a non-amplified oligonucleotide detection assay configured for detecting at least one CFTR allele or the corresponding wild-type sequence.

In some embodiments, the at least one CFTR allele is 2789+5G>A, or the wild-type version thereof. In other embodiments, the at least one CFTR allele is 2789+5G>A.

In other embodiments, the present invention provides methods for the generation and analysis of limited cycle, multiplexed amplification of a large collection of CFTR loci. In some embodiments, the collection comprises at least one CFTR allele or the corresponding wild-type sequence. In other embodiments, the collection of CFTR alleles is 2789+5G>A.

In certain embodiments, the oligonucleotide detection assays are selected from sequencing assays, polymerase chain reaction assays, hybridization assays, hybridization assays employing a probe complementary to a mutation, microarray assays, bead array assays, primer extension assays, enzyme mismatch cleavage assays, branched hybridization assays, rolling circle replication assays, NASBA assays, molecular beacon assays, cycling probe assays, ligase chain reaction assays, invasive cleavage structure assays, ARMS assays, and sandwich hybridization assays.

In some embodiments, the present invention provides methods of detecting an allele in the CFTR gene or method for diagnosing cystic fibrosis (or carrier status), comprising; a) providing; i) a sample from a subject; and ii) a composition comprising an oligonucleotide detection assay (e.g. as described herein); and b) contacting said sample with said composition such that the presence or absence of at least one allele in said CFTR gene is determined. In some embodiments, the sample is a blood sample or blood fraction sample (e.g. plasma, serum, red blood cells), mouth swab sample, e.g. buccal cells, cervical swab, stool, saliva sample, or other biological fluid sample from the subject such as pleural fluid, sputum, urine, amnion, cerebrospinal fluid, or sweat.

The present invention further provides a method for detecting a plurality of CFTR alleles, comprising: a) providing a sample comprising CFTR target nucleic acid; b) amplifying the CFTR target nucleic acid with 25 (e.g., 24, 23, 22, 21, 20, ...) cycles or fewer of a polymerase chain reaction to generate amplified target nucleic acid; and c) exposing the amplified target nucleic acid to a plurality of detection assays configured to detect a plurality of CFTR alleles under conditions such that the presence or absence of said CFTR alleles is detected. In some embodiments, the plurality of CFTR alleles comprise twenty or more (e.g., 21, 22, 23, 24, ...) different CFTR alleles. In some embodiments, the PCR is conducted within a single reaction vessel. In some preferred embodiments, the amplifying and exposing are conducted simultaneously. In preferred embodiments, the assays comprise invasive cleavage assays.

In particular, the invention provides a method for determining the presence or absence of mutations and polymorphisms at a plurality of loci in CFTR target nucleic acid comprising the 2789+5G>A allele, comprising:

a) providing a sample comprising CFTR target nucleic acid;

b) amplifying said CFTR target nucleic acid in a single reaction vessel with 17 cycles or fewer of a polymerase chain reaction to generate amplified target DNA, wherein said polymerase chain reaction contains primers pairs for the amplification of at least 20 different CFTR amplification targets suspected of containing mutant or polymorphic loci, and wherein the concentrations of the primers used in the polymerase chain reaction are modulated so that said at least 20 different CFTR amplification targets are amplified with approximately equivalent efficiency; and

c) exposing said amplified target DNA to a plurality of detection assays, wherein said detection assays comprise invasive cleavage assays comprising first and second oligonucleotides configured to form invasive cleavage structures to detect a plurality of CFTR mutations and polymorphisms at loci in said amplified target nucleic acid, under conditions such that the presence or absence of said CFTR mutations and polymorphisms at said loci is determined.

### DEFINITIONS

To facilitate an understanding of the present invention, a number of terms and phrases are defined below:

As used herein, the terms "subject" and "patient" refer to any organisms including plants, microorganisms and animals (e.g., mammals such as dogs, cats, livestock, and humans).

As used herein, the term "INVADER assay reagents" refers to one or more reagents for detecting target sequences, said reagents comprising oligonucleotides capable of forming an invasive cleavage structure in the presence of the target sequence. In some embodiments, the INVADER assay reagents further comprise an agent for detecting the presence of an invasive cleavage structure (e.g., a cleavage agent). In some embodiments, the oligonucleotides comprise first and second oligonucleotides, said first oligonucleotide comprising a 5' portion complementary to a first region of the target nucleic acid and said second oligonucleotide comprising a 3' portion and a 5' portion, said 5' portion complementary to a second region of the target nucleic acid downstream of and contiguous to the first portion. In some embodiments, the 3' portion of the second oligonucleotide comprises a 3' terminal nucleotide not complementary to the target nucleic acid. In preferred embodiments, the 3' portion of the second oligonucleotide consists of a single nucleotide not complementary to the target nucleic acid.

In some embodiments, INVADER assay reagents are configured to detect a target nucleic acid sequence comprising first and second non-contiguous single-stranded regions separated by an intervening region comprising a double-stranded region. In preferred embodiments, the INVADER assay reagents comprise a bridging oligonucleotide capable of binding to said first and second non-contiguous single stranded regions of a target nucleic acid sequence. In particularly preferred embodiments, either or both of said first or said second oligonucleotides of said INVADER assay reagents are bridging oligonucleotides.

In some embodiments, the INVADER assay reagents further comprise a solid support. For example, in some embodiments, the one or more oligonucleotides of the assay reagents (e.g., first and/or second oligonucleotide, whether bridging or non-bridging) is attached to said solid support. In some embodiments, the INVADER assay reagents further comprise a buffer solution. In some preferred embodiments, the buffer solution comprises a source of divalent cations (e.g., $Mn^{2+}$ and/or $Mg^{2+}$ ions). Individual ingredients (e.g., oligonucleotides, enzymes, buffers, target nucleic acids) that collectively make up INVADER assay reagents are termed "INVADER assay reagent components".

In some embodiments, the INVADER assay reagents further comprise a third oligonucleotide complementary to a third portion of the target nucleic acid upstream of the first portion of the first target nucleic acid. In yet other embodiments, the INVADER assay reagents further comprise a target nucleic acid. In some embodiments, the INVADER assay reagents further comprise a second target nucleic acid. In yet other embodiments, the INVADER assay reagents further comprise a third oligonucleotide comprising a 5' portion complementary to a first region of the second target nucleic acid. In some specific embodiments, the 3' portion of the third oligonucleotide is covalently linked to the second target nucleic acid. In other specific embodiments, the second target nucleic acid further comprises a 5' portion, wherein the 5' portion of the second target nucleic acid is the third oligonucleotide. In still other embodiments, the INVADER assay reagents further comprise an ARRESTOR molecule (e.g., ARRESTOR oligonucleotide).

In some preferred embodiments, the INVADER assay reagents further comprise reagents for detecting a nucleic acid cleavage product. In some embodiments, one or more oligonucleotides in the INVADER assay reagents comprise a label. In some preferred embodiments, said first oligonucleotide comprises a label. In other preferred embodiments, said third oligonucleotide comprises a label. In particularly preferred embodiments, the reagents comprise a first and/or a third oligonucleotide labeled with moieties that produce a fluorescence resonance energy transfer (FRET) effect.

In some embodiments one or more the INVADER assay reagents may be provided in a predispensed format (*i.e.*, premeasured for use in a step of the procedure without re-measurement or re-dispensing). In some embodiments, selected INVADER assay reagent components are mixed and predispensed together. In other embodiments, In preferred embodiments, predispensed assay reagent components are predispensed and are provided in a reaction vessel (including but not limited to a reaction tube or a well, as in, e.g., a microtiter plate). In particularly preferred embodiments, predispensed INVADER assay reagent components are dried down (e.g., desiccated or lyophilized) in a reaction vessel.

In some embodiments, the INVADER assay reagents are provided as a kit. As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

In some embodiments, the present invention provides INVADER assay reagent kits comprising one or more of the components necessary for practicing the present invention. For example, the present invention provides kits for storing or delivering the enzymes and/or the reaction components necessary to practice an INVADER assay. The kit may include any and all components necessary or desired for assays including, but not limited to, the reagents themselves, buffers, control reagents (e.g., tissue samples, positive and negative control target oligonucleotides, etc.), solid supports, labels, written and/or pictorial instructions and product information, inhibitors, labeling and/or detection reagents, package environmental controls (e.g., ice, desiccants, etc.), and the like. In some embodiments, the kits provide a sub-set of the required components, wherein it is expected that the user will supply the remaining components. In some embodiments, the kits comprise two or more separate containers wherein each container houses a subset of the components to be delivered. For example, a first container (e.g., box) may contain an enzyme (e.g., structure specific cleavage enzyme in a suitable storage buffer and container), while a second box may contain oligonucleotides (e.g., INVADER oligonucleotides, probe oligonucleotides, control target oligonucleotides, etc.).

The term "label" as used herein refers to any atom or molecule that can be used to provide a detectable (preferably quantifiable) effect, and that can be attached to a nucleic acid or protein. Labels include but are not limited to dyes; radiolabels such as $^{32}$P; binding moieties such as biotin; haptens such as digoxgenin; luminogenic, phosphorescent or fluorogenic moieties; mass tags; and fluorescent dyes alone or in combination with moieties that can suppress or shift emission spectra by fluorescence resonance energy transfer (FRET). Labels may provide signals detectable by fluorescence, radioactivity, colorimetry, gravimetry, X-ray diffraction or absorption, magnetism, enzymatic activity, characteristics of mass or behavior affected by mass (e.g., MALDI time-of-flight mass spectrometry), and the like. A label may be a charged moiety (positive or negative charge) or alternatively, may be charge neutral. Labels can include or consist of nucleic acid or protein sequence, so long as the sequence comprising the label is detectable.

As used herein, the term "distinct" in reference to signals refers to signals that can be differentiated one from another, e.g., by spectral properties such as fluorescence emission wavelength, color, absorbance, mass, size, fluorescence polarization properties, charge, etc., or by capability of interaction with another moiety, such as with a chemical reagent, an enzyme, an antibody, etc.

As used herein, the terms "complementary" or "complementarity" are used in reference to polynucleotides (*i.e.*, a sequence of nucleotides such as an oligonucleotide or a target nucleic acid) related by the base-pairing rules. For example, the sequence " 5'-A-G-T-3'," is complementary to the sequence "3'-T-C-A-5'." Complementarity may be "partial," in which only some of the nucleic acids' bases are matched according to the base pairing rules. Or, there may be "complete" or "total" complementarity between the nucleic acids. The degree of complementarity between nucleic acid strands has significant effects on the efficiency and strength of hybridization between nucleic acid strands. This is of particular importance in amplification reactions, as well as detection methods which depend upon binding between nucleic acids. Either term may also be used in reference to individual nucleotides, especially within the context of polynucleotides. For example, a particular nucleotide within an oligonucleotide may be noted for its complementarity, or lack thereof, to a nucleotide within another nucleic acid strand, in contrast or comparison to the complementarity between the rest of the oligonucleotide and the nucleic acid strand.

The term "homology" and "homologous" refers to a degree of identity. There may be partial homology or complete homology. A partially homologous sequence is one that is less than 100% identical to another sequence.

As used herein, the term "hybridization" is used in reference to the pairing of complementary nucleic acids. Hybridization and the strength of hybridization (*i.e.*, the strength of the association between the nucleic acids) is influenced by such factors as the degree of complementary between the nucleic acids, stringency of the conditions involved, and the $T_m$ of the formed hybrid. "Hybridization" methods involve the annealing of one nucleic acid to another, complementary nucleic acid, *i.e.*, a nucleic acid having a complementary nucleotide sequence. The ability of two polymers of nucleic acid containing complementary sequences to find each other and anneal through base pairing interaction is a well-recognized phenomenon. The initial observations of the "hybridization" process by Marmur and Lane, Proc. Natl. Acad. Sci. USA 46:453 (1960) and Doty et al., Proc. Natl. Acad. Sci. USA 46:461 (1960) have been followed by the refinement of this process into an essential tool of modem biology.

The complement of a nucleic acid sequence as used herein refers to an oligonucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

As used herein, the term "$T_m$" is used in reference to the "melting temperature." The melting temperature is the temperature at which a population of double-stranded nucleic acid molecules becomes half dissociated into single strands. Several equations for calculating the $T_m$ of nucleic acids are well known in the art. As indicated by standard references, a simple estimate of the $T_m$ value may be calculated by the equation: $T_m = 81.5 + 0.41$ (% G + C), when a nucleic acid is in aqueous solution at 1 M NaCl (*see* e.g., Anderson and Young, Quantitative Filter Hybridization, in Nucleic Acid Hybridization (1985). Other references (*e.g.*, Allawi, H.T. & SantaLucia, J., Jr. Thermodynamics and NMR of internal G.T mismatches in DNA. Biochemistry 36, 10581-94 (1997) include more sophisticated computations which take structural and environmental, as well as sequence characteristics into account for the calculation of $T_m$.

The term "gene" refers to a DNA sequence that comprises control and coding sequences necessary for the production of an RNA having a non-coding function (e.g., a ribosomal or transfer RNA), a polypeptide or a precursor. The RNA or polypeptide can be encoded by a full length coding sequence or by any portion of the coding sequence so long as the desired activity or function is retained.

The term "wild-type" refers to a gene or a gene product that has the characteristics of that gene or gene product when isolated from a naturally occurring source. A wild-type gene is that which is most frequently observed in a population and is thus arbitrarily designated the "normal" or wild-type" form of the gene. In contrast, the term "modified" ,"mutant" or "polymorphic" refers to a gene or gene product which displays modifications in sequence and or functional properties (*i.e.*, altered characteristics) when compared to the wild-type gene or gene product. It is noted that naturally-occurring mutants can be isolated; these are identified by the fact that they have altered characteristics when compared to the wild-type gene or gene product.

The term "recombinant DNA vector" as used herein refers to DNA sequences containing a desired heterologous sequence. For example, although the term is not limited to the use of expressed sequences or sequences that encode an expression product, in some embodiments, the heterologous sequence is a coding sequence and appropriate DNA sequences necessary for either the replication of the coding sequence in a host organism, or the expression of the operably linked coding sequence in a particular host organism. DNA sequences necessary for expression in prokaryotes include a promoter, optionally an operator sequence, a ribosome binding site and possibly other sequences. Eukaryotic cells are known to utilize promoters, polyadenlyation signals and enhancers.

The term "oligonucleotide" as used herein is defined as a molecule comprising two or more deoxyribonucleotides or ribonucleotides, preferably at least 5 nucleotides, more preferably at least about 10-15 nucleotides and more preferably at least about 15 to 30 nucleotides. The exact size will depend on many factors, which in turn depend on the ultimate function or use of the oligonucleotide. The oligonucleotide may be generated in any manner, including chemical synthesis, DNA replication, reverse transcription, PCR, or a combination thereof.

Because mononucleotides are reacted to make oligonucleotides in a manner such that the 5' phosphate of one mononucleotide pentose ring is attached to the 3' oxygen of its neighbor in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have 5' and 3' ends. A first region along a nucleic acid strand is said to be upstream of another region if the 3' end of the first region is before the 5' end of the second region when moving along a strand of nucleic acid in a 5' to 3' direction.

When two different, non-overlapping oligonucleotides anneal to different regions of the same linear complementary nucleic acid sequence, and the 3' end of one oligonucleotide points towards the 5' end of the other, the former may be called the "upstream" oligonucleotide and the latter the "downstream" oligonucleotide. Similarly, when two overlapping oligonucleotides are hybridized to the same linear complementary nucleic acid sequence, with the first oligonucleotide positioned such that its 5' end is upstream of the 5' end of the second oligonucleotide, and the 3' end of the first oligonucleotide is upstream of the 3' end of the second oligonucleotide, the first oligonucleotide may be called the "upstream" oligonucleotide and the second oligonucleotide may be called the "downstream" oligonucleotide.

The term "primer" refers to an oligonucleotide that is capable of acting as a point of initiation of synthesis when placed under conditions in which primer extension is initiated. An oligonucleotide "primer" may occur naturally, as in a purified restriction digest or may be produced synthetically.

A primer is selected to be "substantially" complementary to a strand of specific sequence of the template. A primer must be sufficiently complementary to hybridize with a template strand for primer elongation to occur. A primer sequence need not reflect the exact sequence of the template. For example, a non-complementary nucleotide fragment may be attached to the 5' end of the primer, with the remainder of the primer sequence being substantially complementary to the strand. Non-complementary bases or longer sequences can be interspersed into the primer, provided that the primer sequence has sufficient complementarity with the sequence of the template to hybridize and thereby form a template primer complex for synthesis of the extension product of the primer.

The term "cleavage structure" as used herein, refers to a structure that is formed by the interaction of at least one probe oligonucleotide and a target nucleic acid, forming a structure comprising a duplex, the resulting structure being cleavable by a cleavage means, including but not limited to an enzyme. The cleavage structure is a substrate for specific cleavage by the cleavage means in contrast to a nucleic acid molecule that is a substrate for non specific cleavage by agents such as phosphodiesterases which cleave nucleic acid molecules without regard to secondary structure (*i.e.*, no formation of a duplexed structure is required).

The term "cleavage means" or "cleavage agent" as used herein refers to any means that is capable of cleaving a cleavage structure, including but not limited to enzymes. "Structure-specific nucleases" or "structure-specific enzymes" are enzymes that recognize specific secondary structures in a nucleic molecule and cleave these structures. The cleavage means of the invention cleave a nucleic acid molecule in response to the formation of cleavage structures; it is not necessary that the cleavage means cleave the cleavage structure at any particular location within the cleavage structure.

The cleavage means may include nuclease activity provided from a variety of sources including the Cleavase enzymes, the FEN-1 endonucleases (including RAD2 and XPG proteins), *Taq* DNA polymerase and *E. coli* DNA polymerase I. The cleavage means may include enzymes having 5' nuclease activity (*e.g.*, Taq DNA polymerase (DNAP), *E. coli* DNA polymerase I). The cleavage means may also include modified DNA polymerases having 5' nuclease activity but lacking synthetic activity. Examples of cleavage means suitable for use in the method and kits of the present invention are provided in U.S. Patent Nos. 5,614,402; 5,795,763; 5,843,669; 6,090; PCT Appln. Nos WO 98/23774; WO 02/070755A2; and WO0190337A2).

The term "thermostable" when used in reference to an enzyme, such as a 5' nuclease, indicates that the enzyme is functional or active (i.e., can perform catalysis) at an elevated temperature, i.e., at about 55°C or higher.

The term "cleavage products" as used herein, refers to products generated by the reaction of a cleavage means with a cleavage structure (i.e., the treatment of a cleavage structure with a cleavage means).

The term "target nucleic acid" refers to a nucleic acid molecule containing a sequence that has at least partial complementarity with at least a probe oligonucleotide and may also have at least partial complementarity with an INVADER oligonucleotide. The target nucleic acid may comprise single- or double-stranded DNA or RNA.

The term "non-target cleavage product" refers to a product of a cleavage reaction that is not derived from the target nucleic acid. As discussed above, in the methods of the present invention, cleavage of the cleavage structure generally occurs within the probe oligonucleotide. The fragments of the probe oligonucleotide generated by this target nucleic acid-dependent cleavage are "non-target cleavage products."

The term "probe oligonucleotide" refers to an oligonucleotide that interacts with a target nucleic acid to form a cleavage structure in the presence or absence of an INVADER oligonucleotide. When annealed to the target nucleic acid, the probe oligonucleotide and target form a cleavage structure and cleavage occurs within the probe oligonucleotide.

The term "INVADER oligonucleotide" refers to an oligonucleotide that hybridizes to a target nucleic acid at a location near the region of hybridization between a probe and the target nucleic acid, wherein the INVADER oligonucleotide comprises a portion (*e.g.*, a chemical moiety, or nucleotide-whether complementary to that target or not) that overlaps with the region of hybridization between the probe and target. In some embodiments, the INVADER oligonucleotide contains sequences at its 3' end that are substantially the same as sequences located at the 5' end of a probe oligonucleotide.

The term "cassette" as used herein refers to an oligonucleotide or combination of oligonucleotides configured to generate a detectable signal in response to cleavage of a probe oligonucleotide in an INVADER assay. In preferred embodiments, the cassette hybridizes to a non-target cleavage product from cleavage of the probe oligonucleotide to form a second invasive cleavage structure, such that the cassette can then be cleaved.

In some embodiments, the cassette is a single oligonucleotide comprising a hairpin portion (*i.e.,* a region wherein one portion of the cassette oligonucleotide hybridizes to a second portion of the same oligonucleotide under reaction conditions, to form a duplex). In other embodiments, a cassette comprises at least two oligonucleotides comprising complementary portions that can form a duplex under reaction conditions. In preferred embodiments, the cassette comprises a label. In particularly preferred embodiments, cassette comprises labeled moieties that produce a fluorescence resonance energy transfer (FRET) effect.

The term "substantially single-stranded" when used in reference to a nucleic acid substrate means that the substrate molecule exists primarily as a single strand of nucleic acid in contrast to a double-stranded substrate which exists as two strands of nucleic acid which are held together by inter-strand base pairing interactions.

As used herein, the phrase "non-amplified oligonucleotide detection assay" refers to a detection assay configured to detect the presence or absence of a particular polymorphism (e.g., SNP, repeat sequence, etc.) in a target sequence (e.g. genomic DNA) that has not been amplified (e.g. by PCR), without creating copies of the target sequence. A "non-amplified oligonucleotide detection assay" may, for example, amplify a signal used to indicate the presence or absence of a particular polymorphism in a target sequence, so long as the target sequence is not copied.

The term "sequence variation" as used herein refers to differences in nucleic acid sequence between two nucleic acids. For example, a wild-type structural gene and a mutant form of this wild-type structural gene may vary in sequence by the presence of single base substitutions and/or deletions or insertions of one or more nucleotides. These two forms of the structural gene are said to vary in sequence from one another. A second mutant form of the structural gene may exist. This second mutant form is said to vary in sequence from both the wild-type gene and the first mutant form of the gene.

The term "liberating" as used herein refers to the release of a nucleic acid fragment from a larger nucleic acid fragment, such as an oligonucleotide, by the action of, for example, a 5' nuclease such that the released fragment is no longer covalently attached to the remainder of the oligonucleotide.

The term "$K_m$" as used herein refers to the Michaelis-Menten constant for an enzyme and is defined as the concentration of the specific substrate at which a given enzyme yields one-half its maximum velocity in an enzyme catalyzed reaction.

The term "nucleotide analog" as used herein refers to modified or non-naturally occurring nucleotides including but not limited to analogs that have altered stacking interactions such as 7-deaza purines (*i.e.*, 7-deaza-dATP and 7-deaza-dGTP); base analogs with alternative hydrogen bonding configurations (*e.g.*, such as Iso-C and Iso-G and other non-standard base pairs described in U.S. Patent No. 6,001,983 to S. Benner); non-hydrogen bonding analogs (*e.g.*, non-polar, -aromatic nucleoside analogs such as 2,4-difluorotoluene, described by B.A. Schweitzer and E.T. Kool, J. Org. Chem., 1994, 59, 7238-7242, B.A. Schweitzer and E.T. Kool, J. Am. Chem. Soc., 1995, 117, 1863-1872); "universal" bases such as 5-nitroindole and 3-nitropyrrole; and universal purines and pyrimidines (such as "K" and "P" nucleotides, respectively; P. Kong, et al., Nucleic Acids Res., 1989, 17, 10373-10383, P. Kong et al., Nucleic Acids Res., 1992, 20, 5149-5152). Nucleotide analogs include comprise modified forms of deoxyribonucleotides as well as ribonucleotides.

The term "polymorphic locus" is a locus present in a population that shows variation between members of the population (e.g.., the most common allele has a frequency of less than 0.95). In contrast, a "monomorphic locus" is a genetic locus at little or no variations seen between members of the population (generally taken to be a locus at which the most common allele exceeds a frequency of 0.95 in the gene pool of the population).

The term "microorganism" as used herein means an organism too small to be observed with the unaided eye and includes, but is not limited to bacteria, virus, protozoans, fungi, and ciliates.

The term "microbial gene sequences" refers to gene sequences derived from a microorganism.

The term "bacteria" refers to any bacterial species including eubacterial and archaebacterial species.

The term "virus" refers to obligate, ultramicroscopic, intracellular parasites incapable of autonomous replication (i.e., replication requires the use of the host cell's machinery).

The term "multi-drug resistant" or multiple-drug resistant" refers to a microorganism that is resistant to more than one of the antibiotics or antimicrobial agents used in the treatment of said microorganism.

The term "sample" in the present specification and claims is used in its broadest sense. On the one hand it is meant to include a specimen or culture (e.g., microbiological cultures). On the other hand, it is meant to include both biological and environmental samples. A sample may include a specimen of synthetic origin.

Biological samples may be animal, including human, fluid, solid (*e.g.*, stool) or tissue, as well as liquid and solid food and feed products and ingredients such as dairy items, vegetables, meat and meat by-products, and waste. In particular, biological samples may include blood or blood fractions (e.g. plasma, serum, red blood cells), urine, stool, cerebrospinal fluid, pleural fluid, amnion, sputum, buccal swabs, cervical swabs, formalin fixed tissue samples, skin, or tumor tissue. Biological samples may be obtained from all of the various families of domestic animals, as well as feral or wild animals, including, but not limited to, such animals as ungulates, bear, fish, lagomorphs, rodents, etc.

Environmental samples include environmental material such as surface matter, soil, water, air, and industrial samples, as well as samples obtained from food and dairy processing instruments, apparatus, equipment, utensils, disposable and non-disposable items. These examples are not to be construed as limiting the sample types applicable to the present invention.

The term "source of target nucleic acid" refers to any sample that contains nucleic acids (RNA or DNA). Particularly preferred sources of target nucleic acids are biological samples including, but not limited to blood, saliva, cerebral spinal fluid, pleural fluid, milk, lymph, sputum and semen.

An oligonucleotide is said to be present in "excess" relative to another oligonucleotide (or target nucleic acid sequence) if that oligonucleotide is present at a higher molar concentration that the other oligonucleotide (or target nucleic acid sequence). When an oligonucleotide such as a probe oligonucleotide is present in a cleavage reaction in excess relative to the concentration of the complementary target nucleic acid sequence, the reaction may be used to indicate the amount of the target nucleic acid present. Typically, when present in excess, the probe oligonucleotide will be present at at least a 100-fold molar excess; typically at least 1 pmole of each probe oligonucleotide would be used when the target nucleic acid sequence was present at about 10 fmoles or less.

A sample "suspected of containing" a first and a second target nucleic acid may contain either, both or neither target nucleic acid molecule.

The term "reactant" is used herein in its broadest sense. The reactant can comprise, for example, an enzymatic reactant, a chemical reactant or light (e.g., ultraviolet light, particularly short wavelength ultraviolet light is known to break oligonucleotide chains). Any agent capable of reacting with an oligonucleotide to either shorten (i.e., cleave) or elongate the oligonucleotide is encompassed within the term "reactant."

As used herein, the term "purified" or "to purify" refers to the removal of contaminants from a sample. For example, recombinant CLEAVASE nucleases are expressed in bacterial host cells and the nucleases are purified by the removal of host cell proteins; the percent of these recombinant nucleases is thereby increased in the sample.

As used herein the term "portion" when in reference to a protein (as in "a portion of a given protein") refers to fragments of that protein. The fragments may range in size from four amino acid residues to the entire amino acid sequence minus one amino acid (e.g., 4, 5, 6, ..., n-1).

The term "nucleic acid sequence" as used herein refers to an oligonucleotide, nucleotide or polynucleotide, and fragments or portions thereof, and to DNA or RNA of genomic or synthetic origin which may be single or double stranded, and represent the sense or antisense strand. Similarly, "amino acid sequence" as used herein refers to peptide or protein sequence.

As used herein, the terms "purified" or "substantially purified" refer to molecules, either nucleic or amino acid sequences, that are removed from their natural environment, isolated or separated, and are at least 60% free, preferably 75% free, and most preferably 90% free from other components with which they are naturally associated. An "isolated polynucleotide" or "isolated oligonucleotide" is therefore a substantially purified polynucleotide.

The term "continuous strand of nucleic acid" as used herein is means a strand of nucleic acid that has a continuous, covalently linked, backbone structure, without nicks or other disruptions. The disposition of the base portion of each nucleotide, whether base-paired, single-stranded or mismatched, is not an element in the definition of a continuous strand. The backbone of the continuous strand is not limited to the ribose-phosphate or deoxyribose-phosphate compositions that are found in naturally occurring, unmodified nucleic acids. A nucleic acid of the present invention may comprise modifications in the structure of the backbone, including but not limited to phosphorothioate residues, phosphonate residues, 2' substituted ribose residues (*e.g.*, 2'-O-methyl ribose) and alternative sugar (e.g., arabinose) containing residues.

The term "continuous duplex" as used herein refers to a region of double stranded nucleic acid in which there is no disruption in the progression of basepairs within the duplex (i.e., the base pairs along the duplex are not distorted to accommodate a gap, bulge or mismatch with the confines of the region of continuous duplex). As used herein the term refers only to the arrangement of the basepairs within the duplex, without implication of continuity in the backbone portion of the nucleic acid strand. Duplex nucleic acids with uninterrupted basepairing, but with nicks in one or both strands are within the definition of a continuous duplex.

The term "duplex" refers to the state of nucleic acids in which the base portions of the nucleotides on one strand are bound through hydrogen bonding the their complementary bases arrayed on a second strand. The condition of being in a duplex form reflects on the state of the bases of a nucleic acid. By virtue of base pairing, the strands of nucleic acid also generally assume the tertiary structure of a double helix, having a major and a minor groove. The assumption of the helical form is implicit in the act of becoming duplexed.

The term "template" refers to a strand of nucleic acid on which a complementary copy is built from nucleoside triphosphates through the activity of a template-dependent nucleic acid polymerase. Within a duplex the template strand is, by convention, depicted and described as the "bottom" strand. Similarly, the non-template strand is often depicted and described as the "top" strand.

## DESCRIPTION OF THE DRAWINGS

Figure 1 shows a schematic diagram of INVADER oligonucleotides, probe oligonucleotides and FRET cassettes for detecting two different alleles (*e.g.*, differing by a single nucleotide) in a single reaction.

Figure 2 shows a table of invasive cleavage structure assay components (e.g., oligonucleotide INVADER assay components) for use in detecting the indicated mutations or genes. The INVADER assay components may be used as individual sets (*e.g.*, the components used to detect a mutation at an individual locus) or may be grouped as they would be used together in a single pooled or multiplex reaction (See Exemplary Pool column). Examples of such combinations are also described below, *e.g.*, in Examples 1.

Figure 3 shows a table of invasive cleavage structure assay components (e.g. oligonucleotide INVADER assay components) for use in detecting the indicated mutations. The INVADER assay components may be used in monoplex or biplex INVADER assays.

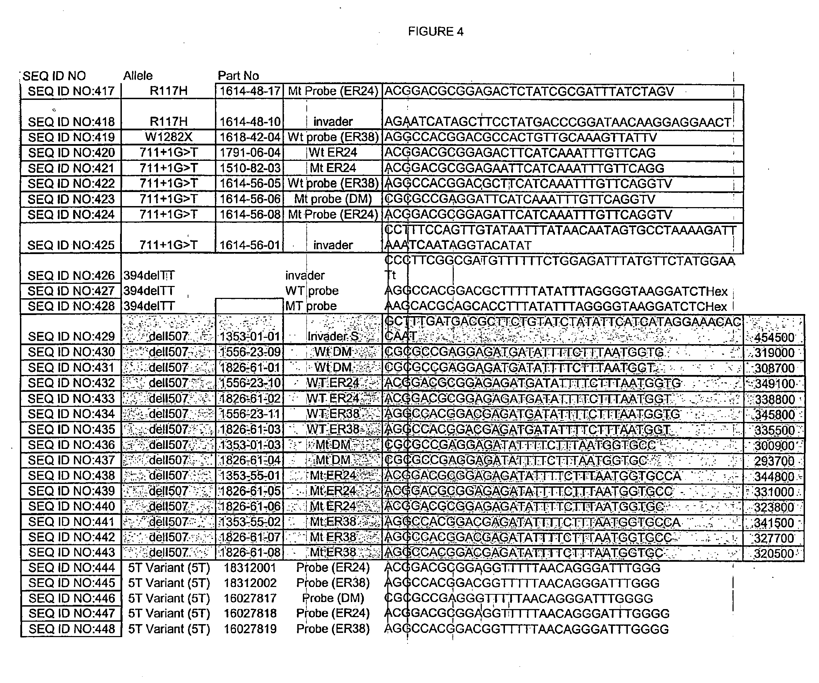

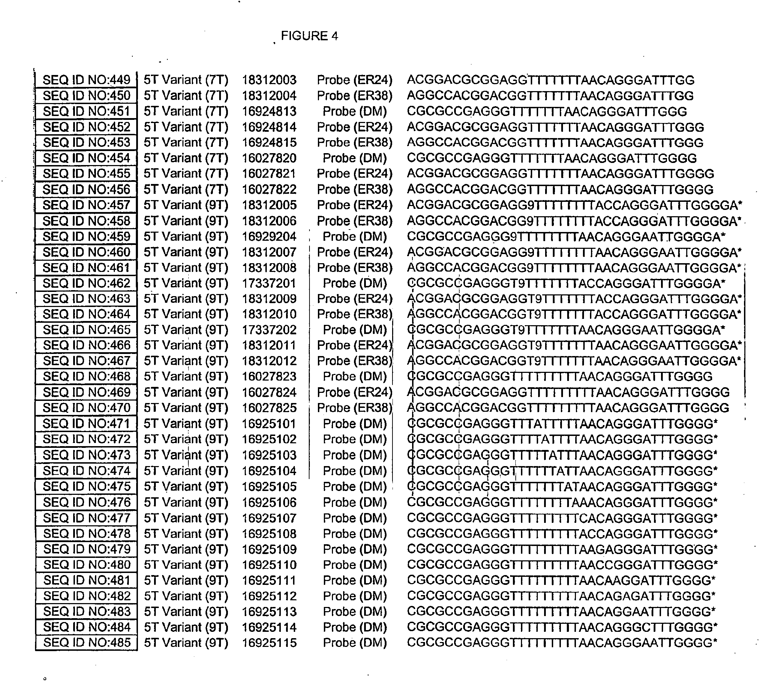

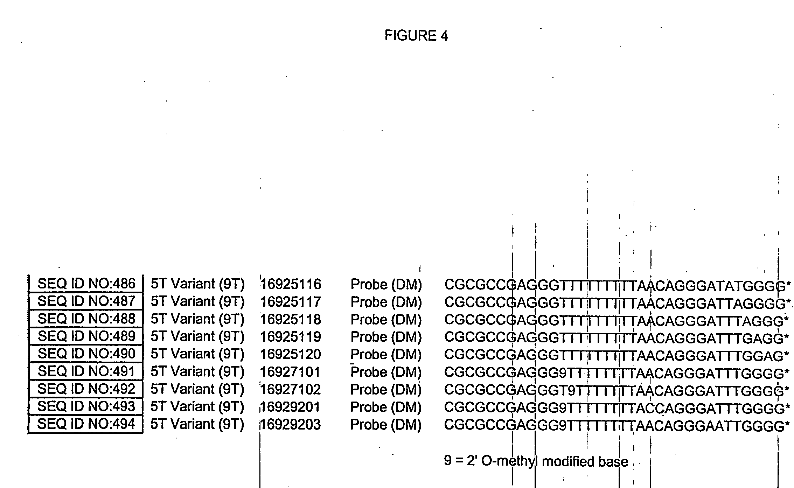

Figure 4 presents additional invasive cleavage structure assay components.

Figure 5 presents primers suitable for use in PCR reactions to amplify portions of the CFTR gene.

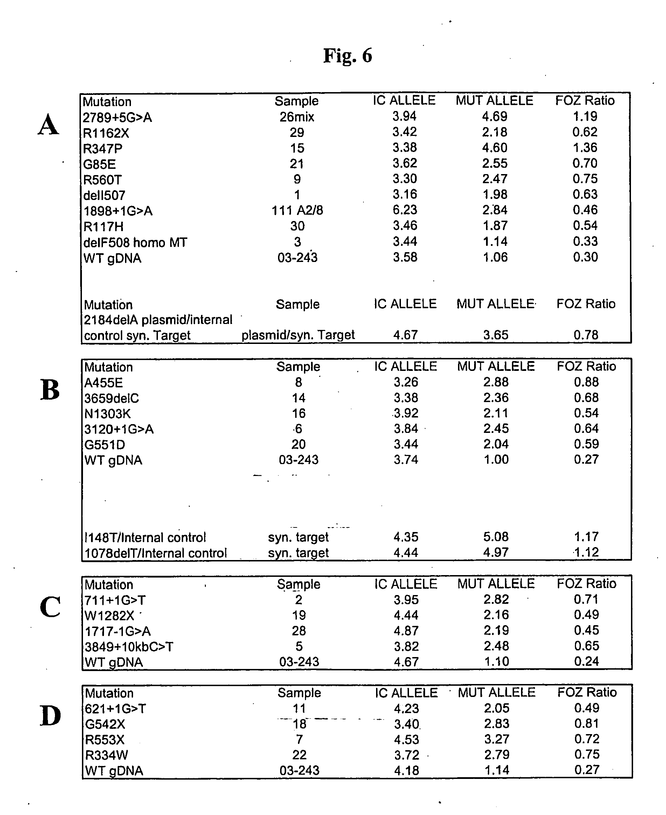

Figure 6 provides an example of data generated using the procedure described in Example 1 in combination with the indicated oligonucleotide INVADER assay reagents, as described herein and as shown in Figure 2.

Figure 7 provides an example of data generated using the procedure described in Example 7 in combination with the indicated oligonucleotide INVADER assay reagents, as described herein and as shown in Figure 2.

Figure 8 presents exemplary data from invasive cleavage assay analysis of the IVS-8 5T/7T/9T polymorphism.

Figure 9 presents exemplary data from invasive cleavage assay experiments carried out on DNA fragments amplified from the CFTR gene.

Figure 10 shows a flow chart outlining the steps that may be performed in order to generated a primer set useful in multiplex PCR.

## DESCRIPTION OF THE INVENTION

The present invention is defined in the claims and provides means for forming a nucleic acid cleavage structure that is dependent upon the presence of a target nucleic acid and cleaving the nucleic acid cleavage structure so as to release distinctive cleavage products. 5' nuclease activity, for example, is used to cleave the target-dependent cleavage structure and the resulting cleavage products are indicative of the presence of specific target nucleic acid sequences in the sample. When two strands of nucleic acid, or oligonucleotides, both hybridize to a target nucleic acid strand such that they form an overlapping invasive cleavage structure, as described below, invasive cleavage can occur. Through the interaction of a cleavage agent (e.g., a 5' nuclease) and the upstream oligonucleotide, the cleavage agent can be made to cleave the downstream oligonucleotide at an internal site in such a way that a distinctive fragment is produced. Such embodiments have been termed the INVADER assay (Third Wave Technologies) and are described in U.S. Patent Appl. Nos. 5,846,717, 5,985,557, 5,994,069,6,001,567, 6,090,543, 6,348,314, and 6,458,535; WO 97/27214 WO 98/42873; and publications including Lyamichev et al., Nat. Biotech., 17:292 (1999), Hall et al., PNAS, USA, 97:8272 (2000).

The INVADER assay detects hybridization of probes to a target by enzymatic cleavage of specific structures by structure specific enzymes (*See,* INVADER assays, Third Wave Technologies; *See e.g.,* U.S. Patent Nos. 5,846,717; 6,090,543; 6,001,567; 5,985,557; 6,090,543; 5,994,069; 6,348,314; 6,458,535; Lyamichev et al., Nat. Biotech., 17:292 (1999), Hall et al., PNAS, USA, 97:8272 (2000), WO97/27214 and WO98/42873).

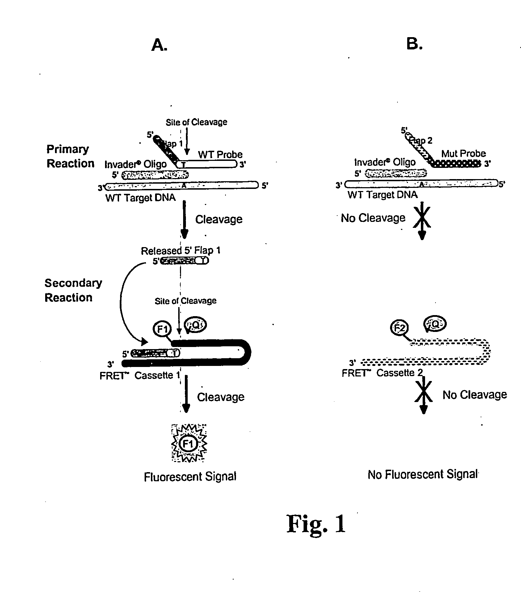

The INVADER assay detects specific DNA and RNA sequences by using structure-specific enzymes (*e.g.* FEN endonucleases) to cleave a complex formed by the hybridization of overlapping oligonucleotide probes (*See, e.g.* Figure 1). Elevated temperature and an excess of one of the probes enable multiple probes to be cleaved for each target sequence present without temperature cycling. In some embodiments, these cleaved probes then direct cleavage of a second labeled probe. The secondary probe oligonucleotide can be 5'-end labeled with fluorescein that is quenched by an internal dye. Upon cleavage, the de-quenched fluorescein labeled product may be detected using a standard fluorescence plate reader.

The INVADER assay detects specific mutations and SNPs in unamplified, as well as amplified, RNA and DNA, including genomic DNA. In the embodiments shown schematically in Figure 1, the INVADER assay uses two cascading steps (a primary and a secondary reaction) both to generate and then to amplify the target-specific signal. For convenience, the alleles in the following discussion are described as wild-type (WT) and mutant (MT), even though this terminology does not apply to all genetic variations. In the primary reaction (Figure 1, panel A), the WT primary probe and the INVADER oligonucleotide hybridize in tandem to the target nucleic acid to form an overlapping structure. An unpaired "flap" is included on the 5' end of the WT primary probe. A structure-specific enzyme (*e.g.* the CLEAVASE enzyme, Third Wave Technologies) recognizes the overlap and cleaves off the unpaired flap, releasing it as a target-specific product. In the secondary reaction, this cleaved product serves as an INVADER oligonucleotide on the WT fluorescence resonance energy transfer (WT-FRET) probe to again create the structure recognized by the structure specific enzyme (panel A). When the two dyes on a single FRET probe are separated by cleavage (indicated by the arrow in Figure 1), a detectable fluorescent signal above background fluorescence is produced. Consequently, cleavage of this second structure results in an increase in fluorescence, indicating the presence of the WT allele (or mutant allele if the assay is configured for the mutant allele to generate the detectable signal). In some embodiments, FRET probes having different labels (*e.g.* resolvable by difference in emission or excitation wavelengths, or resolvable by time-resolved fluorescence detection) are provided for each allele or locus to be detected, such that the different alleles or loci can be detected in a single reaction. In such embodiments, the primary probe sets and the different FRET probes may be combined in a single assay, allowing comparison of the signals from each allele or locus in the same sample.

If the primary probe oligonucleotide and the target nucleotide sequence do not match perfectly at the cleavage site (*e.g.*, as with the MT primary probe and the WT target, Figure 1, panel B), the overlapped structure does not form and cleavage is suppressed. The structure specific enzyme (e.g., CLEAVASE VIII enzyme, Third Wave Technologies) used cleaves the overlapped structure more efficiently (e.g. at least 340-fold) than the non-overlapping structure, allowing excellent discrimination of the alleles.

The probes turn over without temperature cycling to produce many signals per target (*i.e.*, linear signal amplification). Similarly, each target-specific product can enable the cleavage of many FRET probes.

The primary INVADER assay reaction is directed against the target DNA (or RNA) being detected. The target DNA is the limiting component in the first invasive cleavage, since the INVADER and primary probe are supplied in molar excess. In the second invasive cleavage, it is the released flap that is limiting. When these two cleavage reactions are performed sequentially, the fluorescence signal from the composite reaction accumulates linearly with respect to the target DNA amount.

In certain embodiments, the INVADER assay, or other nucleotide detection assays, are performed with accessible site designed oligonucleotides and/or bridging oligonucleotides. Such methods, procedures and compositions are described in U.S. Pat. 6,194,149, WO9850403, and WO0198537.

In certain embodiments, the target nucleic acid sequence is amplified prior to detection (e.g. such that synthetic nucleic acid is generated). In some embodiments, the target nucleic acid comprises genomic DNA. In other embodiments, the target nucleic acid comprises synthetic DNA or RNA. In some preferred embodiments, synthetic DNA within a sample is created using a purified polymerase. In some preferred embodiments, creation of synthetic DNA using a purified polymerase comprises the use of PCR. In some preferred embodiments, PCR amplification is carried out with multiple primer sets to lead to the generation of several target amplification products in a single reaction vessel as described in Makowski et al., Ann. Clin. Lab. Sci. (2003) 33: 243-250. In some embodiments, such amplified products are further characterized using sequence analysis methods including, but not limited to, sequencing, mini-sequencing, allele specific PCR, and pyrosequencing (as described, *e.g.*, U.S. Patent No. 6,258,568). In some preferred embodiments, such multiplex PCR amplification is limited in terms of the number of amplification cycles carried out. In some embodiments, the amplification products produced using limited amplification cycles are detected using nucleic acid detection methods that allow further target amplification, e.g. AS-PCR, TaqMan, TMA, NASBA, LCR. In other particularly preferred embodiments, the products of limited cycle amplification are detected using methods that amplify a target-specific signal, e.g. CPR (*e.g.*, as described in U.S. Patent No. 5,403,711), bDNA (branched DNA), SAT (as described, *e.g.*, in U.S. Patent No. 5,902,724), or the INVADER assay. Other detection methods suitable for use in detecting the products of limited cycle amplification include but are not limited to bead arrays (Illumina, San Diego, CA; *See e.g.,* PCT Publications WO 99/67641 and WO 00/39587), charge or mass tags, (*e.g.*, Aclara ETAG reporters, as described in 6,514,700, and 6,627,400), the SNP-IT primer extension assay (Orchid Biosciences, Princeton, NJ; *See e.g.,* U.S. Patent Nos. 5,952,174 and 5,919,626). Many additional labels, tags, and methods of detecting nucleic acids are well known to those skilled in the art, and such means of product analysis would be readily adaptable by one of skill to analysis of the amplification products of the present invention.

In other preferred embodiments, creation of synthetic DNA using a purified DNA polymerase, suitable for use with the methods of the present invention, comprises use of rolling circle amplification, (*e.g.*, as in U.S. Pat. Nos. 6,210,884, 6,183,960 and 6,235,502). In other preferred embodiments, creation of synthetic DNA comprises copying genomic DNA by priming from a plurality of sites on a genomic DNA sample. In some embodiments, priming from a plurality of sites on a genomic DNA sample comprises using short (*e.g.*, fewer than about 8 nucleotides) oligonucleotide primers. In other embodiments, priming from a plurality of sites on a genomic DNA comprises extension of 3' ends in nicked, double-stranded genomic DNA (*i.e.*, where a 3' hydroxyl group has been made available for extension by breakage or cleavage of one strand of a double stranded region of DNA). Some examples of making synthetic DNA using a purified polymerase on nicked genomic DNAs, suitable for use with the methods and compositions of the present invention, are provided in U.S. Patent Nos. 6,117,634, issued September 12, 2000, and 6,197,557, issued March 6, 2001, and in PCT application WO 98/39485.

In some embodiments, the present invention provides methods for detecting a target sequence, comprising: providing a) a sample containing DNA amplified by extension of 3' ends in nicked double-stranded genomic DNA, said genomic DNA suspected of containing said target sequence; b) oligonucleotides capable of forming an invasive cleavage structure in the presence of said target sequence; and c) exposing the sample to the oligonucleotides and the agent. In some embodiments, the agent comprises a cleavage agent. In some particularly preferred embodiments, the method of the invention further comprises the step of detecting said cleavage product.

In some preferred embodiments, the exposing of the sample to the oligonucleotides and the agent comprises exposing the sample to the oligonucleotides and the agent under conditions wherein an invasive cleavage structure is formed between said target sequence and said oligonucleotides if said target sequence is present in said sample, wherein said invasive cleavage structure is cleaved by said cleavage agent to form a cleavage product.

In some particularly preferred embodiments, the target sequence comprises a first region and a second region, said second region downstream of and contiguous to said first region, and said oligonucleotides comprise first and second oligonucleotides, wherein at least a portion of said first oligonucleotide is completely complementary to said first portion of said target sequence and wherein said second oligonucleotide comprises a 3' portion and a 5' portion, wherein said 5' portion is completely complementary to said second portion of said target nucleic acid.

In other embodiments, synthetic DNA suitable for use with the methods and compositions of the present invention is made using a purified polymerase on multiply-primed genomic DNA, as provided, *e.g.*, in U.S. Patent Nos. 6,291,187, and 6,323,009, and in PCT applications WO 01/88190 and WO 02/00934. In these embodiments, amplification of DNA such as genomic DNA is accomplished using a DNA polymerase, such as the highly processive Φ 29 polymerase (as described, *e.g.*, in US Patent Nos. 5,198,543 and 5,001,050) in combination with exonuclease-resistant random primers, such as hexamers.

In some embodiments, the present invention provides methods for detecting a target sequence, comprising: providing a) a sample containing DNA amplified by extension of multiple primers on genomic DNA, said genomic DNA suspected of containing said target sequence; b) oligonucleotides capable of forming an invasive cleavage structure in the presence of said target sequence; and c) exposing the sample to the oligonucleotides and the agent. In some embodiments, the agent comprises a cleavage agent. In some preferred embodiments, said primers are random primers. In particularly preferred embodiments, said primers are exonuclease resistant. In some particularly preferred embodiments, the method of the invention further comprises the step of detecting said cleavage product.

In some preferred embodiments, the exposing of the sample to the oligonucleotides and the agent comprises exposing the sample to the oligonucleotides and the agent under conditions wherein an invasive cleavage structure is formed between said target sequence and said oligonucleotides if said target sequence is present in said sample, wherein said invasive cleavage structure is cleaved by said cleavage agent to form a cleavage product.

In some preferred embodiments, the exposing of the sample to the oligonucleotides and the agent comprises exposing the sample to the oligonucleotides and the agent under conditions wherein an invasive cleavage structure is formed between said target sequence and said oligonucleotides if said target sequence is present in said sample, wherein said invasive cleavage structure is cleaved by said cleavage agent to form a cleavage product.

In some particularly preferred embodiments, the target sequence comprises a first region and a second region, said second region downstream of and contiguous to said first region, and said oligonucleotides comprise first and second oligonucleotides, said wherein at least a portion of said first oligonucleotide is completely complementary to said first portion of said target sequence and wherein said second oligonucleotide comprises a 3' portion and a 5' portion, wherein said 5' portion is completely complementary to said second portion of said target nucleic acid.

The present invention further provides assays in which the target nucleic acid is reused or recycled during multiple rounds of hybridization with oligonucleotide probes and cleavage of the probes without the need to use temperature cycling (i.e., for periodic denaturation of target nucleic acid strands) or nucleic acid synthesis (i.e., for the polymerization-based displacement of target or probe nucleic acid strands). When a cleavage reaction is run under conditions in which the probes are continuously replaced on the target strand (*e.g.* through probe-probe displacement or through an equilibrium between probe/target association and disassociation, or through a combination comprising these mechanisms, [The kinetics of oligonucleotide replacement. Luis P. Reynaldo, Alexander V. Vologodskii, Bruce P. Neri and Victor I. Lyamichev. J. Mol. Biol. 97: 511-520 (2000)], multiple probes can hybridize to the same target, allowing multiple cleavages, and the generation of multiple cleavage products.

In particular, the invention provides a method for determining the presence or absence of mutations and polymorphisms at a plurality of loci in CFTR target nucleic acid comprising the 2789+5G>A allele, comprising:

a) providing a sample comprising CFTR target nucleic acid;

b) amplifying said CFTR target nucleic acid in a single reaction vessel with 17 cycles or fewer of a polymerase chain reaction to generate amplified target DNA, wherein said polymerase chain reaction contains primers pairs for the amplification of at least 20 different CFTR amplification targets suspected of containing mutant or polymorphic loci, and wherein the concentrations of the primers used in the polymerase chain reaction are modulated so that said at least 20 different CFTR amplification targets are amplified with approximately equivalent efficiency; and

c) exposing said amplified target DNA to a plurality of detection assays, wherein said detection assays comprise invasive cleavage assays comprising first and second oligonucleotides configured to form invasive cleavage structures to detect a plurality of CFTR mutations and polymorphisms at loci in said amplified target nucleic acid, under conditions such that the presence or absence of said CFTR mutations and polymorphisms at said loci is determined.

**The INVADER Assay Reaction:**

In the INVADER DNA Assay, two oligonucleotides (a discriminatory Primary Probe and an INVADER Oligo) hybridize in tandem to the target DNA to form an overlapping structure. The 5'-end of the Primary Probe includes a 5'-flap that does not hybridize to the target DNA (Figure 1). The 3'-nudeotide of the bound INVADER Oligo overlaps the Primary Probe, but need not hybridize to the target DNA. The CLEAVASE enzyme recognizes this overlapping structure and cleaves off the unpaired 5'-flap of the Primary Probe, releasing it as a target-specific product. The Primary Probe is designed to have a melting temperature close to the reaction temperature. Thus, under the isothermal assay conditions, Primary Probes, which are provided in excess, cycle on the target DNA. This allows for multiple rounds of Primary Probe cleavage for each target DNA, and amplification of the number of released 5'-flaps.

In the secondary reaction, each released 5'-flap can serve as an INVADER oligonucleotide on a fluorescence resonance energy transfer (FRET) Cassette to create another overlapping structure that is recognized and cleaved by the CLEAVASE enzyme (Figure 1). When the FRET Cassette is cleaved, the fluorophore (F) and quencher (Q) are separated, generating detectable fluorescence signal. Similar to the initial reaction, the released 5'-flap and the FRET Cassette cycle, resulting in amplified fluorescence signal. The initial and secondary reactions run concurrently in the same well.

The biplex format of the INVADER DNA assay enables simultaneous detection of two DNA sequences in a single well. Most often, this involves detection of two variants of a particular polymorphism. The biplex format uses two different discriminatory Primary Probes, each with a unique 5'-flap, and two different FRET Cassettes, each with a spectrally distinct fluorophore. By design, the released 5'-flaps will bind only to their respective FRET Cassettes to generate a target-specific signal. Kits are not part of the invention.

In some embodiments, the present invention is related to kits comprising one or more of the components necessary for practicing the present invention. For example, the present invention relates to kits for storing or delivering the enzymes of the present invention and/or the reaction components necessary to practice a cleavage assay (e.g., the INVADER assay).

In some reference embodiments, the kits provide the following reagents:

| CLEAVASE enzyme (e.g., CLEAVASE X) | Primary Oligos |
|---|---|
| DNA Reaction Buffer 1 | INVADER Oligo |
| | FRET Cassette 1 (*e.g.,* F) |
| | FRET Cassette 2 (*e.g.*, R) |
| | Mutant DNA controls |
| | Wild type DNA controls |
| | "No Target" Blank control |

In some reference embodiments, the kits provide the following reagents:

| CLEAVASE enzyme mix (e.g., CLEAVASE X) in 140 mM $MgCl_2$, 24% glycerol | Mutation Mixes containing the following constituents in 25 mM MOPS, pH 7.5: |
|---|---|
| | Primary Oligos |
| | INVADER Oligos |

(continued)

FRET Cassette 1 (e.g., F)
FRET Cassette 2 (*e.g.*, a
second F cassette)
FRET Cassette 3 (e.g. R)
Mutant DNA controls
Internal DNA controls
"No Target" Blank control

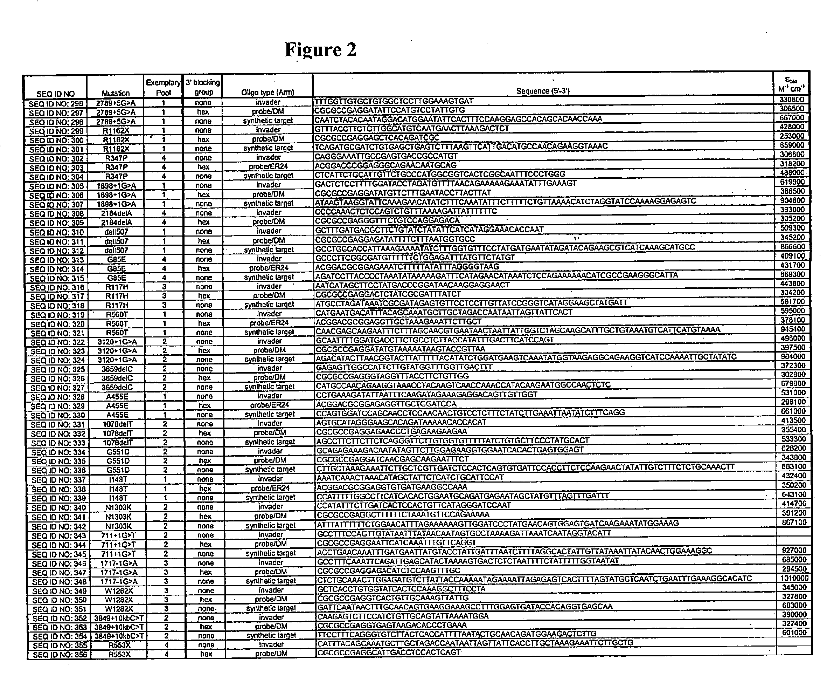

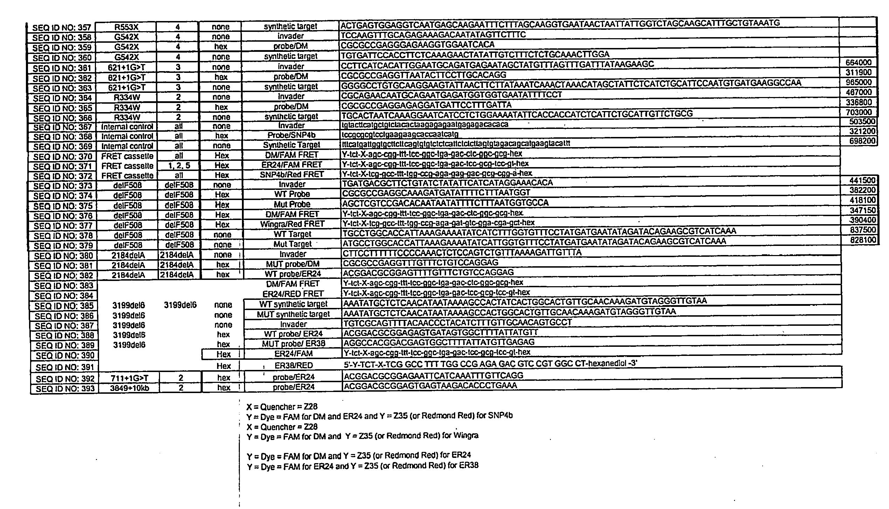

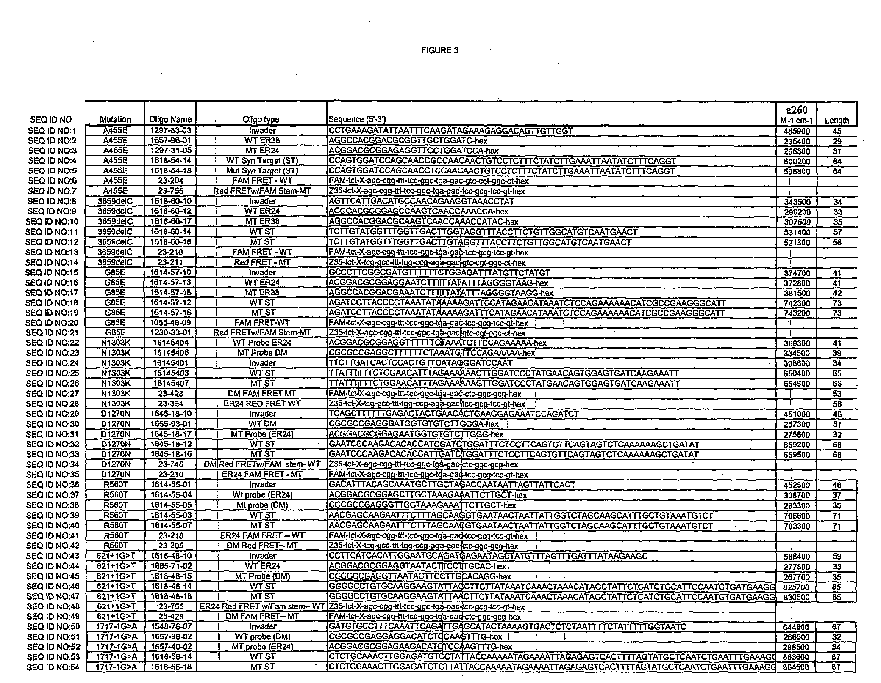

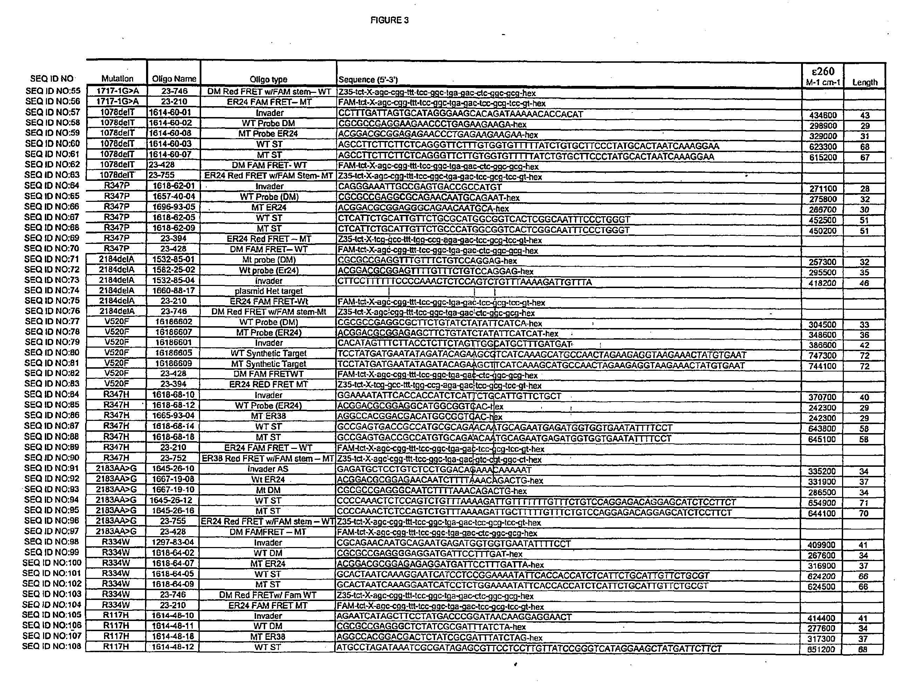

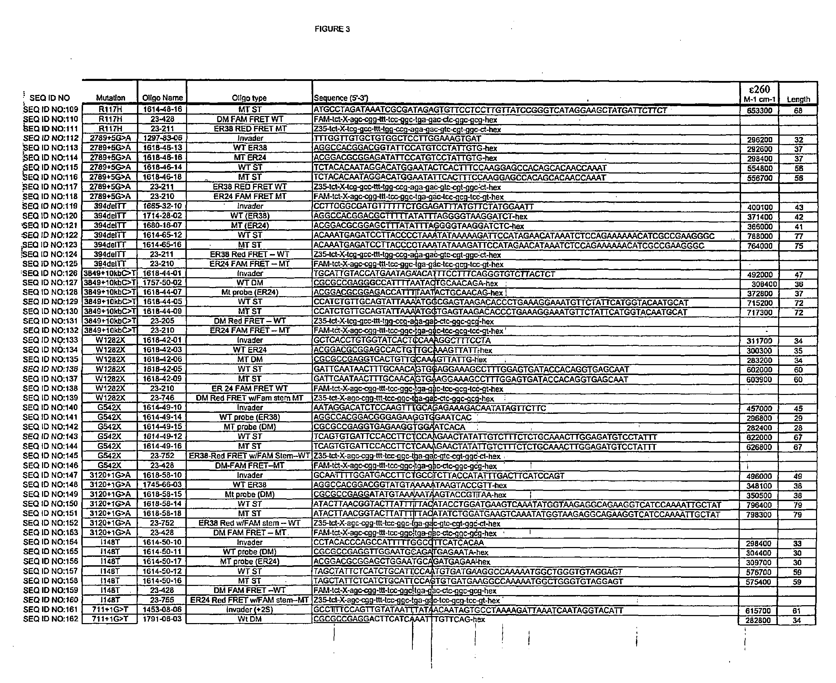

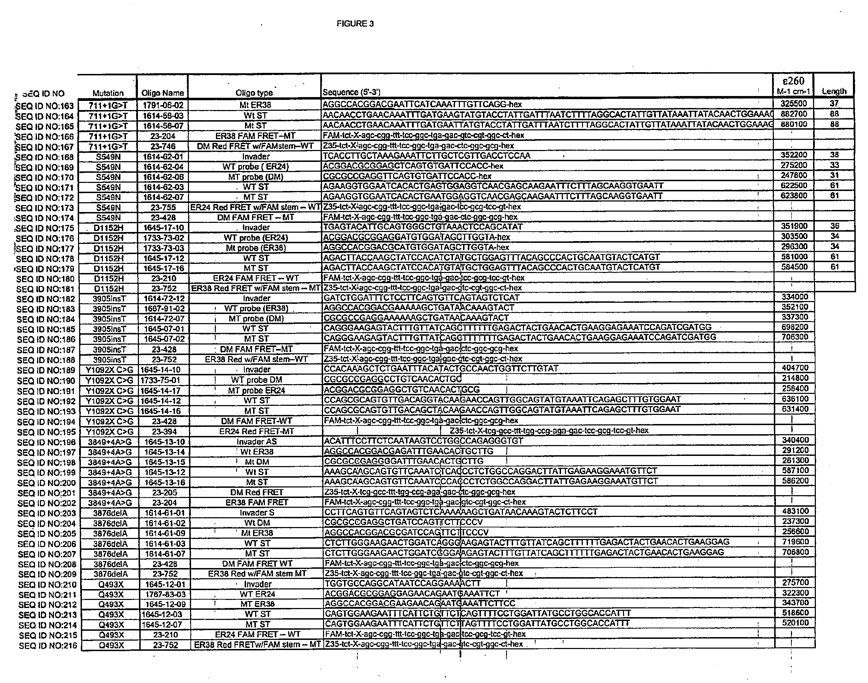

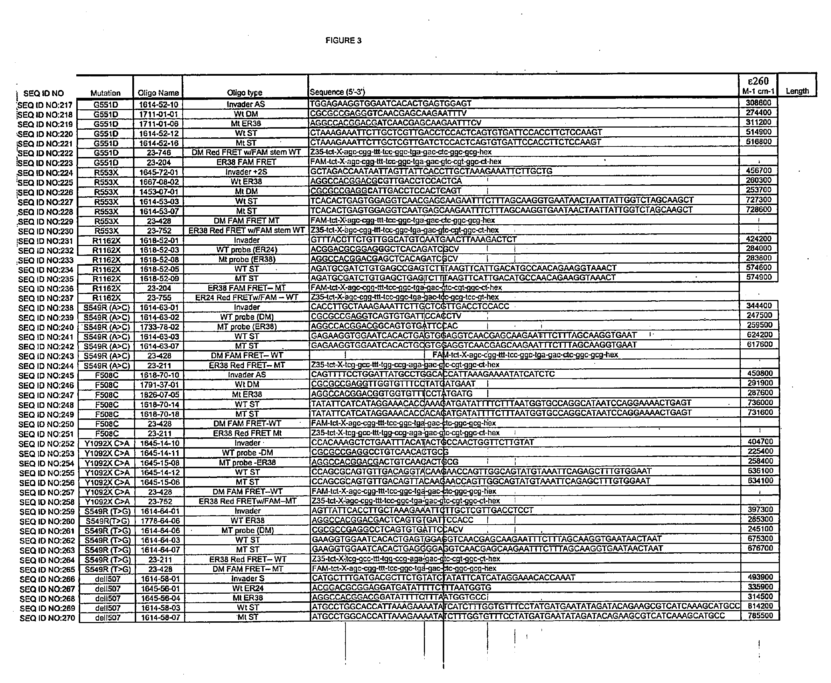

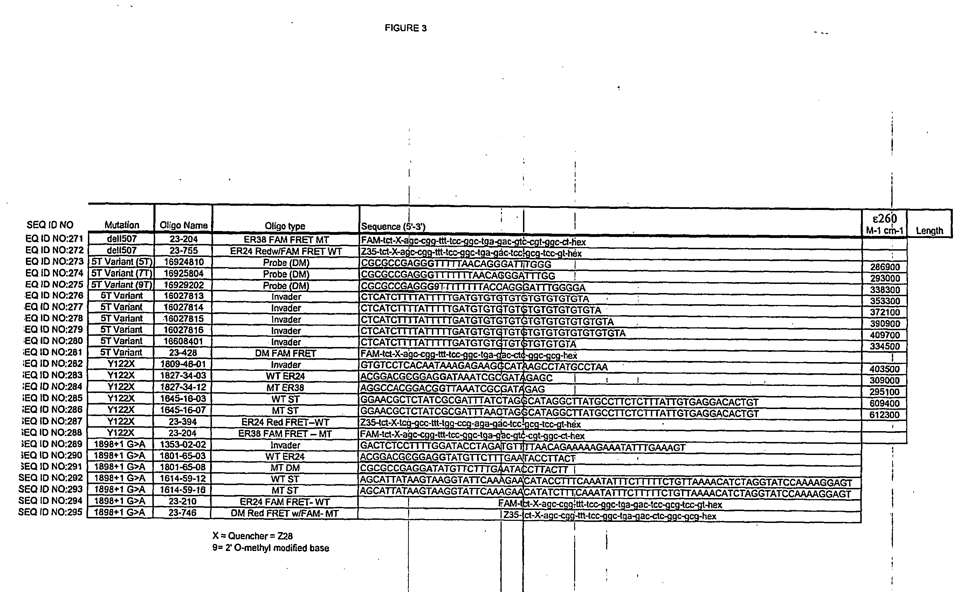

Examples of Primary Oligonucleotides, Secondary Oligonucleotides, and FRET Cassettes suitable for use with the methods the present invention are provided in Figures 2 and 3. While the oligonucleotides shown therein may find use in a number of the methods, and variations of the methods, of the present invention, these INVADER assay oligonucleotide sets find particular use with kits. The oligonucleotide sets shown in Figures 2 and 3 may be used as individual sets to detect individual target DNAs, or may be combined in biplex or multiplex reactions for the detection of two or more analytes or controls in a single reaction.

In preferred embodiments, the oligonucleotides shown in Figures 2 and 3 (or similar oligonucleotides) are used in invasive cleavage structure assays (e.g. INVADER assays) to detect alleles in the CFTR gene. In preferred embodiments, pools or sets of the assay configurations shown in Figures 2 and 3 are used to simultaneously detect a plurality of CFTR alleles (e.g.1-8 CFTR alleles are detected simultaneously in a single reaction container). In this regard, for example, the approximately 25 different alleles shown in Figure 2 could be split into 4-5 pools (as shown) which would only require 4-5 different reaction vessels to detect all of the CFTR alleles shown. In other embodiments, the 25 different alleles shown in figure 2 are split into 5 pools, plus separate SNP detection for ΔF508 which would only require 6 different reaction vessels to detect all of the CFTR alleles shown. In still other embodiments, assays to determine the genotype of at least one CFTR allele can be carried out in a single vessel, and many such assays can be carried out in parallel, e.g. in a multi-well microtiter plate.

Certain design considerations can be used to design pools or sets of CFTR alleles to detect by invasive cleavage structure assays. One consideration that may be used is to avoid physical overlap of oligonucleotides designed to detect closely spaced mutations (this is satisfied by the exemplary pools shown in Figure 2). Another consideration has to do with the signal generation capabilities of the individual invasive cleavage structure assays. For example, often the signal generated from a particular INVADER oligonucleotide and probe pair is higher or lower than that generated from another pair assayed under the same reaction conditions. While in some cases it is feasible and/or desirable to alter oligonucleotide design to modulate such differences in signal generation capabilities, in other cases it may not possible or worthwhile to do so. As such, CFTR mutations can be pooled based on variability in signal generation that dictates that certain pairs be grouped together such that relatively weak signal generating pairs are not overwhelmed by relatively strong signal generating pairs.

An additional consideration has to do with undesired effects resulting from particular combinations of oligonucleotides in a single reaction. One such effect is target-independent generation of background signal. Certain oligonucleotides in combination with others may generate signal in the INVADER assay in the absence of the particular target being detected. Separation of these oligonucleotide combinations into different pools can be used to alleviate this effect. Similarly, certain oligonucleotide combinations can artificially repress signal generation from a desired target. Again, separation of these combinations into different pools can alleviate this effect. It is contemplated that the designs of these probe sets (e.g., the oligonucleotides and/or their sequences) may be adapted for use in RNA detection assays, using the guidelines for reaction design and optimization provided herein.

It is further contemplated that the designs of these probe sets may be used with amplified targets, e.g. PCR amplicons. In some embodiments, PCR amplicons may be generated through multiplexed, limited cycle amplification as described in US Patent Application Ser. Nos: 10/321,039 and 60/511,955, and as described in Example 10, below. In some preferred embodiments, primers for such multiplex PCR amplification are designed as described in Example 10. In some particularly preferred embodiments, such primers are designed to avoid complementarity between the terminal 3 nucleotides, (i.e. of their 3' ends) and to have Tms that are approximately equivalent to one another and be between 20-30 bases long. Such primers may further be designed to generate amplicons of minimal length, i.e. less than approximately 400 base pairs.

In some reference embodiments, a kit provides a list of additional components (*e.g.*, reagents, supplies, and/or equipment) to be supplied by a user in order to perform the methods of the invention. For example, and without intending to limit such additional component lists to any particular components, one embodiment of such a list comprises the following:

■ Clear CHILLOUT-14 liquid wax (MJ Research) or RNase-free, optical grade mineral oil (Sigma, Cat. No. M-5904)

- 96-well polypropylene microplate (MJ Research, Cat. No. MSP-9601)
- Sterile 1.5-ml or 2.0-ml microcentrifuge tubes
- Sterile, DNase/RNase free disposable aerosol barrier pipet tips
- Multichannel pipets (0.5-10μl, 2.5-20μl)
- Thermal cycler or other heat source (*e.g.*, lab oven or heating block).
- Miscellaneous laboratory equipment (tube racks, micropipetors, multichannel pipet, microcentrifuge, vortex mixer).
- Fluorescence microplate reader (a preferred plate reader is top-reading, equipped with light filters have the following characteristics:

| Excitation (Wavelength/Bandwidth) | Emission (Wavelength/Bandwidth) |
|---|---|
| 485 nm/20 nm | 530 nm/25 nm |
| 560 nm/20 nm | 620 nm/40 nm |

In some reference embodiments, a kit provides a list of optional components (e.g., reagents, supplies, and/or equipment) to be supplied by a user to facilitate performance of the methods of the invention. For example, and without intending to limit such optional components lists to any particular components, one embodiment of such a list comprises the following:

- Sterile 8-tube strip or microplate (optional)
- Disposable plastic trough (optional)
- Plate sealing tape (optional)

In some reference embodiments, a kit provides a list of required components to be supplied by a user to facilitate performance of the methods of the invention for which multiple alternatives are acceptable (e.g. sample preparation kits). For example, and without intending to limit such optional components lists to any particular components, one embodiment of such a list comprises the following:

- QIAGEN QIAamp® Blood Kit
- Gentra Systems PUREGENE™ Kit
- Gentra Systems GENERATION® Products

In some reference embodiments of a kit, detailed protocols are provided. In preferred reference embodiments, protocols for the assembly of INVADER assay reactions (*e.g.*, formulations and preferred procedures for making reaction mixtures) are provided. In particularly preferred embodiments, protocols for assembly of reaction mixtures include computational or graphical aids to reduce risk of error in the performance of the methods of the present invention (*e.g.*, tables to facilitate calculation of volumes of reagents needed for multiple reactions, and plate-layout guides to assist in configuring multi-well assay plates to contain numerous assay reactions). By way of example, and without intending to limit such protocols to any particular content or format, kits may comprise the following protocol:

### I. DETAILED DNA BIPLEX INVADER ASSAY PROTOCOL

**1.** Determine the number of samples and controls to be tested.
**2.** Plan the microplate layout for each experimental run (*e.g.*, samples, controls). Inclusion of a No Target Control (tRNA Carrier in buffered, nuclease-free water) is required for a valid result.
**3.** Prepare the INVADER DNA Assay Reaction Mix for the biplex assay format. To calculate the volumes of reaction components needed for the assay (X Volume), multiply the total number of reactions (samples and controls) by 1.25 [X Volume (μl) = # reactions x 1.25]. Vortex the INVADER DNA Assay Reaction Mix briefly after the last reagent addition to mix thoroughly.

### INVADER DNA Assay Reaction Mix

### Biplex Assay Format

| Reaction Components | IX Volume | ___X Volume |
|---|---|---|
| DNA Reaction Buffer 1 | 5.0 μl | |
| FRET F Cassette | 1.0 μl | |
| FRET R Cassette | 1.0 μl | |
| Primary Probes | 1.0 μl | |
| INVADER Oligo | 1.0 μl | |
| CLEAVASE enzyme | 1.0 μl | |
| **Total Mix Volume (IX)** | 10.0 μl | |

4. Add 10μl of each control or DNA sample ( 150 ng DNA) to the appropriate well and mix by pipetting up and down 1-2 times. Overlay each reaction with 20μl of clear CHILLOUT or mineral oil. Seal microplate with Thermaseal well tape (optional).

5. Incubate reactions for 5 minutes at 95°C in a thermal cycler or oven.

6. Lower the temperature to 63 °C in the thermal cycler or transfer the plate to a 63 °C heat block, then add 10 μl of the INVADER® DNA Assay Reaction Mix to each well and mix well by pipetting up and down 3 to 5 times. An 8-tube strip or microplate may be used to facilitate addition of the INVADER® DNA Assay Reaction Mix using a multichannel pipet. When adding the INVADER® DNA Assay Reaction Mix, be sure to add the mix below the level of the mineral oil or Chill-out™ 14 liquid wax.

7. Cover the microplate with plate sealing tape (optional) and incubate at 63 °C for 4 hours.

8. After the 4-hour incubation, place the microplate in the plate holder of the fluorescence plate reader. Remove plate sealing tape, if used.

9. Read the plate at the two different wavelength settings (The dye corresponding to the WT and Mut signal is not necessarily the same for all biplex assays).

10. The gain should be set so that Control 4 reads between 100 and 200 for each scan. The Control 4 values do not have to be identical for the F and R dye scans.

NOTE: Remove the microplate seal before reading the microplate.

■ This procedure enables collection of multiple data sets to extend the assay's dynamic range. During the secondary INVADER reaction, read the microplate directly in a top-reading fluorescence microplate reader.

NOTE: Because the optimal gain setting can vary between instruments, adjust the gain as needed to give the best signal/background ratio (sample raw signal divided by the No Target Control signal) or No Target Control sample readings of ~100 RFUs. Fluorescence microplate readers that use a xenon lamp source generally produce higher RFUs. For directly reading the microplates, the probe height of, and how the plate is positioned in, the fluorescence microplate reader may need to be adjusted according to the manufacturer's recommendations.

In another embodiment, such kits and methods may comprise the following protocol.

## POOL ASSAY PROTOCOL

1. Make up the INVADER DNA reaction mixes according to the following recipe.

| Number of Samples | 4 | | | |
|---|---|---|---|---|
| | ΔF608 | Pool | | |
| Number of Reactions | 8 | 7 | | |
| Add 25% | 2 | 1.75 | | |
| Number of Reactions for Mix Calculations | 10 | 8.75 | | |

(continued)

| CFTR (ΔF508) Reaction Mixes | | | | |
|---|---|---|---|---|
| Component | Lot # | Amount Per Reaction (μl) | Volume to Add (μl) | Component Added (Check off after adding) |
| INVADER Assay Reaction Mix | | | | |
| CFTR (ΔF508) INVADER Oligo (I) | | 2 | 20 | |
| CFTR (ΔF508) Primary Probes (P) | | 2 | 20 | |
| CFTR (ΔF508) FRETs (F) | | 4 | 40 | |
| Enzyme Mix (EM)) | | 2 | 20 | |
| Total Volume | | 10 | 100 | |
| **CFTR (Mutation Pool 1) Reaction Mixes** | | | | |
| Component | Lot # | Amount Per Reaction (μl) | Volume to Add (μl) | Component Added (Check off after adding) |
| INVADER Assay Reaction Mix | | | | |
| CFTR Mix 1 (M1) | | 8 | 70 | |
| Enzyme Mix (EM)) | | 2 | 18 | |
| Total Volume | | 10 | 88 | |
| **CFTR (Mutation Pool 2) Reaction Mixes** | | | | |
| Component | Lot # | Amount Per Reaction (μl) | Volume to Add (μl) | Component Added (Check off after adding) |
| INVADER Assay Reaction Mix | | | | |
| CFTR Mix 2 (M2) | | 8 | 70 | |
| Enzyme Mix (EM) | | 2 | 18 | |
| Total Volume | | 10 | 88 | |
| **CFTR (Mutation Pool 3) Reaction Mixes** | | | | |
| Component | Lot # | Amount Per Reaction (μl) | Volume to Add (μl) | Component Added (Check off after adding) |
| INVADER Assay Reaction Mix | | | | |
| CFTR Mix 3 (M3) | | 8 | 70 | |
| Enzyme Mix (EM)) | | 2 | 18 | |
| Total Volume | | 10 | 88 | |
| **CFTR (Mutation Pool 4) Reaction Mixes** | | | | |
| Component | Lot # | Amount Per Reaction (μl) | Volume to Add (μl) | Component Added (Check off after adding) |
| INVADER Assay Reaction Mix | | | | |
| CFTR Mix 4 (M4) | | 8 | 70 | |
| Enzyme Mix (EM) | | 2 | 18 | |

(continued)

| CFTR (Mutation Pool 4) Reaction Mixes | | | | |
|---|---|---|---|---|
| Component | Lot # | Amount Per Reaction ($\mu$l) | Volume to Add ($\mu$l) | Component Added (Check off after adding) |
| INVADER Assay Reaction Mix | | | | |
| Total Volume | | 10 | 88 | |

2. Following the sample layout, aliquot 10 $\mu$l of controls and samples ($\geq$150 ng DNA) into a 96-well low profile microplate.

3. To prevent evaporation, overlay each well with 20 $\mu$l of clear Chill-out™ or mineral oil using a multichannel pipet.

4. Aliquot the 5 reaction mixes into 5 wells of an 8-well strip in the following order:

well 1: $\Delta$F508 mix
well 2: Pool 1 mix
well 3: Pool 2 mix
well 4: Pool 3 mix
well 5: Pool 4 mix

5. Incubate samples at 95°Cfor 5 minutes in a thermal cycler.

6. Lower the temperature of the thermal cycler to 63°C, then add 10 $\mu$l of the appropriate INVADER® DNA Assay Reaction Mix to each well and mix by pipetting up and down 3-5 times. For this addition, use 5-consecutive tips of an 8 channel pipette, and aliquot reaction mix into each well moving down the plate, starting with row A, column 1. **Remember to change pipet tips after each Reaction Mix addition**. If running more than 4 patients, start again at row A, column 6. See Appendix D for full plate layout. Add the mix below the level of the Chill-out™ or mineral oil.

7. Incubate the reactions at 63°C for 5 hours.

8. After the 5 hour incubation place the low profile microplate in a plate holder in the fluorescence plate reader and read using the following parameters:

| | CytoFluor® | | GENios™ | |
|---|---|---|---|---|
| | FAM | Red | FAM | Red |
| Excitation: | 485nm/20nm | 560nm/20nm | 485nm/20nm | 560nm/20nm |
| Emission: | 530nm/25nm | 620nm/40nm | 535nm/25nm | 612nm/10nm |

Adjust the gain setting for each scan to give No Target Blank values between 100 and 200 AFU's.

9. Analyze results according to guidelines for using the ratios of the two fluorescent signals.

In a preferred embodiment, the pool assay format comprises an additional pool such that there are five mutation pool reaction mixes. In this case, the fifth pool is treated as described for pools 1-4 throughout the entire procedure described above, such that detection of all mutations can be accomplished in a total of 6 reaction wells.

**Calculation of Ratios and Guidelines for Interpretation**

In some embodiments of a kit, guidelines for using the ratios of the two fluorescent signals to determine a genotype are provided. For example, for each allele of a given polymorphism, the net signal/background, or Net Fold Over Zero (FOZ - 1), values may be calculated as follows for the signal obtained with each dye:

$$FOZ = \frac{\text{Raw counts from sample}}{\text{Raw counts from No Target Blank}}$$

The two FOZ values (i.e. wild type and mutant) for each sample were used to calculate the WT : Mut Ratio as follows:

$$\text{Ratio} = \frac{(\text{Net WT FOZ})}{(\text{Net Mut FOZ})}$$

where Net FOZ = FOZ - 1

In some embodiments, supplementary documentation, such as protocols for ancillary procedures, *e.g.*, for the preparation of additional reagents, or for preparation of samples for use in the methods of the present invention, are provided. In preferred embodiments, supplementary documentation includes guidelines and lists of precautions provided to facilitate successful use of the methods and kits by unskilled or inexperienced users. In particularly preferred embodiments, supplementary documentation includes a troubleshooting guide, *e.g.*, a guide describing possible problems that may be encountered by users, and providing suggested solutions or corrections to intended to aid the user in resolving or avoiding such problems. Kits are not part of the invention.

For example, and without intending to limit such supplementary documentation to any particular content, kits may comprise any of the following procedures and guidelines:

## II. SAMPLE PREPARATION

In preferred embodiments, samples are diluted to concentrations that correspond to a 10-$\mu$l addition per reaction. The concentration of a 100-ng sample should be 15ng/$\mu$l.

The assay is optimized for performance with genomic DNA samples prepared from whole blood or buffy coat. Several DNA extraction methods/kits have been validated for performance in the Biplex INVADER assay:

- QIAGEN QIAamp® Blood Kit
- Gentra Systems PUREGENE™ Kit
- Gentra Systems GENERATION® Products

Quantitation is not necessary if using one of these recommended sample preparation methods (*i.e.*, QIAGEN or Gentra). In other embodiments, the DNA sample should be quantitated. In a preferred embodiment, such quantitation is accomplished using the PicoGreen® or OliGreen® assay. Quantitating by $A_{260}/A_{280}$ can lead to an overestimation of the amount of DNA in the sample due to RNA contamination. A low $A_{260}/A_{280}$ reading (< 1.5) indicates there is an overabundance of protein in the sample. In particularly preferred embodiments, only samples with a concentration > 10 ng/$\mu$l are used in the INVADER DNA Assay.

| Problem | Possible Solution |
|---|---|
| **No Signal or Low Signal** | Assay:<br>• Mixing inconsistencies. Make sure all reagents are properly mixed prior to assembly of INVADER® DNA Assay Reaction Mix. The controls and INVADER® DNA Assay Reaction Mixes must be mixed thoroughly and consistently before the plate is set up. During addition of INVADER® DNA Assay Reaction Mix to sample plate, mix by pipetting up and down several times, ensuring that all liquid is expelled before removing the tip.<br>• Verify that reagents were added in the correct sequence, to the correct mix, and that the correct mix is added to the appropriate controls/sample wells (refer to sample plate layout).<br>• Verify that all reagents were stored at the proper temperature as indicated in this package insert.<br>• Make sure that 10 $\mu$l of the appropriate control was added to each well.<br>• Make sure that the 10 $\mu$l of the appropriate INVADER® DNA Assay Reaction Mix was added below the level of the mineral oil or Chill-out™ 14 liquid wax. Not adding the correct amount will result in loss of signal. |

(continued)

| Problem | Possible Solution |
|---|---|
| | • Verify that the correct INVADER® DNA Assay Reaction Mix is added to the appropriate control. |
| | • Make sure assay is run for at five hours at 63°C. |
| | • Use mineral oil or clear Chill-out™ 14 liquid wax to prevent evaporation during the reaction. Instrument: |
| | • Verify that the fluorescence plate reader is set to the correct excitation and emission wavelengths for each scan. If possible, run a diagnostic test on the fluorescence plate reader to ensure that the instrument and light source are working properly. Verify that two scans were performed at two different wavelengths. |
| | • Make sure the proper "96-well plate type" has been selected in the fluorescence plate reader. |
| | • Verify that the coordinates of the plate are programmed correctly in the fluorescence plate reader. Signal should be read in the middle of the well and at an optimal distance from the plate for best results. |
| | • Incubations should be conducted in properly calibrated heating units. Checking these units on a regular basis using a thermocouple thermometer equipped with a probe traceable to NIST standards is recommended. |
| | • Make sure that the plate is firmly seated in the thermal cycler or heat block. |
| **High Signal in Control 4 (No Target Blank)** | Assay: |
| | • Use DNase/RNase free aerosol barrier tips and sterile tubes for making the INVADER® DNA Assay Reaction Mix. |
| | • Make sure that pipet tips are changed after each use. |
| | • Wear gloves when setting up the assay. |
| | • Make sure that pipet tips do not touch any other surfaces except the solution being pipetted, since nucleases may be present. |
| | • Do not touch pipet tips with hands. |
| | Instrument: |
| | • Adjust the gain setting of the fluorescence plate reader such that Control 4 (No Target Blank) reads approximately 200 for each scan. |
| **Fluorescent Signal Is Off-scale** | Assay: |
| | • Use DNase/RNase free aerosol barrier tips and sterile tubes for making the INVADER® DNA Assay Reaction Mix. |
| | • Confirm that the incubations were done for the correct amount of time and at the correct temperature. |
| | Instrument: |
| | • Adjust the gain of the fluorescence plate reader. The gain of the two scans should be set so that Control 4 (No Target Blank) reads at least 100 for each scan; however, an approximate level of 200 is recommended. |
| | • Allow the lamp in the fluorescence plate reader to warm up for at least 10 minutes before reading the results. |

**EXAMPLES**

The following examples serve to illustrate certain preferred embodiments and aspects of the present invention and are not to be construed as limiting the scope thereof. Ex. (Example); Fig. (Figure);°C (degrees Centigrade); g (gravitational field); hr (hour); min (minute); olio (oligonualeotide); rxn (reaction); vol (volume); w/v (weight to volume); v/v (volume to volume); BSA (bovine serum albumin); CTAB (cetyltrimethylammonium bromide); HPLC (high pressure liquid chrornatography); DNA (deoxyribonucleic acid); p (plasmid); $\mu$l (microliters); ml (milliliters); $\mu$g (micrograms); mg (milligrams); M (molar); mM (milliMolar); $\mu$M (microMolar); pmoles (picomoles); amoles (attomoles); zmoles (zeptomoles); nm (nanometers); kdal (kilodaltons); OD (optical density); EDTA (ethylene diamine tetra-acetic acid); FITC (fluorescein isothiocyanate); SDS (sodium dodecyl sulfate); NaPO$_4$ (sodium phosphate); NP-40 (Nonidet P-40); Tris (tris(hydroxymethyl)-aminomethane); PMSF (phenylmethylsulfonylfluoride); TBE (Tris-Borate-EDTA, *i.e.*, Tris buffer titrated with boric acid rather than HCl and containing EDTA); PBS (phosphate buffered saline); PPBS (phosphate buffered saline containing 1 mM PMSF); PAGE (polyacrylamide gel electrophoresis); Tween (polyoxyethylene-sorbitan); Red (REDMOND RED Dye, Epoch Biosciences, Bothell WA) Z28 (ECLIPSE Quencher, Epoch Biosciences, Bothell, WA); ATCC (American Type Culture Collection, Rockville, MD); Coriell (Coriell Cell Repositories, Camden, NJ); DSMZ (Deutsche Sammlung von Mikroorganismen und Zellculturen, Braunschweig, Germany); Ambion (Ambion, Inc., Austin, TX); Boehringer (Boehringer Mannheim Biochemical, Indianapolis, IN); MJ Research (MJ Research, Watertown, MA; Sigma (Sigma Chemical Company, St. Louis, MO); Dynal (Dynal A.S., Oslo, Norway); Gull (Gull Laboratories, Salt Lake City, UT); Epicentre (Epicentre Technologies, Madison, WI); Lampire (Biological Labs., Inc., Coopersberg, PA); MJ Research (MJ Research, Watertown,MA); National Biosciences (National Biosciences, Plymouth, MN); NEB (New England Biolabs, Beverly, MA); Novagen (Novagen, Inc., Madison, WI); Perkin Elmer (Perkin-Elmer/ABI, Norwalk, CT); Promega (Promega, Corp., Madison, WI); Stratagene (Stratagene Cloning Systems, La Jolla, CA); Clonetech (Clonetech, Palo Alto, CA) Pharmacia (Pharmacia, Piscataway, NJ); Milton Roy (Milton Roy, Rochester, NY); Amersham (Amersham International, Chicago, IL); and USB (U.S. Biochemical, Cleveland, OH). Glen Research (Glen Research, Sterling, VA); Coriell (Coriell Cell Repositories, Camden, NJ); Gentra (Gentra, Minneapolis, MN); Third Wave Technologies (Third Wave Technologies, Madison, WI); PerSeptive Biosystems (PerSeptive Biosystems, Framington, MA); Microsoft (Microsoft, Redmond, WA); Qiagen (Qiagen, Valencia, CA); Molecular Probes (Molecular Probes, Eugene, OR); VWR (VWR Scientific, ); Advanced Biotechnologies (Advanced Biotechnologies, INC., Columbia, MD).

**Example 1**

**Reagents and Methods for Detection of Cystic Fibrosis Transmembrane Conductance Regulator (CFTR) Mutations**

**Reagents:**

CFTR (2184delA) Control (1 vial marked "2184delA ", 250 $\mu$l)
CFTR (1898+1G>A) Control (1 vial marked "1898+1G>A ", 250 $\mu$l)
CFTR (I148T) Control (1 vial marked "I148T", 250 $\mu$l)
CFTR (1078delT) Control (1 vial marked "1078delT", 250 $\mu$l)
CFTR (W1282X) Control (1 vial marked "W1282X", 250 $\mu$l)
CFTR (621+1G>T) Control (1 vial marked "621+1G>T", 250 $\mu$l)
Control 4 (No Target Blank) (1 vial marked "C4", 1250 $\mu$l)

Cleavase® X/CF Enzyme or Cleavase Enzyme Mix (20 ng/$\mu$l, 1 vial, 1250 $\mu$l)

**Reagent Composition:**

CFTR (2184delA) Control is a plasmid construct containing the 2184delA sequence suspended in yeast tRNA and buffered nuclease-free water. CFTR (1898+1 G>A) Control, CFTR (I148T) Control, CFTR (1078delT) Control, CFTR (W1282X) and CFTR (621+1G>T) Control are synthetic oligonucleotides suspended in yeast tRNA and buffered nuclease-free water. Control 4 (No Target Blank) contains yeast tRNA in buffered nuclease-free water.

**CONTROL USAGE:**

1. Determine the number (singlicate, duplicate, triplicate, quadruplicate) of controls to be tested. Use 10 $\mu$l of control material in each reaction.
2. Treat control materials the same as test samples throughout the INVADER® DNA Assay.

3. Control materials and test samples should be analyzed on a fluorescence plate reader.

Oligonucleotides designed to detect a sequence in the 3' UTR (untranslated region) of the CFTR gene are designated as the internal control. These oligonucleotides were designed to perform in the INVADER assay such that the signal from this portion of the CFTR gene, while present in duplicate in each assay, is approximately equivalent to the amount of signal that would be expected from only a single copy of a given sequence, such that its presence does not interfere with the detection of a mutant allele in any of the pooled sets of reactions.

**EXPECTED RESULTS:**

CFTR (2184delA) Control and CFTR (1898+1G>A) Control material should react with only the CFTR Mix 1 (F dye signal); CFTR (I148T) Control and CFTR (1078delT) material should react with only the CFTR Mix 2 (F dye signal); CFTR (W1282X) Control material should react with only the CFTR Mix 3 (F dye signal); CFTR (621+1G>T) Control should react with only the CFTR Mix 4 (F dye signal); and Control 4 (No Target Blank) material should show only R dye signal but not F dye signal with any of the CFTR Mixes 1-4 (F dye and R dye signals). Actual signal values depend on reaction volumes, test methods, and the fluorescence plate reader used.

1. Preparation of INVADER DNA Reaction Mixes 1-4 (mixes were scaled by number of reactions times 1.25):

| Component (mixed prior to addition) | Volume (per rxn) to Reaction Mix 1 | Volume (per rxn) to Reaction Mix 2 | Volume (per rxn) to Reaction Mix 3 | Volume (per rxn) to Reaction Mix 4 |
|---|---|---|---|---|
| CFTR Mix 1 | 8 $\mu$l | 0 | 0 | 0 |
| CFTR Mix 2 | 0 | 8 $\mu$l | 0 | 0 |
| CFTR Mix 3 | 0 | 0 | 8 $\mu$l | 0 |
| CFTR Mix 4 | 0 | 0 | 0 | 8 $\mu$l |
| Cleavase enzyme mix | 2 $\mu$l | 2 $\mu$l | 2 $\mu$l | 2 $\mu$l |
| **Total** | **10 $\mu$l** | **10 $\mu$l** | **10 $\mu$l** | **10 $\mu$l** |

2. 10 $\mu$l of each target DNA (sample or control) was aliquoted into an assigned reaction well.

3. 20 $\mu$l of Mineral Oil was added to each well to prevent evaporation.

4. Samples were incubated at 95°C for 5 minutes in a thermal cycler.

5. After the temperature was reduced to 63°C; 10 $\mu$l of the INVADER® DNA Assay Reaction Mixes were added to the appropriate wells, taking care to add the reaction mix below the mineral oil.

6. Reactions were incubated at 63°C for 5 hours in a thermal cycler.

7. The reaction plate was read using the following settings on a CytoFluor® Series 4000 Fluorescence Multi-Well Plate Reader:

| Cycle 1 | Cycle 2 |
|---|---|
| Excitation = 485/20 | Excitation = 560/20 |
| Emission = 530/25 | Emission = 620/40 |
| Gain = 40 | Gain = 46 |
| Reads/well = 10 | Reads/well = 10 |

8. QA acceptance criteria for positive and negative samples:

**Table 1. Mix 1 criteria.**

| Ratio = FAM AdjNetFOZ (0.01 if <=0/RedFOZ-1) | | | |
|---|---|---|---|
| **Ratio** | **FamFOZ** | **RedFOZ** | **Genotype** |
| >0.4 | >1.75 | >= 2.0 | Positive |
| >0.4 | <=1.75 | >= 2.0 | Low signal |

(continued)

| Ratio = FAM AdjNetFOZ (0.01 if <=0/RedFOZ-1) | | | |
|---|---|---|---|
| **Ratio** | **FamFOZ** | **RedFOZ** | **Genotype** |
| <0.275 | NA | >= 2.0 | Negative |
| >=0.275 and <=0.4 | NA | >= 2.0 | EQ |
| NA | NA | <2.0 | Low signal |

**Table 2. Mix 2 criteria.**

| Ratio = FAM AdjNetFOZ (0.01 if <=0/RedFOZ-1 | | | |
|---|---|---|---|
| **Ratio** | **FamFOZ** | **RedFOZ** | **Genotype** |
| >0.25 | >1.75 | >= 2.0 | Positive |
| >0.25 | <=1.75 | >= 2.0 | Low signal |
| <0.175 | NA | >= 2.0 | Negative |
| >=0.175 and <=0.25 | NA | >= 2.0 | EQ |
| NA | NA | <2.0 | Low signal |

**Table 3. Mix 3 criteria.**

| Ratio = FAM AdjNetFOZ (0.01 if <=0/RedFOZ-1 | | | |
|---|---|---|---|
| **Ratio** | **FamFOZ** | **RedFOZ** | **Genotype** |
| >0.3 | >1.75 | >= 2.0 | Positive |
| >0.3 | <=1.75 | >= 2.0 | Low signal |
| <0.2 | NA | >= 2.0 | Negative |
| >=0.2 and <=0.3 | NA | >= 2.0 | EQ |
| NA | NA | < 2.0 | Low signal |

**Table 4. Mix 4 criteria.**

| Ratio = FAM AdjNetFOZ (0.01 if <=0/RedFOZ-1 | | | |
|---|---|---|---|
| **Ratio** | **FamFOZ** | **RedFOZ** | **Genotype** |
| >0.275 | >1.75 | >= 2.25 | Positive |
| >0.275 | <=1.75 | >= 2.25 | Low signal |
| <0.175 | NA | >= 2.25 | Negative |
| >=0.175 and <=0.275 | NA | >= 2.25 | EQ |
| NA | NA | < 2.25 | Low signal |

**Example 2**

**Alternative Oligonucleotide and Pool Configurations**

In another embodiment, alternative designs were created for some of the oligonucleotides, and some oligonucleotides were included in different pools. These alternative reaction mixes were applied to the analysis of samples as described in Example 1.

**Reagents:**

CFTR (I148T) M1 Mut Control (1 vial marked " CA ", 250 µl)
CFTR (1898+1G>A) M1 Mut Control (1 vial marked "CB", 250 µl)
CFTR (1078delT) M2 Mut Control (1 vial marked "CC ", 250 µl)
CFTR (621+1G>T) M3 Mut Control (1 vial marked "CD", 250 µl)
CFTR (G542X) M4 Mut Control (1 vial marked "CE", 250 µl)
CFTR (2184delA) M5 Mut Control (1 vial marked "CF", 250 µl)
Control 4 (No Target Blank) (1 vial marked "C4", 1250 µl)

CLEAVASE X/CF Enzyme or CLEAVASE Enzyme Mix (20 ng/µl, 1 vial, 1250 µl)

**Reagent Composition:**

CFTR (2184delA) M5 Mut Control is a plasmid construct containing the 2184delA sequence suspended in yeast tRNA and buffered nuclease-free water. CFTR (I148T) M1 Mut Control, CFTR (1898+1G>A) M1 Mut Control, CFTR (1078delT) M2 Mut Control, CFTR (621+1G>T) M3 Mut Control, and CFTR (G542X) M4 Mut Control are synthetic oligonucleotides suspended in yeast tRNA and buffered nuclease-free water. Control 4 (No Target Blank) contains yeast tRNA in buffered nuclease-free water.

**CONTROL USAGE:**

1. Determine the number (singlicate, duplicate, triplicate, quadruplicate) of controls to be tested. Use 10 µl of control material in each reaction.
2. Treat control materials the same as test samples throughout the INVADER® DNA Assay.
3. Control materials and test samples should be analyzed on a fluorescence plate reader.

**EXPECTED RESULTS:**

The CFTR (I148T) Control should react only with assays designed to detect the presence of the CFTR (I148T) mutant-allele. The CFTR (1898+1G>A) Control should react only with assays designed to detect the presence of the CFTR (1898+1 G>A) mutant allele. The CFTR (1078delT) Control should react only with assays designed to detect the presence of the CFTR (1078delT) mutant allele. The CFTR (621+1G>T) Control should react only with assays designed to detect the presence of the CFTR (621+1G>T) mutant allele. The CFTR (G542X) Control should react only with assays designed to detect the presence of the CFTR (G542X) mutant allele. The CFTR (2184delA) Control should react only with assays designed to detect the presence of the CFTR (2184delA) mutant allele. Control 4 (No Target Blank) does not contain any CFTR sequence and, therefore, should not react with any assay designed to detect the presence of a CFTR allele.
1. Preparation of INVADER DNA Reaction Mixes 1-4 (mixes were scaled by number of reactions times 1.25):

| Component (mixed prior to addition) | Volume (per rxn) to Reaction Mix 1 | Volume (per rxn) to Reaction Mix 2 | Volume (per rxn) to Reaction Mix 3 | Volume (per rxn) to Reaction Mix 4 | Volume (per rxn) to Reaction Mix 5 |
|---|---|---|---|---|---|
| CFTR Mix 1 | 8 µl | 0 | 0 | 0 | 0 |
| CFTR Mix 2 | 0 | 8 µl | 0 | 0 | 0 |
| CFTR Mix 3 | 0 | 0 | 8 µl | 0 | 0 |
| CFTR Mix 4 | 0 | 0 | 0 | 8 µl | 0 |
| CFTR Mix 5 | 0 | 0 | 0 | 0 | 8 µl |
| Cleavase enzyme mix | 2 µl | 2 µl | 2 µl | 2 µl | 2 µl |

(continued)

| Component (mixed prior to addition) | Volume (per rxn) to Reaction Mix 1 | Volume (per rxn) to Reaction Mix 2 | Volume (per rxn) to Reaction Mix 3 | Volume (per rxn) to Reaction Mix 4 | Volume (per rxn) to Reaction Mix 5 |
|---|---|---|---|---|---|
| Total | 10 μl | 10 μl | 10 μl | 10 μl | 10 μl |

2. 10 μl of each target DNA (sample or control) was aliquoted into an assigned reaction well.

3. 20 μl of Mineral Oil was added to each well to prevent evaporation.

4. Samples were incubated at 95°C for 5 minutes in a thermal cycler.

5. After the temperature was reduced to 63°C; 10 μl of the INVADER® DNA Assay Reaction Mixes were added to the appropriate wells, taking care to add the reaction mix below the mineral oil.

6. Reactions were incubated at 63°C for 5 hours in a thermal cycler.

7. The reaction plate was read using the following settings on a CytoFluor® Series 4000 Fluorescence Multi-Well Plate Reader:

| Cycle 1 | Cycle 2 |
|---|---|
| Excitation = 485/20 | Excitation = 560/20 |
| Emission = 530/25 | Emission = 620/40 |
| Gain = 40 | Gain = 46 |
| Reads/well = 10 | Reads/well = 10 |

8. QA acceptance criteria for positive and negative samples:

**Table 5. Mix 1 criteria.** Ratio = FAMFOZ-1 (Adj to 0.01 if <=0)/RedFOZ-1

| Ratio | FamFOZ | RedFOZ | Genotype |
|---|---|---|---|
| >0.35 | >=1.75 | >= 2.0 | Positive |
| >0.35 | <1.75 | >= 2.0 | EQ |
| <0.2 | NA | >= 2.0 | Negative |
| >=0.200 and <=0.35 | NA | >= 2.0 | EQ |
| NA | NA | < 2.0 | Low signal |

**Table 6. Mix 2 criteria.**

| Ratio = FAMFOZ-1 (Adj to 0.01 if <=0)/RedFOZ-1 | | | |
|---|---|---|---|
| Ratio | FamFOZ | RedFOZ | Genotype |
| >0.25 | >=1.5 | >= 2.0 | Positive |
| >0.25 | <1.5 | >= 2.0 | EQ |
| <0.125 | NA | >= 2.0 | Negative |
| >=0.125 and <=0.25 | NA | >= 2.0 | EQ |
| NA | NA | <2.0 | Low signal |

### Table 7. Mix 3 criteria.

| Ratio = FAMFOZ-1 (Adj to 0.01 if <=0)/RedFOZ-1 | | | |
|---|---|---|---|
| **Ratio** | **FamFOZ** | **RedFOZ** | **Genotype** |
| >0.275 | >=1.5 | >= 2.0 | Positive |
| >0.275 | <1.5 | >= 2.0 | EQ |
| <0.15 | NA | >= 2.0 | Negative |
| >=0.15 and <=0.275 | NA | >= 2.0 | EQ |
| NA | NA | < 2.0 | Low signal |

### Table 8. Mix 4 criteria.

| Ratio = FAMFOZ-1 (Adj to 0.01 if <=0)/RedFOZ-1 | | | |
|---|---|---|---|
| **Ratio** | **FamFOZ** | **RedFOZ** | **Genotype** |
| >0.5 | >=1.75 | >= 2.0 | Positive |
| >0.5 | <1.75 | >= 2.0 | EQ |
| <0.225 | NA | >= 2.0 | Negative |
| >=0.225 and <=0. 5 | NA | >= 2.0 | EQ |
| NA | NA | < 2.0 | Low signal |

### Table 9. Mix 5 criteria.

Ratio = FAMFOZ-1 (Adj to 0.01 if <=0)/RedFOZ-1

**Pool 5**
Ratio = Fam AdjNetFOZ (0.01 if <=0) / RedFOZ-1

| **Ratio** | **FamFOZ** | **RedFOZ** | **Genotype** |
|---|---|---|---|
| >0.8 | >=1.75 | >=2.0 | Positive |
| >0.8 | <1.75 | >= 2.0 | EQ |
| <0.65 | NA | >= 2.0 | Negative |
| >=0.65 and <=0.8 | NA | >= 2.0 | EQ |
| NA | NA | < 2.0 | Low signal |

**Example 3**

**Reagents and Methods for Detection of the ΔF508 Mutation in Cystic Fibrosis Transmembrane Conductance Regulator (CFTR) Gene in a Biplex Format**

**Reagents:**

CFTR (ΔF508) Control 1 (WT) (1 vial marked "C1", 250 μl)
CFTR (ΔF508) Control 2 (HET) (1 vial marked "C2", 250 μl)
CFTR (ΔF508) Control 3 (MT) (1 vial marked "C3", 250 μl)

Control 4 (No Target Blank) (1 vial, marked "C4", 1250 μl)

**Reagent Storage:**

Store at -20 °C

**Reagent Composition:**

CFTR (ΔF508) Control 1 (WT), CFTR (ΔF508) Control 2 (HET), and CFTR (ΔF508) Control 3 (MT) are synthetic oligonucleotides suspended in yeast tRNA and buffered nuclease-free water. Control 4 (No Target Blank) contains yeast tRNA in buffered nuclease-free water.

**CONTROL USAGE:**

**1.** Determine the number (singlicate, duplicate, triplicate, quadruplicate) of controls to be tested. Use 10 μl of control material in each reaction.

2. Treat control materials the same as test samples throughout the INVADER® DNA Assay.

3. Control materials and test samples should be analyzed on a fluorescence plate reader.

**EXPECTED RESULTS:**

CFTR (ΔF508) Control 1 material should react with only the CFTR (ΔF508) WT Primary Probe (F dye signal); CFTR (ΔF508) Control 3 material should react with only the CFTR (ΔF508) MT Primary Probe (R dye signal); CFTR (ΔF508) Control 2 material should react with both the CFTR (ΔF508) Primary Probes (F dye and R dye signals); and Control 4 (No Target Blank) material should show no specific reaction with either one or both of the CFTR (ΔF508) Primary Probes (F dye and R dye signals). Actual signal values depend on reaction volumes, test methods, and the fluorescence plate reader used.

We evaluated the effectiveness of our design by testing the assay on characterized genomic samples, where available, and synthetic oligonucleotide targets when no genomic samples could be obtained. The first set of INVADER® oligonucleotides placed the F508C polymorphism at position -1, one of the critical bases required for specificity. This set did not detect the F508C DNA. The second set, designed to detect the wild type DNA in the presence of all polymorphisms, namely F508C, I507V, and I 506V, placed the polymorphisms at positions 3, 7 and 10, respectively. This set resulted in the generation of signal from the wild-type probe in the presence of synthetic targets containing either the I507V or I506V benign polymorphisms, consistent with the phenotypes of such alterations.

Genotype calls were made using the following criteria:

| Ratio | FamFOZ | RedFOZ | Genotype |
|---|---|---|---|
| >=5 | >=1.75 | NA | WT |
| >=0.5 and <=2 | >=1.75 | >=1.75 | Het |
| <=0.2 | NA | >=1.75 | Mut |

A second requirement was the proper discrimination of ΔF508 and ΔI507. The detection of the mutation ΔI507 is relegated to a separate test; the purpose of the 508 test is to report only the ΔF508 mutation. However, the ΔF508 and ΔI507 sequences are extremely similar, differing by only one base. Due to the INVADER assay's tolerance of a mismatch at specific positions, we incorporated a second, adjacent mismatch into the ΔF508 probe to avoid detection of the ΔI507 sequence. This resulted in a mismatch at position 5 on the ΔF508 target, and at positions 4 and 5 on the ΔI507 target. The mismatch at position 5 is tolerated by the assay, generating robust signal on the ΔF508 target, while the two adjacent mismatches at positions 4 and 5 are sufficient to prevent signal generation from the ΔI507 target.

**Example 4**

**Reagents and Methods for Detection of the 2184delA Mutation in Cystic Fibrosis Transmembrane Conductance Regulator (CFTR) Gene in a Biplex Format**

In an alternative embodiment to that described in Example 2, Pool 5 comprises a biplex format assay for detecting a wild type or mutant sequence at position 2184.

**Reagents:**

CFTR (2184delA) Control 1 (WT) (1 vial marked "C1", 250 μl)
CFTR (2184delA) Control 2 (HET) (1 vial marked "C2", 250 μl)
CFTR (2184delA) Control 3 (Mut) (1 vial marked "C3", 250 μl)
Control 4 (No Target Blank) (1 vial marked "C4", 1250 μl)

**Reagent Storage:**

Store at -20 °C

**Reagent Composition:**

CFTR (2184delA) Controls 1-3 are plasmid constructs containing the 2184delA sequences suspended in yeast tRNA and buffered nuclease-free water. Control 4 (No Target Blank) contains yeast tRNA in buffered nuclease-free water.

**CONTROL USAGE:**

1. Determine the number (singlicate, duplicate, triplicate, quadruplicate) of controls to be tested. Use 10 μl of control material in each reaction.

2. Treat control materials the same as test samples throughout the INVADER® DNA Assay.

4. Control materials and test samples should be analyzed on a fluorescence plate reader.

Exemplary criteria for making genotype calls using this assay are as follows:

| Ratio | FamFOZ | RedFOZ | Genotype |
| --- | --- | --- | --- |
| >=5 | NA | >=1.75 | WT |
| >=0.5 and <=2 | >=1.75 | >=1.75 | Het |
| <=0.2 | >=1.75 | NA | Mut |

Exemplary criteria for the 2184delA plasmid control are as follows:

| **2184delA Control 2**<br>Ratio = Red AdjNetFOZ(0.15)/Fam AdjNetFOZ(0.15) | | | |
| --- | --- | --- | --- |
| **Ratio** | **FamFOZ** | **RedFOZ** | **Genotype** |
| >=5 | NA | >=1.75 | WT |

(continued)

| Ratio | FamFOZ | RedFOZ | Genotype |
|---|---|---|---|
| >=0.375 and <=1.3 | >=1.75 | >=1.75 | Het |
| <=0.2 | >=1.75 | NA | Mut |

### EXPECTED RESULTS:

CFTR (2184delA) Control 1 material should react only with assays designed to detect the CFTR (2184delA) WT allele (R dye). CFTR (2184delA) Control 3 material should react only with assays designed to detect the CFTR (2184delA) Mut allele (F dye). Assays designed to detect the CFTR (2184delA) Mut allele should not react with samples containing the 2183>AA variant. CFTR (2184delA) Control 2 material should react only with assays designed to detect the CFTR (2184delA) WT and Mut alleles (R and F dyes). Control 4 (No Target Blank) does not contain any CFTR sequence and, therefore, should not react with any assay designed to detect the presence of a CFTR allele. Actual signal values depend on reaction volumes, test methods, and the fluorescence plate reader used.

### Example 5

### Reagents and Methods for Detection of the 3199del6 Mutation in Cystic Fibrosis Transmembrane Conductance Regulator (CFTR) Gene in a Biplex Format

In some embodiments, it may be desired to determine the genotype of a sample at nucleotide 3199del6. For example, in some cases, it may be of importance to reflex a sample found to contain the I148T allele to determine whether or not it further contains the 3199del6 variation.

### Reagents:

    CFTR (3199del6) Control 1 (WT) (1 vial marked "C1", 150 µl)
    CFTR (3199del6) Control 2 (HET) (1 vial marked "C2", 150 µl)
    CFTR (3199del6) Control 3 (Mut) (1 vial marked "C3", 150 µl)
    Control 4 (No Target Blank) (1 vial marked "C4", 1250 µl)

### Reagent Storage:

Store at -20 °C

### Reagent Composition:

CFTR (3199del6) Controls 1-3 are synthetic oligonucleotides containing the 3199del6 sequences suspended in yeast tRNA and buffered nuclease-free water. Control 4 (No Target Blank) contains yeast tRNA in buffered nuclease-free water.

### CONTROL USAGE:

    1. Determine the number (singlicate, duplicate, triplicate, quadruplicate) of controls to be tested. Use 10 µl of control material in each reaction.

    2. Treat control materials the same as test samples throughout the INVADER DNA Assay.

    3. Control materials and test samples should be analyzed on a fluorescence plate reader.

**EXPECTED RESULTS:**

CFTR (3199del6) Control 1 material should react only with assays designed to detect the CFTR (3199del6) WT allele (F dye). CFTR (3199del6) Control 3 material should react only with assays designed to detect the CFTR (3199del6) Mut allele (R dye). Control 2 material should react only with assays designed to detect the CFTR (3199del6) WT and Mut alleles (F and R dyes). Control 4 (No Target Blank) does not contain any CFTR sequence and, therefore, should not react with any assay designed to detect the presence of a CFTR allele. Actual signal values depend on reaction volumes, test methods, and the fluorescence plate reader used.

**Example 6**

**Reagents and Methods for Detection of the 2183AA>G Mutation in Cystic Fibrosis Transmembrane Conductance Regulator (CFTR) Gene in a Biplex Format**

In an alternative embodiment, an additional assay comprises a biplex format assay for detecting a wild type or mutant sequence at position 2183.

**Reagents:**

CFTR (2183AA>G) Control 1 (WT) (1 vial marked "C1", 250 $\mu$l)
CFTR (2183AA>G) Control 2 (HET) (1 vial marked "C2", 250 $\mu$l)
CFTR (2183AA>G) Control 3 (Mut) (1 vial marked "C3", 250 $\mu$l)
Control 4 (No Target Blank) (1 vial marked "C4", 1250 $\mu$l)

**Reagent Storage:**

Store at -20 °C

**Reagent Composition:**

CFTR (2183AA>G) Controls 1-3 are synthetic targets containing the 2183AA>G sequences suspended in yeast tRNA and buffered nuclease-free water. Control 4 (No Target Blank) contains yeast tRNA in buffered nuclease-free water.

**CONTROL USAGE:**

1. Determine the number (singlicate, duplicate, triplicate, quadruplicate) of controls to be tested. Use 10 $\mu$l of control material in each reaction.
2. Treat control materials the same as test samples throughout the INVADER® DNA Assay.
3. Control materials and test samples should be analyzed on a fluorescence plate reader.

**EXPECTED RESULTS:**

CFTR (2183AA>G) Control 1 material should react only with assays designed to detect the CFTR (2183AA>G) WT allele (F dye). CFTR (2183AA>G) Control 3 material should react only with assays designed to detect the CFTR (2183AA>G) Mut allele (R dye). CFTR (2183AA>G) Control 2 material should react only with assays designed to detect the CFTR (2183AA>G) WT and Mut alleles (F and R dyes). Control 4 (No Target Blank) does not contain any CFTR sequence and, therefore, should not react with any assay designed to detect the presence of a CFTR allele. Actual signal values depend on reaction volumes, test methods, and the fluorescence plate reader used.

**Example 7**

**Reagents and Methods for Determining the Genotype of Cystic Fibrosis Transmembrane Conductance Regulator (CFTR) Gene in a Biplex Format at**

**Multiple Loci**

In a further alternative embodiment, additional methods comprise biplex format assays for detecting a wild type or mutant sequence at any of several CFTR loci, including, but not limited to: A455E, 3659delC, G85E, N1303K, D1270N, R560T, 621+ 1G>T, 1717-1G>A, 1078delT, R347P, 2184delA (as in Example 4), V520F, R347H, 2183AA>G (as in Example 6), ΔF508 (as in Example 3), R334W, R117H, 2789+5G>A, 394delTT, 3849+10kbC>T, W1282X, G542X, 3120+1G>A, I148T, 711+1G>T, S549N, D1152H, 3199del6 (as in Example 5), 3905insT, Y1092X C>G, 3849+4A>G, 3876delA, Q493X, G551D, R553X, R1162X, F508C, Y1092X C>A, S549R A>C, S549R T>G, ΔI507, IVS8 5T/7T/9T, 1898+1 G>A, and Y122X. Assays to determine genotypes at any of these positions in CFTR may be carried out as described herein or in the previously described examples, as indicated.

Experiments were conducted to determine the genotypes of 288 blood samples as well as several genomic DNA samples obtained from Coriell (Coriell Cell Repositories, Camden, NJ) at three distinct CFTR loci: G85E, A455E, and 3659delC. In each case, characterized heterozygous samples were obtained from Coriell (cat. nos. NA11282 and NA13423 for G85E; NA11283 and NA11290 for A455E, and NA11275 for 3659delC). Genomic DNA was extracted from blood samples using up to three commercially available DNA purification kits according to the manufacturer's instructions, as indicated in the figure: QIAamp DNA Blood Mini Kit (Qiagen, Inc. Valencia, CA); GENERATION Capture Column Kit (Gentra Systems, Minneapolis, MN); or PUREGENE DNA Purification Kit (Gentra Systems). Homozygous mutant samples were synthetic DNA targets (SEQ ID NO: 19, SEQ ID NO: 5, and SEQ ID NO: 12 for G85E mutant, A455E mutant, and 3659delC mutant, respectively). All DNA targets were denatured at 95°C for 5 minutes prior to incubation with the INVADER assay reagents.

The particular FRET probes used in each assay were as indicated in Figure 2. INVADER assays were carried out in 384-well skirted hard-shell microtiter plates (MJ Research, HSP 3801 or 3901). Reactions comprised the following reagents in a final volume of 6 μl.

| Reagent | Final concentration |
|---|---|
| MOPS, pH 7.5 | 10 mM |
| $MgCl_2$ | 14 mM |
| CLEAVASE X enzyme | 2 ng/μl |
| Wild-type probe oligonucleotide | 0.5 μM |
| Mutant probe oligonucleotide | 0.5 μM |
| INVADER oligonucleotide | 0.05 μM |
| FRET oligonucleotide (FAM dye) | 0.25 μM |
| FRET oligonucleotide (RED dye) | 0.25 μM |
| Final volume | 3 μl |

Aliquots (3 μl) of the reagents listed in the table were added to the appropriate wells of a 384-well microtiter plate containing 3 μl of the appropriate DNA or no target control sample. Genomic DNA was at a concentration of approximately 10 ng/μl; synthetic DNA controls contained synthetic target at a final concentration of 5 fM, and no target controls contained 3 μl of tRNA in distilled $H_2O$ at 100ng/μl. Reactions were overlaid with 6.5 μl of mineral oil and incubated at the reaction temperature of 63°C in an air incubator (LICONIC Instruments, Mauren Lichtenstein) for 4 hours. The microplates were then read in a SAFIRE (Tecan Group Ltd, Maennedorf, Switzerland) microtiter plate reader.

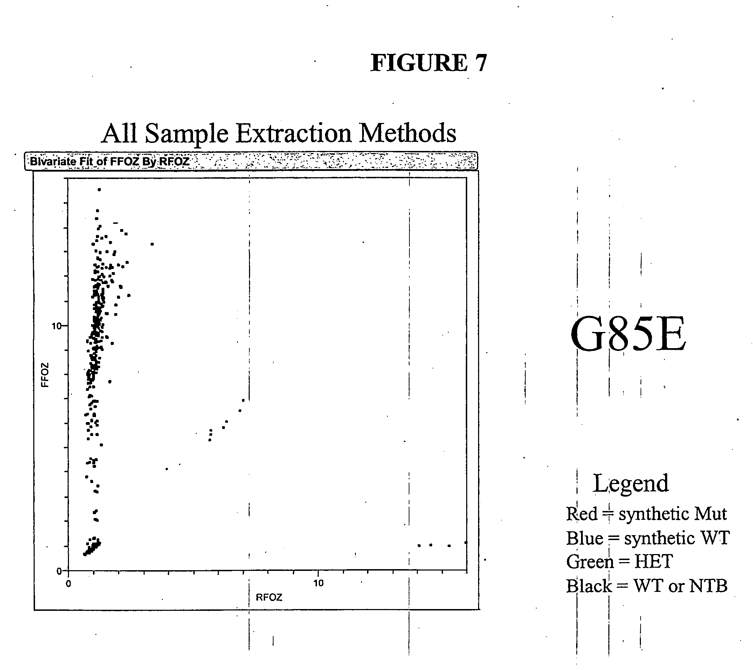

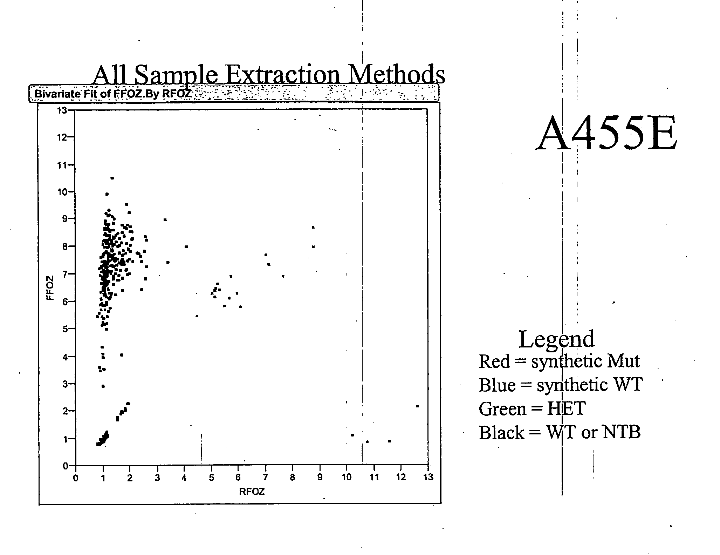

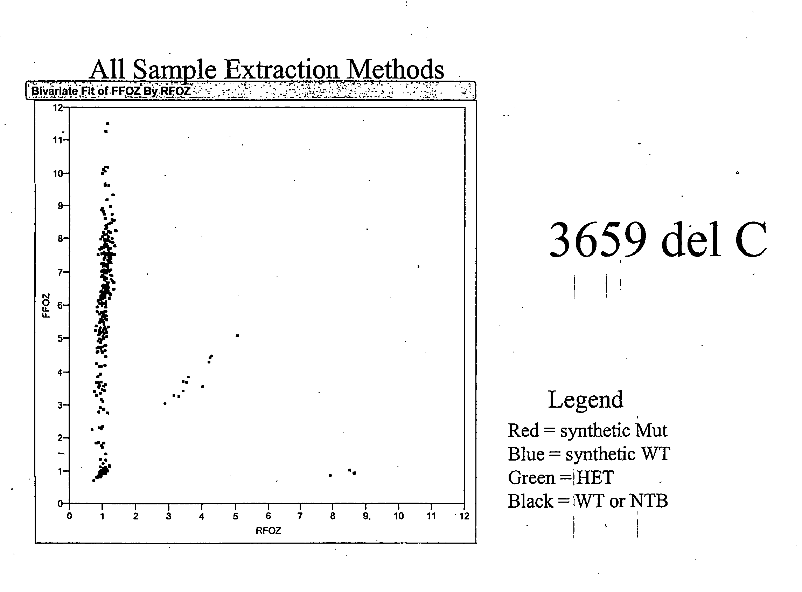

Representative results for the analysis of these three alleles are shown in Figure 7. These results indicate that the INVADER assays can distinguish homozygous wild type, heterozygous, and homozygous mutant sequences at all three loci. Similar results have been obtained with assays carried out on between 96-288 genomic DNA samples purified as described above and at least one heterozygous and one homozygous mutant sample, either genomic DNA obtained from Coriel, synthetic DNA or plasmid DNA (i.e. if no Coriel sample was available with the desired genotype), to detect additional CFTR variants using oligonucleotide sequences included in Figure 3. Exemplary criteria for making genotype calls from such experiments are as follows:

| Ratio | FamFOZ | RedFOZ | Genotype |
|---|---|---|---|
| >=5 | >=1.75 | NA | WT |
| >=0,5 and <=2 | >=1.75 | >=1.75 | Het |
| <=0.2 | NA | >=1.75 | Mut |

For assays to detect 2184delA, the Net FOZ is calculated as follows:

FAM FOZ-1 for Net FAM FOZ
RED FOZ-1.2 for Net RED FOZ

For assays to detect N1303K, the Net FOZ is calculated as follows:

FAM FOZ-1.1 for Net FAM FOZ
RED FOZ-1 for Net RED FOZ.

These values are then used to calculate ratios as described above.

Alternatively, the data can be viewed and assessed graphically as in Figure 7.

### Example 8

### Discrimination of the 5T/7T/9T polymorphism

A variant in the polypyrimidine tract of intron 8, often referred to as IVS-8 5T/7T/9T, has been associated with adverse phenotypes when present with additional CFTR mutations, especially the R117H mutation (Richards, C.S. et al., Genetics in Medicine, (2002) 4: 379-391; Strom, C.M. et al., Genetics in Medicine, (2002) 4: 289-296). In particular, the 5T allele in cis with R117H makes R117H a deleterious CF allele (Strom et al, supra). Homozygosity for the 5T variant has been associated with CBAVD (congential absence of the vas deferens). There may be further clinical significance of the 5T allele in trans with other CF alleles (Strom et al., supra). INVADER and probe oligonucleotides were designed to detect and discriminate these three alleleic variants of IVS8. In the present example, each assay was run in a separate well (i.e. 5T, 7T, and 9T) and was biplexed with an oligonucleotide set designed to detect an internal control sequence in the 3' UTR (untranslated region) of the CFTR gene. Reactions were carried out as described in Example 7. In order to ensure inclusion of known genotypic variations in the vicinity of this locus, multiple INVADER oligonucleotides were included in the three different reactions. The following table indicates which oligonucleotides were included in these reactions.

Reactions comprised standard INVADER assay reagents as in Example 7, except that individual probes for each assay were combined with multiple INVADER oligos were included as follows:

| Oligo | 5T | 7T | 9T |
|---|---|---|---|
| Probe (10 μM) | SEQ ID NO: 273 | SEQ ID NO:274 | SEQ ID NO: 275 |
| INVADER oligo (0.1 μM) | SEQ ID NO: 276 | SEQ ID NO: 276 | SEQ ID NO: 276 |
| INVADER oligo (0.1 μM) | SEQ ID NO: 277 | SEQ ID NO: 277 | SEQ ID NO: 277 |
| INVADER oligo (0.1 μM) | SEQ ID NO: 278 | SEQ ID NO: 278 | SEQ ID NO: 278 |
| INVADER oligo (0.1 μM) | SEQ ID NO: 279 | SEQ ID NO: 279 | SEQ ID NO: 279 |
| INVADER oligo (0.1 μM) | SEQ ID NO: 280 | SEQ ID NO: 280 | SEQ ID NO: 280 |
| FAM FRET (5μM) | SEQ ID NO: 281 | SEQ ID NO: 281 | SEQ ID NO: 281 |
| IC probe (10μM) | SEQ ID NO: 368 | SEQ ID NO: 368 | SEQ ID NO: 368 |
| IC INVADER oligo (1μM) | SEQ ID NO: 367 | SEQ ID NO: 367 | SEQ ID NO: 367 |
| RED FRET (5μM) | SEQ ID NO: 372 | SEQ ID NO: 372 | SEQ ID NO: 372 |

From each of the tests (5T biplexed with IC, 7T biplexed with IC, and 9T biplexed with IC), FOZ ratios were calculated as follows:

$$FOZ = \frac{\text{Raw counts from sample}}{\text{Raw counts from No Target Blank}}$$

Using the oligos in Figure 3 for the 5T/7T/9T assay, it has been seen in previous experiments that a positive result in the 9T assay may also be indicative of the presence of the 7T allele. Therefore, alternative means of manipulating the FOZs obtained from these assays have been contemplated to use the combined output of all three assays to arrive at genotype determinations. In one approach, ratios of the adj Net FOZ values (FOZ-1, unless FOZ-1<0.15, then use 0.15) of the 9T and 7T assays relative to the adj Net FOZ values of their Ics were used to calculate a 9T/7T ratio and the two values compared in order to arrive at the poly T genotype, calls for all three alleles as follows: In all cases "+" indicates a ratio 1.75 of the FOZ of the test assay (i.e. 5T, 7T, or 9T) . NA=not applicable.

| Genotype | 5T FOZ | 7T FOZ | 9T FOZ | 9T/7T ratio |
|---|---|---|---|---|
| 5T/5T | + | - | - | NA |
| 5T/9T | + | - | + | ≥5.16 |
| 5T/7T | + | + | +/- | ≤ 0.67 |
| 7T/7T | - | + | +/- | ≤ 0.67 |
| 7T/9T 9T/9T | - | + | + | 0.99 - 2.24 |
| 9T/9T | - | - | + | ≥5.16 |

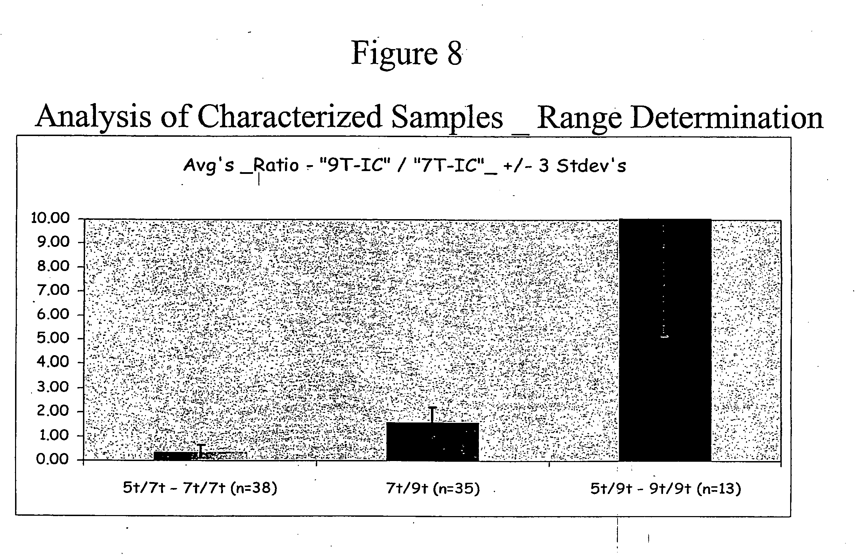

From this analysis, it can be determined whether or not the 7T allele is present.
The 5T and 7T assays in combination with the 9T/7T ratios enables the determination of each genotype. Representative results are presented in Figure 8.
In another approach, the ratio of the 7T adj Net FOZ value (FOZ - 1, unless FOZ-1<0.15, then use 0.15)/IC adj Net FOZ value is subtracted from the ratio of the 9T adj Net FOZ value/IC adj Net FOZ. The relation to zero (i.e., positive or negative) of the difference is compared to the FOZ value for each T allele in order to arrive at the poly T genotype calls for all three alleles as follows:
In all cases "+" indicates a ratio 1.75 of the FOZ of the test assay (i.e. 5T, 7T, or 9T).
NA= not applicable.

| Genotype | 5T FOZ | 7T FOZ | 9T FOZ | 9T-7T difference |
|---|---|---|---|---|
| 5T/5T | + | - | - | NA |
| 5T/9T | + | - | + | Positive |
| 5T/7T | + | + | +/- | Negative |
| 7T/7T | - | + | +/- | Negative |
| 7T/9T | - | + | + | Positive |
| 9T/9T | - | - | + | Positive |

The 5T and 7T assays in combination with the 9T-7T difference enables determination of each genotype. Negative values in the 9T-7T difference column indicate absence of the 9T allele; positive values indicate presence of the 9T allele.

**Example 9**

**Multiplex analysis of limited-cycle PCR reactions**

Multiplexed amplification is a method whereby multiple loci are co-amplified in a single reaction vessel. In some embodiments, such amplified fragments are then subjected to subsequent analysis. This example relates to the analysis of a large collection of loci from a single gene, i.e. CFTR, involving limited cycle multiplex PCR amplification (described in US App. Ser. Nos. 10/321,039, and 60/511,955, and in Example 10) to generate multiple amplicons in a single reaction vessel. Alternative multiplex PCR approaches for analysis of CFTR mutations are described in Makowski, G.S. et al., Ann. Clin. Lab. Sci., (2003) 33: 243-250.

In this example, two different approaches were used to combine limited cycle, multiplexed PCR amplification with subsequent mutation detection by the INVADER assay. In one approach, the genotyping assays described in Example 7 were used to analyze limited-cycle, multiplex PCR products. In the other approach, the pooling assays described in Examples 1 and 2 were used. In both cases, PCR amplification was carried out as follows.

A master mix was made containing all of the primers in the following table at a concentration of 1 μM each. Primers were designed according to the following rules:

1. Primers were selected to amplify all regions of the CFTR gene where one or more mutations are located.
2. Primers were selected to amplify multiple adjacent mutations whenever possible.
3. Primers were selected to minimize amplicon length and never exceed 400 bp. In the cases where multiple mutations were on the same exon but at 1-200 bp from each other, primers were designed to amplify the entire region and also to amplify two separate, smaller regions.
4. Primers were designed to be between 20 and 30 bases long.
5. Primers were selected to NOT have the following 3' sequences, representing the 5' flaps

   ... GAGGX or GAGGXX
   ...GGAGX or ...GGAGXX
   ...GACGX or GACGXX
   ...GACAX or ..>GACAXX

6. Primers were selected to have a Tm of 67.6 °C (min, 67.2 °C, max 68.2 °C) or a Tm using the PRIMERDESIGNER algorithm (described in US Pat. Appl. No: ) of about 58.2 °C.
7. Primers were selected to have one of the following 3' ends

AAA, ACA, ACC, AGA, AAC, CAA, ACA, CCA, CCC, GAC, GGA, GCC, GAA, TAC, TCA, TAA, CAC

8. When several primer options were available, the primer that gave the shortest amplified sequence and/or the one that had a length closest to 25 bases was selected.
9. Primer sequences were checked for accuracy against the Golden Path July 2003 genome assembly.

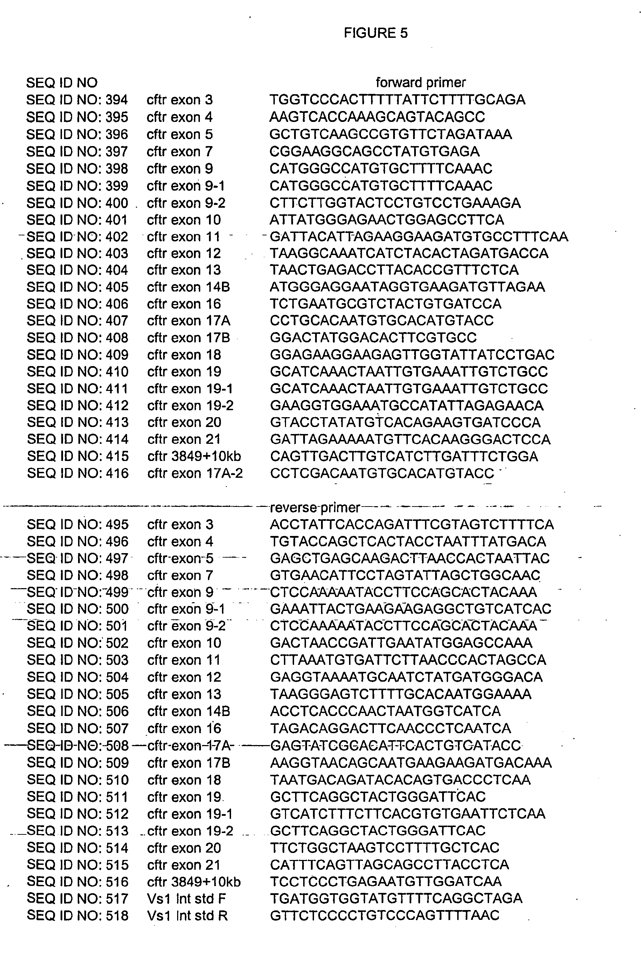

Primers used are listed in Figure 5.
A PCR master mix was made to amplify characterized genomic DNA samples obtained from Coriel, containing the following components and run for 20 cycles of amplification. The number of cycles was chosen based on preliminary experiments to calibrate amplification factor relative to starting DNA quantity. A reduced quantity of DNA (i.e. 2 ng) was added to these experiments relative to previous experiments in which 17 cycles of amplification were applied to starting DNA quantities of 20 ng (as described in U.S. Patent Appl. Ser. No. 60/511955, filed on October 16, 2003).
The Coriell samples were numbered as follows (e.g. "C" n)

**Coriell # Genotype**

1 NA11277 I507 del HET
2 NA11280 711+1G>T/621+1G>T HET
3 NA01531 delF508 HOM
4 NA04539 delF508 HOM
5 NA07381 delF508/3849+10kb HET
6 NA07441 3120+1G>A/621+1G>T HET
7 NA07469 delF508/R553X
8 NA11283 A455E/delF508 HET

**9** NA11284 R560T/delF508 HET
**10** NA11286 delF508/G551D HET
**11** NA11290 A455E/621+1G>T HET
**12** NA07552 delF508/R553X
**13** NA08342 delF508/G551D HET
**14** NA11275 3659delC/delF508 HET
**15** NA12785 G551D/R347P HET
**16** NA11472 G1349D/N1303K HET
**17** NA11496 G542X HOM
**18** NA11497 G542X HET
**19** NA11723 W1282X HET
**20** NA11761 G551D/R553X HET
**21** NA11282 G85E/621+1G>T HET
**22** NA12960 R334W/unknown mutation HET
**23** NA13032 I506V
**24** NA13033 F508C
**25** NA13423 G85E/D1152H HET
**26** NA11859 2789+5G>A HOM
**27** NA11860 3849+10kb HOM
**28** NA12444 1717-1G>A HET
**29** NA12585 R1162X HET
**30** NA13591 delF508/R117H HET
**31** NA08338 G551D
**32** NA11281 621+1G>T/delF508
**34** NA12961 V520F
**35** NA11278 Q493X/delF508
**36** NA11285 Y1092X (C>A)/delF508
**38** NA00946 AN1
**39** NA00130 AN2

**Materials:**

1. Genomic DNAs, 10ng/$\mu$l NON denatured. (Dilute to 1ng/$\mu$l below). 2ng genomic DNA were added per 20$\mu$l reaction
2. tRNA, 30$\mu$g/ml
3. dNTPs 1.25mM.
4. 10x PCR buffer PE biosystems lot: D09868
5. Taq polymerase (Amplitaq- PE Biosystems) lot: D09775
6. Taqstart antibody S 1476, lot 3040648
7. Thinwalled tubes
8. Te (negative control)
9. nuclease-free water
10. 20plex CFTR primer pairs, 1$\mu$M each

**Methods:**

1. Dilute genomic DNAs 1:10 in Te. (20$\mu$l of 10ng/$\mu$l + 180$\mu$l Te)= 1ng/$\mu$l
2. Prepare PCR mixes minus 2$\mu$l genomic DNA:

| A. Taq Ab | | 20ulrxn | | |
|---|---|---|---|---|
| | | 1x | | 40x |
| taq | | 0.4 | ul | 16 |
| Taqstart ab | | 0.4 | ul | 16 |

(continued)

| A. Taq Ab | | 20ulrxn | | |
|---|---|---|---|---|
| water | | 8 | ul | 320 |
| dNTPs | | 3.2 | ul | 128 |
| 10xPCR buffer | | 2 | ul | 80 |
| CFTR 20plex | | 4 | ul | 160 |
| primers | | | | |
| | | 18 | ul | 720 |

Divide into 39 wells of an MJ plate, 18μl each

To each well add 2μl 1ng/μl genomic DNA or Te

Cover all with 15ul chill out and foil

C "n" refers to the numbers of the genomic DNA samples.

| | 2 | 3 | 4 | 5 | 6 | 7 | 8 | 9 | 10 | 11 |
|---|---|---|---|---|---|---|---|---|---|---|
| **A** | C1 | | C3 | C4 | C5 | C6 | C7 | C8 | C9 | C10 |
| **B** | | | | | | | | | | |
| **C** | C11 | C12 | C13 | C14 | C15 | C16 | C17 | C18 | C19 | C20 |
| **D** | | | | | | | | | | |
| **E** | C21 | C22 | C23 | C24 | C25 | C26 | C27 | C28 | C29 | C30 |
| **F** | | | | | | | | | | |
| **G** | C31 | C32 | | C34 | C35 | C36 | | Te | Te | Te |
| **H** | | | | | C14, 20ng | | | | | |

**Incubate** plate "PCR20"

|  |  |
|---|---|
| 95C | 2.5 min |
|  |  |
| 95C | 30 sec |
| 55C | 1.5 min |
| 72C | 2.5 min |
|  | **x20 cycles** |
| 99C | 10 min |
| 10C | hold |

**Sample dilution**

1. Bring plates to room temp to melt chill out.
Dilute samples 1:5:

to each 20μl PCR reaction add 80 μl **Te**=100μl of 1:5. Bring plates to 4C to harden chill out wax.

2. Further dilute samples (in 96well reaction plate):

$$1:10 \ (15\mu l \ of \ 1:5 + 135\mu l \ \textbf{Te}) = 150\mu l \ of \ 1:50$$

Cover plates tightly with foil and heat all 95C 5 min; cool to room temp.

Dilution plate layout, (duplicate dilutions):

|   | 1 | 2 | 3 | 4 | 5 | 6 | 7 | 8 | 9 | 10 | 11 | 12 |
|---|---|---|---|---|---|---|---|---|---|----|----|----|
| A |   | C1 | C10 | C18 | C26 | C35 | C1 | C10 | C18 | C26 | C35 |   |
| B |   | C3 | C11 | C19 | C27 | C36 | C3 | C11 | C19 | C27 | C36 |   |
| C |   | C4 | C12 | C20 | C28 | C14 20ng | C4 | C12 | C20 | C28 | C14 20ng |   |
| D |   | C5 | C13 | C21 | C29 | NTC | C5 | C13 | C21 | C29 | NTC |   |
| E |   | C6 | C14 | C22 | C30 |   | C6 | C14 | C22 | C30 |   |   |
| F |   | C7 | C15 | C23 | C31 |   | C7 | C15 | C23 | C31 |   |   |
| G |   | C8 | C16 | C24 | C32 |   | C8 | C16 | C24 | C32 |   |   |
| H |   | C9 | C17 | C25 | C34 |   | C9 | C17 | C25 | C34 |   |   |

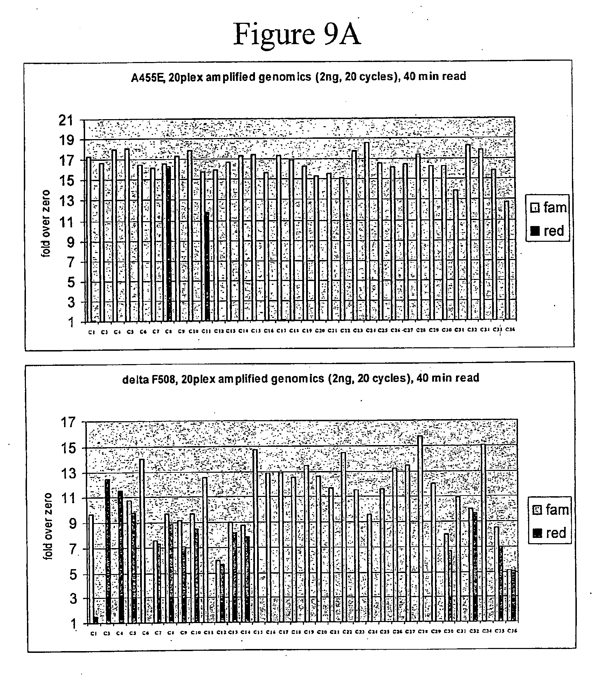

PCR reaction products were diluted 1:50 in Te prior to testing by the INVADER assay in 96 well microtiter plates. Aliquots of 10 $\mu$l of a master mix containing two probe and one INVADER oligo, two FRET cassettes, CLEAVASE X, MgCl2, and MOPS at the final concentrations indicated in Example 7 were added to the appropriate wells. The probe, INVADER, and FRET cassette sequences were as indicated in Figure 3 for the various alleles tested. Aliquots of 10 $\mu$l of denatured, diluted PCR products were added to the appropriate wells, the reactions were overlaid with 15 $\mu$l mineral oil and incubated at 63°C. Reactions were read in a CYTOFLUOR fluorescence microplate reader at 20 and 40 minute intervals. Representative results are presented in Figure 9A. Comparison of these and other similar results for the other alleles tested indicated that there was complete concordance between the genotype determinations made using the INVADER assay to analyze PCR amplified targets and the genotypes as described by Coriel. Similar results were obtained with PCR reactions run for 17 cycles.

Additional experiments were carried out on 2 mm punches from FTA cards spotted with human blood samples. Paper punches containing blood spots were treated in several different ways as follows:

Extraction variations, each using one 2mm punch. Sets of 5 tubes at each condition

1. water, 3x150$\mu$l (15'each),
2. water +0.2% Tween-20, 2x150$\mu$l (15'each), water, 1x150$\mu$l
3. FTA purification reagent, 2x150$\mu$l (15'each), water, 1x150$\mu$l
4. PBS, 2x150$\mu$l (3' each); water 1x150$\mu$l,
5. PBS +0.2% Tween-20, 2x150$\mu$l (3' each), 1x150$\mu$l water,

Test samples were treated as follows:

1-5: Washing done at room temp.
6-10: Washing done at 37C
11-15: Washing done at room temp.
16-20: Washing done at 37C
21-22: Punch from clear area (no blood). Washed 3 X 150 $\mu$l, 15 min each, in water.

After last step drain well and cap. Hold over weekend at 4C.

Each treated punch was added to PCR reactions as follows.

Use one 2mm punch per 10$\mu$l reaction, with 17 and 20 cycles of amplification. Dilute reactions 1:10 and use 2 and 4$\mu$l in 20$\mu$l invader reactions.

**Materials:**

11. Blood card punches

1, 11
2, 12
3, 13
6, 16
7, 17
8, 18
21,22 (NTC)

12. tRNA, 30μg/ml pN 21319 lot 3050121
13. dNTPs 1.25mM 1591-56
14. 10x PCR buffer PE biosystems lot: D09868
15. Taq polymerase (Amplitaq- PE Biosystems) lot: D09775
16. Taqstart ab S1476 3040648
17. Thinwalled tubes
18. Te (-control) 21004 A156414
19. nuclease-free water
20. 20plex CFTR primer pairs, 1μM each diluted on 1789-06
21. G2, 10ng/μl

**Methods:**

1. Prepare PCR mixes minus 2μl genomic DNA:

| A. Taq Ab | | 10ulrxn | | |
|---|---|---|---|---|
| | | **1x** | | **18x** |
| taq | | 0.2 | ul | 3.6 |
| Taqstart ab | | 0.2 | ul | 3.6 |
| water | | 3 | ul | 54 |
| dNTPs | | 1.6 | ul | 28.8 |
| 10xPCR buffer | | 1 | ul | 18 |
| CFTR 20plex primers | | 2 | ul | 36 |
| | | **8** | **ul** | **144** |

Divide into 14 thin walled tubes with wet punches, 8μl reaction mix each

To tubes 8 and 16 add 2μl genomic DNA (G2, 10ng/μl)

Cover all with 15μl chill out

| | **punch** | **cycles** |
|---|---|---|
| **1** | 1 | 17 |
| **2** | 2 | 17 |
| **3** | 3 | 17 |
| **4** | 6 | 17 |
| **5** | 7 | 17 |
| **6** | 8 | 17 |
| **7** | NTC | 17 |
| **8** | purified genomic | 17 |

(continued)

| | punch DNA (10ng/$\mu$l) | cycles |
|---|---|---|
| **9** | 11 | 20 |
| **10** | 12 | 20 |
| **11** | 13 | 20 |
| **12** | 16 | 20 |
| **13** | 17 | 20 |
| **14** | 18 | 20 |
| **15** | NTC | 20 |
| **16** | purified genomic DNA (10ng/$\mu$l) | 20 |

**Incubate** tubes 1-8 "PCR-NJ"

| 95C | 2.5 min |
|---|---|
| 95C | 30 sec |
| 55C | 1.5 min |
| 72C | 2.5 min |
| | **x17 cycles** |
| 99C | 10 min |
| 10C | hold |

**Incubate** tubes 9-16 "PCR20"

| 95C | 2.5 min |
|---|---|
| 95C | 30 sec |
| 55C | 1.5 min |
| 72C | 2.5 min |
| | **20 cycles** |
| 99C | 10 min |
| 10C | hold |

**Sample dilution**

Bring plates to room temp to melt chill out.
Dilute samples 1:10:

to each 10$\mu$l PCR reaction add 90 $\mu$l **Te**=100$\mu$l of 1:10.

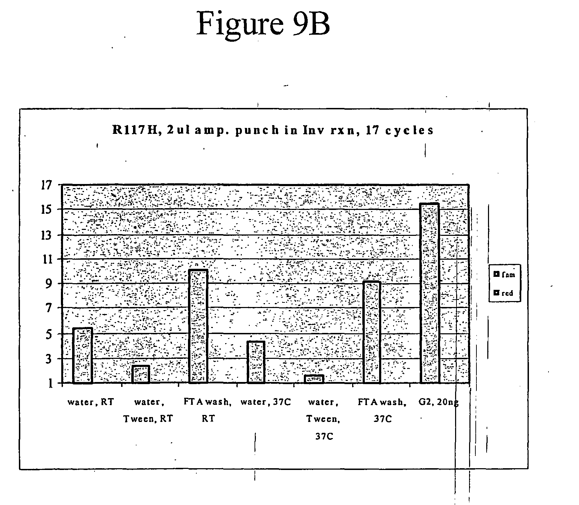

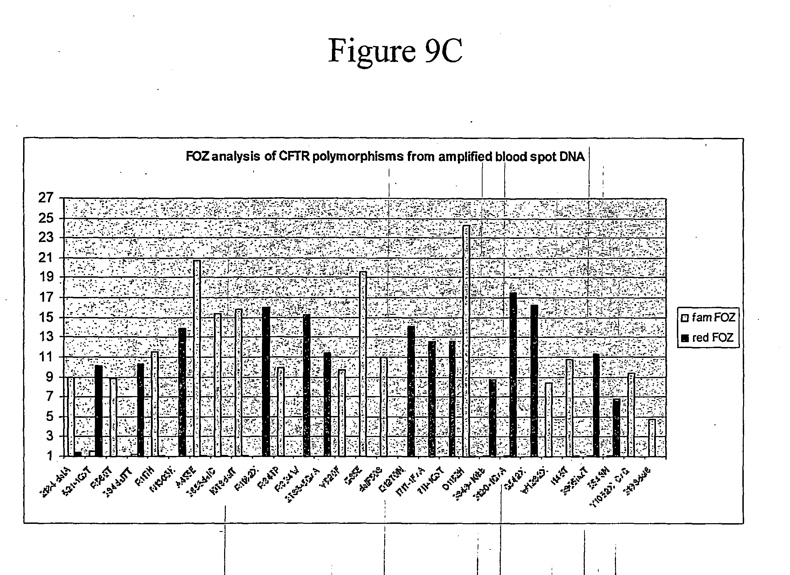

Cap tubes tightly and heat all 95C 5 min; cool to room temp.
INVADER assays were run as described above using appropriate probe, INVADER, and FRET oligonucleotides from Figure 3. Results presented in Figure 9B indicate the performance in representative INVADER assays of the punches treated in the various ways described. Representative results obtained with FTA punches washed under condition 3 (FTA wash buffer at room temperature) are presented in Figure 9C and were concordant with previously established genotypes for each sample. These results indicate that the INVADER assay can be applied to blood samples obtained from FTA cards and amplified in a limited cycle, multiplex PCR reaction.
Similar experiments were carried out using pooled INVADER assays as described in Examples 1 and 2. In this case, as in Examples 1 and 2, INVADER reactions are directed to mutant alleles of select CFTR loci and to an internal control sequence. PCR primers were designed to amplify the internal control sequence detected using INVADER and probe oligos in Figure 5. Note that in the experiments described above, SEQ ID NO: 407 was used as the forward primer for exon 17A. In this experiment, SEQ ID NO: 416 was used. The PCR primers for the internal control were as follows:

Vs1 Int std F (SEQ ID NO: 517) TGATGGTGGTATGTTTTCAGGCTAGA
Vs1 Int std R (SEQ ID NO: 518) GTTCTCCCCTGTCCCAGTTTTAAC

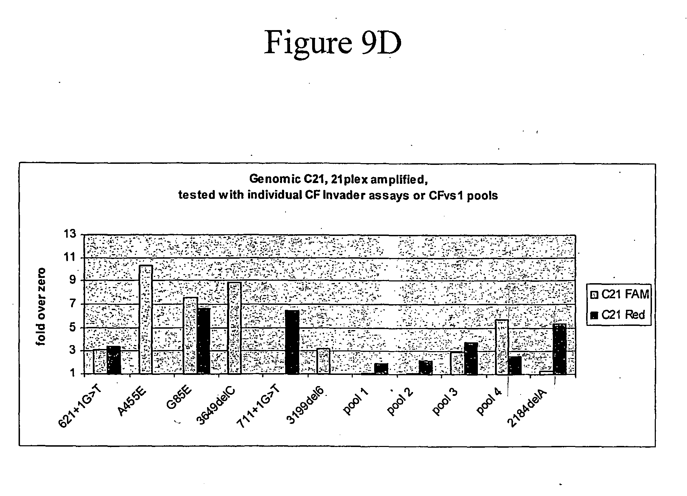

21-plex PCR reactions were carried out as described above, for 17 cycles, with the addition of the internal control primer pair. Amplicons were diluted 1:20 in Te prior to analysis in the pooled INVADER assays of Example 2 as well as a select subset of the genotyping assays as described above and as indicated in the figure. Reactions were run with either 2 or 4 μl of the 1:20 diluted multiplex PCR reactions in the appropriate wells. Representative results are presented in Figure 9D and indicate that in each case, the INVADER assay was able to discriminate the presence of a mutant allele, e.g. in pool 3 (621+1G>T) and pool 4 (G85E). Moreover, the internal control was detected in each pooled reaction. As for assays carried out directly from genomic DNA, there is a desire when using amplified target DNA to confirm that an appropriate amount of DNA is added to the INVADER reactions such that the internal control signal is within the indicated ranges for making valid determinations (examples of such ranges are presented in Examples 1 and 2).

## EXAMPLE 10

## MULTIPLEX NUCLEIC ACID DETECTION ASSAYS

The present invention provides methods for developing and optimizing nucleic acid detection assays for use in basic research, clinical research, and for the development of clinical detection assays. In particular, the present invention provides methods for designing oligonucleotide primers to be used in multiplex amplification reactions. The present invention also provides methods to optimize multiplex amplification reactions. The present invention also provides methods to perform Highly Multiplexed PCR in Combination with the INVADER Assay.

With the completion of the nucleic acid sequencing of the human genome, the demand for fast, reliable, cost-effective and user-friendly tests for genomics research and relateddrug design efforts has greatly increased. A number of institutions are actively mining the available genetic sequence information to identify correlations between genes, gene expression and phenotypes (e.g., disease states, metabolic responses, and the like). These analyses include an attempt to characterize the effect of gene mutations and genetic and gene expression heterogeneity in individuals and populations. However, despite the wealth of sequence information available, information on the frequency and clinical relevance of many polymorphisms and other variations has yet to be obtained and validated. For example, the human reference sequences used in current genome sequencing efforts do not represent an exact match for any one person's genome. In the Human Genome Project (HGP), researchers collected blood (female) or sperm (male) samples from a large number of donors. However, only a few samples were processed as DNA resources, and the source names are protected so neither donors nor scientists know whose DNA is being sequenced. The human genome sequence generated by the private genomics company Celera was based on DNA samples collected from five donors who identified themselves as Hispanic, Asian, Caucasian, or African-American. The small number of human samples used to generate the reference sequences does not reflect the genetic diversity among population groups and individuals. Attempts to analyze individuals based on the genome sequence information will often fail. For example, many genetic detection assays are based on the hybridization of probe oligonucleotides to a target region on genomic DNA or mRNA. Probes generated based on the reference sequences will often fail (e.g., fail to hybridize properly, fail to properly characterize the sequence at specific position of the target) because the target sequence for many individuals differs from the reference sequence. Differences maybe on an individual-by-individual basis, but many follow regional population patterns (e.g., many correlate highly to race, ethnicity, geographic local, age, environmental exposure, etc.). With the limited utility of information currently available, the art is in need of systems and methods for acquiring, analyzing, storing, and applying large volumes of genetic information with the goal of providing an array of detection assay technologies for research and clinical analysis of biological samples.

The present invention provides methods and routines for developing and optimizing nucleic acid detection assays for use in-basic research, clinical research, and for the development of clinical detection assays.

In some embodiments, the present invention provides methods comprising; a) providing target sequence information for at least Y target sequences, wherein each of the target sequences comprises; i) a footprint region, ii) a 5' region immediately upstream of the footprint region, and iii) a 3' region immediately downstream of the footprint region, and b) processing the target sequence information such that a primer set is generated, wherein the primer set comprises a forward and a reverse primer sequence for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide A or C, and N[2]-N[1]-3' of each of the forward and reverse primers is not complementary to N[2]-N[1]-3' of any of the forward and reverse primers in the primer set.

In other embodiments, the present invention provides methods comprising; a) providing target sequence information for at least Y target sequences, wherein each of the target sequences comprises; i) a footprint region, ii) a 5' region immediately upstream of the footprint region, and iii) a 3' region immediately downstream of the footprint region, and b) processing the target sequence information such that a primer set is generated, wherein the primer set comprises a forward and a reverse primer sequence for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide G or T, and N[2]-N[1]-3' of each of the forward and reverse primers is not complementary to N[2]-N[1]-3' of any of the forward and reverse primers in the primer set.

In particular embodiments, a method comprising; a) providing target sequence information for at least Y target sequences, wherein each of the target sequences comprises; i) a footprint region, ii) a 5' region immediately upstream of the footprint region, and iii) a 3' region immediately downstream of the footprint region, and b) processing the target sequence information such that a primer set is generated, wherein the primer set comprises; i) a forward primer sequence identical to at least a portion of the 5' region for each of the Y target sequences, and ii) a reverse primer sequence identical to at least a portion of a complementary sequence of the 3' region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide A or C, and N[2]-N[1]-3' of each of the forward and reverse primers is not complementary to N[2]-N[1]-3' of any of the forward and reverse primers in the primer set.

In other embodiments, the present invention provides methods comprising a) providing target sequence information for at least Y target sequences, wherein each of the target sequences comprises; i) a footprint region, ii) a 5' region immediately upstream of the footprint region, and iii) a 3' region immediately downstream of the footprint region, and b) processing the target sequence information such that a primer set is generated, wherein the primer set comprises; i) a forward primer sequence identical to at least a portion of the 5' region for each of the Y target sequences, and ii) a reverse primer sequence identical to at least a portion of a complementary sequence of the 3' region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide G or T, and N[2]-N[1]-3' of each of the forward and reverse primers is not complementary to N[2]-N[1]-3' of any of the forward and reverse primers in the primer set.

In particular embodiments, the present invention provides methods comprising a) providing target sequence information for at least Y target sequences, wherein each of the target sequences comprises a single nucleotide polymorphism, b) determining where on each of the target sequences one or more assay probes would hybridize in order to detect the single nucleotide polymorphism such that a footprint region is located on each of the target sequences, and c) processing the target sequence information such that a primer set is generated, wherein the primer set comprises; i) a forward primer sequence identical to at least a portion of the target sequence immediately 5' of the footprint region for each of the Y target sequences, and ii) a reverse primer sequence identical to at least a portion of a complementary sequence of the target sequence immediately 3' of the footprint region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide A or C, and N[2]-N[1]-3' of each of the forward and reverse primers is not complementary to N[2]-N[1]-3' of any of the forward and reverse primers in the primer set.

In some embodiments, the present invention provides methods comprising a) providing target sequence information for at least Y target sequences, wherein each of the target sequences comprises a single nucleotide polymorphism, b) determining where on each of the target sequences one or more assay probes would hybridize in order to detect the single nucleotide polymorphism such that a footprint region is located on each of the target sequences, and c) processing the target sequence information such that a primer set is generated, wherein the primer set comprises; i) a forward primer sequence identical to at least a portion of the target sequence immediately 5' of the footprint region for each of the Y target sequences, and ii) a reverse primer sequence identical to at least a portion of a complementary sequence of the target sequence immediately 3' of the footprint region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide T or G, and N[2]-N[1]-3' of each of the forward and reverse primers is not complementary to N[2]-N[1]-3' of any of the forward and reverse primers in the primer set.

In certain embodiments, the primer set is configured for performing a multiplex PCR reaction that amplifies at least Y amplicons, wherein each of the amplicons is defined by the position of the forward and reverse primers. In other embodiments, the primer set is generated as digital or printed sequence information. In some embodiments, the primer set is generated as physical primer oligonucleotides.

In certain embodiments, N[3]-N[2]-N[1]-3' of each of the forward and reverse primers is not complementary to N[3]-N[2]-N[1]-3' of any of the forward and reverse primers in the primer set. In other embodiments, the processing comprises initially selecting N[1] for each of the forward primers as the most 3' A or C in the 5' region. In certain embodiments, the processing comprises initially selecting N[1] for each of the forward primers as the most 3' G or T in the 5' region. In some embodiments, the processing comprises initially selecting N[1] for each of the forward primers as the most 3' A or C in the 5' region, and wherein the processing further comprises changing the N[1] to the next most 3' A or C in the 5' region for the forward primer sequences that fail the requirement that each of the forward primer's N[2]-N[1]-3' is not complementary to N[2]-N[1]-3' of any of the forward and reverse primers in the primer set.

In other embodiments, the processing comprises initially selecting N[1] for each of the reverse primers as the most 3' A or C in the complement of the 3' region. In some embodiments, the processing comprises initially selecting N[1] for each of the reverse primers as the most 3' G or T in the complement of the 3' region. In further embodiments, the processing comprises initially selecting N[1] for each of the reverse primers as the most 3' A or C in the 3' region, and wherein the processing further comprises changing the N[1] to the next most 3' A or C in the 3' region for the reverse primer sequences that fail the requirement that each of the reverse primer's N[2]-N[1]-3' is not complementary to N[2]-N[1]-3' of any of the forward and reverse primers in the primer set.

In particular embodiments, the footprint region comprises a single nucleotide polymorphism. In some embodiments, the footprint comprises a mutation. In some embodiments, the footprint region for each of the target sequences comprises a portion of the target sequence that hybridizes to one or more assay probes configured to detect the single nucleotide polymorphism. In certain embodiments, the footprint is this region where the probes hybridize. In other embodiments, the footprint further includes additional nucleotides on either end.

In some embodiments, the processing further comprises selecting N[5]-N[4]-N[3]-N[2]-N[1]-3' for each of the forward and reverse primers such that less than 80 percent homology with a assay component sequence is present. In preferred embodiments, the assay component a FRET probe sequence. In certain embodiments, the target sequence is about 300-500 base pairs in length, or about 200-600 base pair in length. In certain embodiments, Y is an integer between 2 and 500, or between 2-10,000.

In certain embodiments, the processing comprises selecting x for each of the forward and reverse primers such that each of the forward and reverse primers has a melting temperature with respect to the target sequence of approximately 50 degrees Celsius (e.g. 50 degrees, Celsius, or at least 50 degrees Celsius, and no more than 55 degrees Celsius). In preferred embodiments, the melting temperature of a primer (when hybridized to the target sequence) is at least 50 degrees Celsius, but at least 10 degrees different than a selected detection assay's optimal reaction temperature.

In some embodiments, the forward and reverse primer pair optimized concentrations are determined for the primer set. In other embodiments, the processing is automated. In further embodiments, the processing is automated with a processor.

In other embodiments, the present invention provides a kit comprising the primer set generated by the methods of the present invention, and at least one other component. (e.g. cleavage agent, polymerase, INVADER oligonucleotide). In certain embodiments, the present invention provides compositions comprising the primers and primer sets generated by the methods of the present invention.

In particular embodiments, the present invention provides methods comprising; a) providing; i) a user interface configured to receive sequence data, ii) a computer system having stored therein a multiplex PCR primer software application, and b) transmitting the sequence data from the user interface to the computer system, wherein the sequence data comprises target sequence information for at least Y target sequences, wherein each of the target sequences comprises; i) a footprint region, ii) a 5' region immediately upstream of the footprint region, and iii) a 3' region immediately downstream of the footprint region, and c) processing the target sequence information with the multiplex PCR primer pair software application to generate a primer set, wherein the primer set comprises; i) a forward primer sequence identical to at least a portion of the target sequence immediately 5' of the footprint region for each of the Y target sequences, and ii) a reverse primer sequence identical to at least a portion of a complementary sequence of the target sequence immediately 3' of the footprint region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide A or C, and N[2]-N[1]-3' of each of the forward and reverse primers is not complementary to N[2]-N[1]-3' of any of the forward and reverse primers in the primer set.

In some embodiments, the present invention provides methods comprising; a) providing;

i) a user interface configured to receive sequence data, ii) a computer system having stored therein a multiplex PCR primer software application, and b) transmitting the sequence data from the user interface to the computer system, wherein the sequence data comprises target sequence information for at least Y target sequences, wherein each of the target sequences comprises; i) a footprint region, ii) a 5' region immediately upstream of the footprint region, and iii) a 3' region immediately downstream of the footprint region, and c) processing the target sequence information with the multiplex PCR primer pair software application to generate a primer set, wherein the primer set comprises; i) a forward primer sequence identical to at least a portion of the target sequence immediately 5' of the footprint region for each of the Y target sequences, and ii) a reverse primer sequence identical to at least a portion of a complementary sequence of the target sequence immediately 3' of the footprint region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide G or T, and -N[2]-N[1]-3' of each of the forward and reverse primers in not complementary to N[2]-N[1]-3' of any of the forward and reverse primers in the primer set.

In certain embodiments, the present invention provides systems comprising; a) a computer system configured to receive data from a user interface, wherein the user interface is configured to receive sequence data, wherein the sequence data comprises target sequence information for at least Y target sequences, wherein each of the target sequences comprises; i) a footprint region, ii) a 5' region immediately upstream of the footprint region, and iii) a 3' region immediately downstream of the footprint region, b) a multiplex PCR primer pair software application operably linked to the user interface, wherein the multiplex PCR primer software application is configured to process the target sequence information to generate a primer set, wherein the primer set comprises; i) a forward primer sequence identical to at least a portion of the target sequence immediately 5' of the footprint region for each of the Y target sequences, and ii) a reverse primer sequence identical to at least a portion of a complementary sequence of the target sequence immediately 3' of the footprint region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide A or C, and N[2]-N[1]-3' of each of the forward and reverse primers is not complementary to N[2]-N[1]-3' of any of the forward and reverse primers in the primer set, and c) a computer system having stored therein the multiplex PCR primer pair software application, wherein the computer system comprises computer memory and a computer processor.

In other embodiments, the present invention provides systems comprising; a) a computer system configured to receive data from a user interface, wherein the user interface is configured to receive sequence data, wherein the sequence data comprises target sequence information for at least Y target sequences, wherein each of the target sequences comprises; i) a footprint region, ii) a 5' region immediately upstream of the footprint region, and iii) a 3' region immediately downstream of the footprint region, b) a multiplex PCR primer pair software application operably linked to the user interface, wherein the multiplex PCR primer software application is configured to process the target sequence information to generate a primer set, wherein the primer set comprises; i) a forward primer sequence identical to at least a portion of the target sequence immediately 5' of the footprint region for each of the Y target sequences, and ii) a reverse primer sequence identical to at least a portion of a complementary sequence of the target sequence immediately 3' of the footprint region for each of the at least Y target sequences, wherein each of the forward and reverse primer sequences comprises a nucleic acid sequence represented by 5'-N[x]-N[x-1]- ....-N[4]-N[3]-N[2]-N[1]-3', wherein N represents a nucleotide base, x is at least 6, N[1] is nucleotide G or T, and N[2]-N[1]-3' of each of the forward and reverse primers is not complementary to N[2]-N[1]-3' of any of the forward and reverse primers in the primer set, and c) a computer system having stored therein the multiplex PCR primer pair software application, wherein the computer system comprises computer memory and a computer processor. In certain embodiments, the computer system is configured to return the primer set to the user interface.

Since its introduction in 1988 (Chamberlain, et al. Nucleic Acids Res., 16:11141 (1988)), multiplex PCR has become a routine means of amplifying multiple genetic loci in a single reaction. This approach has found utility in a number of research, as well as clinical, applications. Multiplex PCR has been described for use in diagnostic virology (Elnifro, et al. Clinical Microbiology Reviews, 13: 559 (2000)), paternity testing (Hidding and Schmitt, Forensic Sci. Int., 113: 47 (2000); Bauer et al., Int. J. Legal Med. 116: 39 (2002)), preimplantation genetic diagnosis (Ouhibi, et al., Curr Womens Health Rep. 1: 138 (2001)), microbial analysis in environmental and food samples (Rudi et al., Int J Food Microbiology, 78: 171 (2002)), and veterinary medicine (Zarlenga and Higgins, Vet Parasitol. 101: 215 (2001)), among others. Most recently, expansion of genetic analysis to whole genome levels, particularly for single nucleotide polymorphisms, or SNPs, has created a need highly multiplexed PCR capabilities. Comparative genome-wide association and candidate gene studies require the ability to genotype between 100,000-500,000 SNPs per individual (Kwok, Molecular Medicine Today, 5: 538-5435 (1999); Kwok, Pharmacogenomics, 1: 231 (2000); Risch and Merikangas, Science, 273: 1516 (1996)). Moreover, SNPs in coding or regulatory regions alter gene function in important ways (Cargill et al. Nature Genetics, 22: 231 (1999); Halushka et al., Nature Genetics, 22: 239 (1999)), making these SNPs useful diagnostic tools in personalized medicine (Hagmann, Science, 285: 21 (1999); Cargill et al. Nature Genetics, 22: 231 (1999); Halushka et al., Nature Genetics, 22: 239 (1999)). Likewise, validating the medical association of a set of SNPs previously identified for their potential clinical relevance as part of a diagnostic panel will mean testing thousands of individuals for thousands of markers at a time.

Despite its broad appeal and utility, several factors complicate multiplex PCR amplification. Chief among these is the phenomenon of PCR or amplification bias, in which certain loci are amplified to a greater extent than others. Two classes of amplification bias have been described. One, referred to as PCR drift, is ascribed to stochastic variation in such steps as primer annealing during the early stages of the reaction (Polz and Cavanaugh, Applied and Environmental Microbiology, 64: 3724 (1998)), is not reproducible, and may be more prevalent when very small amounts of target molecules are being amplified (Walsh et al., PCR Methods and Applications, 1: 241 (1992)). The other, referred to as PCR selection, pertains to the preferential amplification of some loci based on primer characteristics, amplicon length, G-C content, and other properties of the genome (Polz, supra).

Another factor affecting the extent to which PCR reactions can be multiplexed is the inherent tendency of PCR reactions to reach a plateau phase. The plateau phase is seen in later PCR cycles and reflects the observation that amplicon generation moves from exponential to pseudo-linear accumulation and then eventually stops increasing. This effect appears to be due to non-specific interactions between the DNA polymerase and the double stranded products themselves. The molar ratio of product to enzyme in the plateau phase is typically consistent for several DNA polymerases, even when different amounts of enzyme are included in the reaction, and is approximately 30:1 product: enzyme. This effect thus limits the total amount of double-stranded product that can be generated in a PCR reaction such that the number of different loci amplified must be balanced against the total amount of each amplicon desired for subsequent analysis, e.g. by gel electrophoresis, primer extension, etc.

Because of these and other considerations, although multiplexed PCR including 50 loci has been reported (Lindblad-Toh et al., Nature Genet. 4: 381 (2000)), multiplexing is typically limited to fewer than ten distinct products. However, given the need to analyze as many as 100,000 to 45,000 SNPs form a single genomic DNA sample there is a clear need for a means of expanding the multiplexing capabilities of PCR reactions.

The present invention provides methods for substantial multiplexing of PCR reactions by, for example, combining the INVADER assay with multiplex PCR amplification. The INVADER assay provides a detection step and signal amplification that allows very large numbers of targets to be detected in a multiplex reaction. As desired, hundreds to thousands to hundreds of thousands of targets may be detected in a multiplex reaction.

Direct genotyping by the INVADER assay typically uses from 5 to 100 ng of human genomic DNA per SNP, depending on detection platform. For a small number of assays, the reactions can be performed directly with genomic DNA without target pre-amplification, however, with more than 100,000 INVADER assays being developed and even larger number expected for genome-wide association studies, the amount of sample DNA may become a limiting factor.

Because the INVADER assay provides from $10^6$ to $10^7$ fold amplification of signal, multiplexed PCR in combination with the INVADER assay would use only limited target amplification as compared to a typical PCR. Consequently, low target amplification level alleviates interference between individual reactions in the mixture and reduces the inhibition of PCR by it's the accumulation of its products, thus providing for more extensive multiplexing. Additionally, it is contemplated that low amplification levels decrease a probability of target cross-contamination and decrease the number of PCR-induced mutations.

Uneven amplification of different loci presents one of biggest challenges in the development of multiplexed PCR. Difference in amplification factors between two loci may result in a situation where the signal generated by an INVADER reaction with a slow-amplifying locus is below the limit of detection of the assay, while the signal from a fast-amplifying locus is beyond the saturation level of the assay. This problem can be addressed in several ways. In some embodiments, the INVADER reactions can be read at different time points, *e.g.*, in real-time, thus significantly extending the dynamic range of the detection. In other embodiments, multiplex PCR can be performed under conditions that allow different loci to reach more similar levels of amplification. For example, primer concentrations can be limited, thereby allowing each locus to reach a more uniform level of amplification. In yet other embodiments, concentrations of PCR primers can be adjusted to balance amplification factors of different loci.

The present invention provides for the design and characteristics of highly multiplex PCR including hundreds to thousands of products in a single reaction. For example, the target pre-amplification provided by hundred-plex PCR reduces the amount of human genomic DNA required for INVADER-based SNP genotyping to less than 0.1 ng per assay. The specifics of highly multiplex PCR optimization and a computer program for the primer design are described below.

The following discussion provides a description of certain preferred illustrative embodiments of the present invention and is not intended to limit the scope of the present invention.

### I. Multiplex PCR Primer Design

The INVADER assay can be used for the detection of single nucleotide polymorphisms (SNPs) with as little as 100-10 ng of genomic DNA without the need for target pre-amplification. However, with more than 50,000 INVADER assays being developed and the potential for whole genome association studies involving hundreds of thousands of SNPs, the amount of sample DNA becomes a limiting factor for large scale analysis. Due to the sensitivity of the INVADER assay on human genomic DNA (hgDNA) without target amplification, multiplex PCR coupled with the INVADER assay requires only limited target amplification ($10^3$-$10^4$) as compared to typical multiplex PCR reactions which require extensive amplification ($10^9$-$10^{12}$) for conventional gel detection methods. The low level of target amplification used for INVADER™ detection provides for more extensive multiplexing by avoiding amplification inhibition commonly resulting from target accumulation.

The present invention provides methods and selection criteria that allow primer sets for multiplex PCR to be generated (e.g. that can be coupled with a detection assay, such as the INVADER assay). In some embodiments, software applications of the present invention automated multiplex PCR primer selection, thus allowing highly multiplexed PCR with the primers designed thereby. Using the INVADER Medically Associated Panel (MAP) as a corresponding platform for SNP detection, as shown in example 2, the methods, software, and selection criteria of the present invention allowed accurate genotyping of 94 of the 101 possible amplicons (~93%) from a single PCR reaction. The original PCR reaction used only 10 ng of hgDNA as template, corresponding to less than 150 pg hgDNA per INVADER assay.

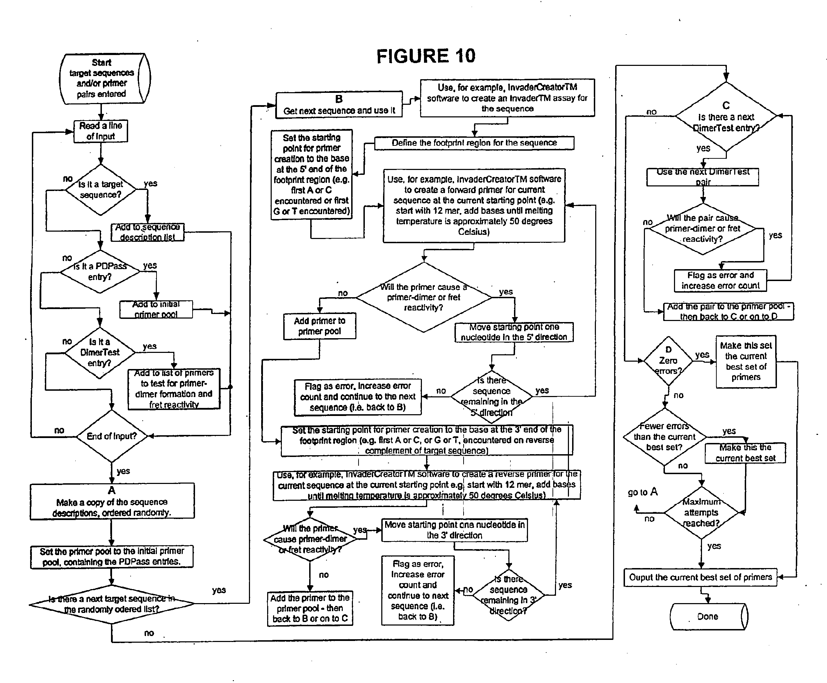

In some embodiments, the selection of primers to make a primer set capable of multiplex PCR is performed in automated fashion (e.g. by a software application). Automated primer selection for multiplex PCR may be accomplished employing a software program designed as shown by the flow chart in Figure 10.

Multiplex PCR commonly requires extensive optimization to avoid biased amplification of select amplicons and the amplification of spurious products resulting from the formation of primer-dimers. In order to avoid these problems, the present invention provides methods and software application that provide selection criteria to generate a primer set configured for multiplex PCR, and subsequent use in a detection assay (e.g. INVADER detection assays).

In some embodiments, the methods and software applications of the present invention start with user defined sequences and corresponding SNP locations. In certain embodiments, the methods and/or software application determines a footprint region within the target sequence (the minimal amplicon required for INVADER detection) for each sequence. The footprint region includes the region where assay probes hybridize, as well as any user defined additional bases extending outward therefore (e.g. 5 additional bases included on each side of where the assay probes hybridize). Next, primers are designed outward from the footprint region and evaluated against several criteria, including the potential for primer-dimer formation with previously designed primers in the current multiplexing set (See, selection steps in Figure 10A). This process may be continued, as shown in Figure 10A, through multiple iterations of the same set of sequences until primers against all sequences in the current multiplexing set can be designed.

Once a primer set is designed for multiplex PCR, this set may be employed. Multiplex PCR may be carried out, for example, under standard conditions using only 10 ng of hgDNA as template. After 10 min at 95°C, Taq (2.5 units) may be added to a 50ul reaction and PCR carried out for 50 cycles. The PCR reaction may be diluted and loaded directly onto an INVADER MAP plate (3ul/well). An additional 3ul of 15mM $MgCl_2$ may be added to each reaction on the INVADER MAP plate and covered with 6ul of mineral oil. The entire plate may then be heated to 95°C for 5 min. and incubated at 63°C for 40 min. FAM and RED fluorescence may then be measured on a Cytofluor 4000 fluorescent plate reader and "Fold Over Zero" (FOZ) values calculated for each amplicon. Results from each SNP may be color coded in a table as "pass" (green), "miscall" (pink), or "no-call" (white) (See, section 2 below).

In some embodiments the number of PCR reactions is from about 1 to about 10 reactions. In some embodiments, the number of PCR reactions is from about 10 to about 50 reactions. In further embodiments, the number of PCR reactions is from about 50 to about 100. In additional embodiments, the number of PCR reactions is from about than 100 to 1,000. In still other embodiments, the number of PCR reactions is greater than 1,000.

The present invention also provides methods to optimize multiplex PCR reactions (e.g. once a primer set is generated, the concentration of each primer or primer pair may be optimized). For example, once a primer set has been generated and used in a multiplex PCR at equal molar concentrations, the primers may be evaluated separately such that the optimum primer concentration is determined such that the multiplex primer set performs better.

Multiplex PCR reactions are being recognized in the scientific, research, clinical and biotechnology industries as potentially time effective and.less expensive means of obtaining nucleic acid information compared to standard, monoplex PCR reactions. Instead of performing only a single amplification reaction per reaction vessel (tube or well of a multi-well plate for example), numerous amplification reactions are performed in a single reaction vessel.

The cost per target is theoretically lowered by eliminating technician time in assay set-up and data analysis, and by the substantial reagent savings (especially enzyme cost). Another benefit of the multiplex approach is that far less target sample is required. In whole genome association studies involving hundreds of thousands of single nucleotide polymorphisms (SNPs), the amount of target or test sample is limiting for large scale analysis, so the concept of performing a single reaction, using one sample aliquot to obtain, for example, 100 results, versus using 100 sample aliquots to obtain the same data set is an attractive option.

To design primers for a successful multiplex PCR reaction, the issue of aberrant interaction among primers should be addressed. The formation of primer dimers, even if only a few bases in length, may inhibit both primers form correctly hybridizing to the target sequence. Further, if the dimers form at or near the 3' ends of the primers, no amplification or very low levels of amplification will occur, since the 3' end is required for the priming event. Clearly, the more primers utilized per multiplex reaction, the more aberrant primer interactions are possible. The methods, systems and applications of the present help prevent primer dimers in large sets of primers, making the set suitable for highly multiplexed PCR.

When designing primer pairs for numerous site (for example 100 sites in a multiplex PCR reaction), the order in which primer pairs are designed can influence the total number of compatible primer pairs for a reaction. For example, if a first set of primers is designed for a first target region that happens to be an A/T rich target region, these primer will be A/T rich. If the second target region chosen also happens to be an A/T rich target region, it is far more likely that the primers designed for these two sets will be incompatible due to aberrant interactions, such as primer dimers. If, however, the second target region chosen is not A/T rich, it is much more likely that a primer set can be designed that will not interact with the first A/T rich set. For any given set of input target sequences, the present invention randomizes the order in which primer sets are designed (See, Figure 10A). Furthermore, in some embodiments, the present invention re-orders the set of input target sequences in a plurality of different, random orders to maximize the number of compatible primer sets for any given multiplex reaction (See, Figure 10A).

The present invention provides criteria for primer design that minimize 3' interactions while maximizing the number of compatible primer pairs for a given set of reaction targets in a multiplex design. For primers described as 5'-N[x]-N[x-1]-.....-N[4]-N[3]-N[2]-N[1]-3', N[1]- is an A or C (in alternative embodiments, N[1] is a G or T). N[2]-N[1] of each of the forward and reverse primers designed should not be complementary to N[2]-N[1] of any other oligonucleotide. In certain embodiments, N[3]-N[2]-N[1] should not be complementary to N[3]-N[2]-N[1] of any other oligonucleotide. In preferred embodiments, if these criteria are not met at a given N[1], the next base in the 5' direction for the forward primer or the next base in the 3' direction for the reverse primer may be evaluated as an N[1] site. This process is repeated, in conjunction with the target randomization, until all criteria are met for all, or a large majority of, the targets sequences (e.g. 95% of target sequences can have primer pairs made for the primer set that fulfill these criteria).

Another challenge to be overcome in a multiplex primer design is the balance between actual, required nucleotide sequence, sequence length, and the oligonucleotide melting temperature (Tm) constraints. Importantly, since the primers in a multiplex primer set in a reaction should function under the same reaction conditions of buffer, salts and temperature, they need therefore to have substantially similar Tm's, regardless of GC or AT richness of the region of interest. The present invention allows for primer design which meet minimum Tm and maximum Tm requirements and minimum and maximum length requirements. For example, in the formula for each primer 5'-N[x]-N[x-1]-..... N[4]-N[3]-N[2]-N[1]-3', x is selected such the primer has a predetermined melting temperature (e.g. bases are included in the primer until the primer has a calculated melting temperature of about 50 degrees Celsius).

Often the products of a PCR reaction are used as the target material for another nucleic acid detection means, such as-a hybridization-type detection assays, or the INVADER reaction assays for example. Consideration should be given to the location of primer placement to allow for the secondary reaction to successfully occur, and again, aberrant interactions between amplification primers and secondary reaction oligonucleotides should be minimized for accurate results and data. Selection criteria may be employed such that the primers designed for a multiplex primer set do not react (e.g. hybridize with, or trigger reactions) with oligonucleotide components of a detection assay. For example, in order to prevent primers from reacting with the FRET oligonucleotide of a bi-plex INVADER assay, certain homology criteria is employed. In particular, if each of the primers in the set are defined as 5'-N[x]-N[x-1]-.....-N[4]-N[3]-N[2]-N[1]-3', then N[4]-N[3]-N[2]-N[1]-3' is selected such that it is less than 90% homologous with the FRET or INVADER oligonucleotides. In mother embodiments, N[4]-N[3]-N[2]-N[1]-3' is selected for each primer such that it is less than 80% homologous with the FRET or INVADER oligonucleotides. In certain embodiments, N[4]-N[3]-N[2]-N[1]-3' is selected for each primer such that it is less than 70% homologous with the FRET or INVADER oligonucleotides.

While employing the criteria of The present invention to develop a primer set, some primer pairs may not meet all of the stated criteria (these may be rejected as errors). For example, in a set of 100 targets, 30 are designed and meet all listed criteria, however, set 31 fails. In the method of the present invention, set 31 may be flagged as failing, and the method could continue through the list of 100 targets, again flagging those sets which do not meet the criteria (See Figure 10A). Once all 100 targets have had a chance at primer design, the method would note the number of failed sets, re-order the 100 targets in a new random order and repeat the design process (See, Figure 10A). After a configurable number of runs, the set with the most passed primer pairs (the least number of failed sets) are chosen for the multiplex PCR reaction (See Figure 10A).

Figure 10A shows a flow chart with the basic flow of certain embodiments of the methods and software application of the present invention. In preferred embodiments, the processes detailed in Figure 10A are incorporated into a software application for ease of use (although, the methods may also be performed manually using, for example, Figure 10A as a guide).

Target sequences and/or primer pairs are entered into the system shown in Figure 10A. The first set of boxes show how target sequences are added to the list of sequences that have a footprint determined (See "B" in Figure 10A), while other sequences are passed immediately into the primer set pool (e.g. PDPass, those sequences that have been previously processed and shown to work together without forming Primer dimers or having reactivity to FRET sequences), as well as DimerTest entries (e.g. pair or primers a user wants to use, but that has not been tested yet for primer dimer or fret reactivity). In other words, the initial set of boxes leading up to "end of input" sort the sequences so they can be later processed properly.

Starting at "A" in Figure 10A, the primer pool is basically cleared our "emptied" to start a fresh run. The target sequences are then sent to "B" to be processed, and DimerTest pairs are sent to "C" to be processed. Target sequences are sent to "B", where a user or software application determines the footprint region for the target sequence (e.g. where the assay probes will hybridize in order to detect the mutation (e.g. SNP) in the target sequence). It is important to design this region (which the user may further expand by defining that additional bases past the hybridization region be added) such that the primers that are designed fully encompass this region. In Figure 10A, the software application INVADER CREATOR is used to design the INVADER oligonucleotide and downstream probes that will hybridize with the target region (although any type of program of system could be used to create any type of probes a user was interested in designing probes for, and thus determining the footprint region for on the target sequence). Thus the core footprint region is then defined by the location of these two assay probes on the target.

Next, the system starts from the 5' edge of the footprint and travels in the 5' direction until the first base is reached, or until the first A or C (or G or T) is reached. This is set as the initial starting point for defining the sequence of the forward primer (i.e. this serves as the initial N[1] site). From this initial N[1] site, the sequence of the primer for the forward primer is the same as those bases encountered on the target region. For example, if the default size of the primer is set as 12 bases, the system starts with the bases selected as N[1] and then adds the next 11 bases found in the target sequences. This 12-mer primer is then tested for a melting temperature (e.g. using INVADER CREATOR), and additional bases are added from the target sequence until the sequence has a melting temperature that is designated by the user as the default minimum and maximum melting temperatures (e.g. about 50 degrees Celsius, and not more than 55 degrees Celsius). For example, the system employs the formula 5'-N[x]-N[x-1]-..... N[4]-N[3]-N[2]-N[1]-3', and x is initially 12. Then the system adjusts x to a higher number (e.g. longer sequences) until the pre-set melting temperature is found. In certain embodiments, a maximum primer size is employed as a default parameter to serve as an upper limit on the length of the primers designed. In some embodiments, the maximum primer size is about 30 bases (e.g. 29 bases, 30, bases, or 31 bases). On other embodiments, the default settings (e.g. minimum and maximum primer size, and minimum and maximum Tm) are able to be modified using standard database manipulation tools.

The next box in Figure 10A, is used to determine if the primer that has been designed so far will cause primer-dimer and/or fret reactivity (e.g. with the other sequences already in the pool). The criteria used for this determination are explained above. If the primer passes this step, the forward primer is added to the primer pool. However, if the forward primer fails this criteria, as shown in Figure 10A, the starting point (N[1] is moved) one nucleotide in the 5' direction (or to the next A or C, or next G or T). The system first checks to make sure shifting over leaves enough room on the target sequence to successfully make a primer. If yes, the system loops back and check this new primer for melting temperature. However, if no sequence can be designed, then the target sequence is flagged as an error (e.g. indicating that no forward primer can be made for this target).

This same process is then repeated for designing the reverse primer, as shown in Figure 10A. If a reverse primer is successfully made, then the pair or primers is put into the primer pool, and the system goes back to "B" (if there are more target sequences to process), or goes onto "C" to test DimerTest pairs.

Starting a "C" in Figure 10A shows how primer pairs that are entered as primers (DimerTest) are processed by the system. If there are no DimerTest pairs, as shown in Figure 10A, the system goes on to "D". However, if there are DimerTest pairs, these are tested for primer-dimer and/or FRET reactivity as described above. If the DimerTest pair fails these criteria they are flagged as errors. If the DimerTest pair passes the criteria, they are added to the primer set pool, and then the system goes back to "C" if there are more DimerTest pairs to be evaluated, or or goes on to "D" if there are no more DimerTest pairs to be evaluated.

Starting at "D" in Figure 10A, the pool of primers that has been created is evaluated. The first step in this section is to examine the number of error (failures) generated by this particular randomized run of sequences. If there were no errors, this set is the best set as maybe ouputted to a user. If there the more than zero errors, the system compares this run to any other previous runs to see what run resulted in the fewest errors. If the current run has fewer errors, it is designated as the current best set. At this point, the system may go back to "A" to start the run over with another randomized set of the same sequences, or the pre-set maximum number of runs (e.g. 5 runs) may have been reached on this run (e.g. this was the 5th run, and the maximum number of runs was set as 5). If the maximum has been reached, then the best set is outputted as the best set. This best set of primers may then be used to generate as physical set of oligonucleotides such that a multiplex PCR reaction may be carried out.

Another challenge to be overcome with multiplex PCR reactions is the unequal amplicon concentrations that result in a standard multiplex reaction. The different loci targeted for amplification may each behave differently in the amplification reaction, yielding vastly different concentrations of each of the different amplicon products. The present invention provides methods, systems, software applications, computer systems, and a computer data storage medium that may be used to adjust primer concentrations relative to a first detection assay read (e.g. INVADER assay read), and then with balanced primer concentrations come close to substantially equal concentrations of different amplicons.

The concentrations for various primer pairs may be determined experimentally. In some embodiments, there is a first run conducted with all of the primers in equimolar concentrations. Time reads are then conducted. Based upon the time reads, the relative amplification factors for each amplicon are determined. Then based upon a unifying correction equation, an estimate of what the primer concentration should be obtained to get the signals closer within the same time point. These detection assays can be on an array of different sizes (384 well plates).

It is appreciated that combining the invention with detection assays and arrays of detection assays provides substantial processing efficiencies. Employing a balanced mix of primers or primer pairs created using the invention, a single point read can be carried out so that an average user can obtain great efficiencies in conducting tests that require high sensitivity and specificity across an array of different targets.

Having optimized primer pair concentrations in a single reaction vessel allows the user to conduct amplification for a plurality or multiplicity of amplification targets in a single reaction vessel and in a single step. The yield of the single step process is then used to successfully obtain test result data for, for example, several hundred assays. For example, each well on a 384 well plate can have a different detection assay thereon. The results of the single step mutliplex PCR reaction has amplified 384 different targets of genomic DNA, and provides you with 384 test results for each plate. Where each well has a plurality of assays even greater efficiencies can be obtained.

Therefore, the present invention provides the use of the concentration of each primer set in highly multiplexed PCR as a parameter to achieve an-unbiased amplification of each PCR product. Any PCR includes primer annealing and primer extension steps. Under standard PCR conditions, high concentration of primers in the order of 1 uM ensures fast kinetics of primers annealing while the optimal time of the primer extension step depends on the size of the amplified product and can be much longer than the annealing step. By reducing primer concentration, the primer annealing kinetics can become a rate limiting step and PCR amplification factor should strongly depend on primer concentration, association rate constant of the primers, and the annealing time.

The binding of primer $P$ with target $T$ can be described by the following model:

$$P + T \xrightarrow{k_a} PT \quad (1)$$

where $k_a$ is the association rate constant of primer annealing. We assume that the annealing occurs at the temperatures below primer melting and the reverse reaction can be ignored.

**The solution for this kinetics under the conditions of a primer excess is well known:**

$$[PT] = T_0(1 - e^{-k_a ct}) \quad (2)$$

where $[PT]$ is the concentration of target molecules associated with primer, $T_0$ is initial target concentration, c is the initial primer concentration, and $t$ is primer annealing time. Assuming that each target molecule associated with primer is replicated to produce full size PCR product, the target amplification factor in a single PCR cycle is

$$Z = \frac{T_0 + [PT]}{T_0} \doteq 2 - e^{-k_a ct} \quad (3)$$

The total PCR amplification factor after n cycles is given by

$$F = Z^n = (2 - e^{-k_a ct})^n \quad (4)$$

As it follows from equation 4, under the conditions where the primer annealing kinetics is the rate limiting step of PCR, the amplification factor should strongly-depend on primer concentration. Thus, biased loci amplification, whether it is caused by individual association rate constants, primer extension steps or any other factors, can be corrected by adjusting primer concentration for each primer set in the multiplex PCR. The adjusted primer concentrations can be also used to correct biased performance of INVADER assay used for analysis of PCR pre-amplified loci. Employing this basic principle, the present invention has demonstrated a linear relationship between amplification efficiency and primer concentration and used this equation to balance primer concentrations of different amplicons, resulting in the equal amplification of ten different amplicons in Example 1. This technique may be employed on any size set of multiplex primer pairs.

**DESIGNING A 10-PLEX (MANUAL): TEST FOR INVADER ASSAYS**

The following experimental example describes the manual design of amplification primers for a multiplex amplification reaction, and the subsequent detection of the amplicons by the INVADER assay.

Ten target sequences were selected from a set of pre-validated SNP-containing sequences, available in a Third Wave Technologies in-house oligonucleotide order entry database. Each target contains a single nucleotide polymorphism (SNP) to which an INVADER assay had been previously designed. The INVADER assay oligonucleotides were designed by the INVADER CREATOR software (Third Wave Technologies, Inc. Madison, WI), thus the footprint region in this example is defined as the INVADER "footprint", or the bases covered by the INVADER and the probe oligonucleotides, optimally positioned for the detection of the base of interest, in this case, a single nucleotide polymorphism. About 200 nucleotides of each of the 10 target sequences were analyzed for the amplification primer design analysis, with the SNP base residing about in the center of the sequence.

Criteria of maximum and minimum probe length (defaults of 30 nucleotides and 12 nucleotides, respectively) were defined, as was a range for the probe melting temperature Tm of 50-60°C. In this example, to select a probe sequence that will perform optimally at a pre-selected reaction temperatures, the melting temperature ($T_m$) of the oligonucleotide is calculated using the nearest-neighbor model and published parameters for DNA duplex formation (Allawi and SantaLucia, Biochemistry, 36:10581 [1997], herein incorporated by reference). Because the assay's salt concentrations are often different than the solution conditions in which the nearest-neighbor parameters were obtained (1M NaCl and no divalent metals), and because the presence and concentration of the enzyme influence optimal reaction temperature, an adjustment should be made to the calculated $T_m$ to determine the optimal temperature at which to perform a reaction. One way of compensating for these factors is to vary the value provided for the salt concentration within the melting temperature calculations. This adjustment is termed a 'salt correction'. The term "salt correction" refers to a variation made in the value provided for a salt concentration for the purpose of reflecting the effect on a $T_m$ calculation for a nucleic acid duplex of a non-salt parameter or condition affecting said duplex. Variation of the values provided for the strand concentrations will also affect the outcome of these calculations. By using a value of 280nM NaCl (SantaLucia, Proc Natl Acad Sci U S A, 95:1460 [1998]) and strand concentrations of about 10 pM of the probe and 1 fM target, the algorithm for used for calculating probe-target melting temperature has been adapted for use in predicting optimal primer design sequences.

Next, the sequence adjacent to the footprint region, both upstream and downstream were scanned and the first A or C was chosen for design start such that for primers described as 5'-N[x]-N[x-1]-.....-N[4]-N[3]-N[2]-N[1]-3', where N[1] should be an A or C. Primer complementarity was avoided by using the rule that: N[2]-N[1] of a given oligonucleotide primer should not be complementary to N[2]-N[1] of any other oligonucleotide, and N[3]-N[2]-N[1] should not be complementary to N[3]-N[2]-N[1] of any other oligonucleotide. If these criteria were not met at a given N[1], the next base in the 5' direction for the forward primer or the next base in the 3' direction for the reverse primer will be evaluated as an N[1] site. In the case of manual analysis, A/C rich regions were targeted in order to minimize the complementarity of 3' ends.

In this example, an INVADER assay was performed following the multiplex amplification reaction. Therefore, a section of the secondary INVADER reaction oligonucleotide (the FRET oligonucleotide sequence) was also incorporated as criteria for primer design; the amplification primer sequence should be less than 80% homologous to the specified region of the FRET oligonucleotide.

All primers were synthisized according to standard oligonucleotide chemistry, desalted (by standard methods) and quantified by absorbance at A260 and diluted to 50 μm concentrated stock.

Multiplex PCR was then carried out using 10-plex PCR using equimolar amounts of primer (0.01uM/primer) under the following conditions; 100mM KCl, 3mM MgCl$_2$, 1.0mM Tris pH8.0, 200uM dNTPs, 2.5U Taq DNA polymerase, and 10ng of human genomic DNA (hgDNA) template in a 50ul reaction. The reaction was incubated for (94C/30sec, 50C/44sec.) for 30 cycles. After incubation, the multiplex PCR reaction was diluted 1:10 with water and subjected to INVADER analysis using INVADER Assay FRET Detection Plates, 96 well genomic biplex, 100ng CLEAVASE VIII enzyme, INVADER assays were assembled as 15ul reactions as follows; 1ul of the 1:10 dilution of the PCR reaction, 3ul of PPI mix, 5ul of 22.5 mM MgCl2, 6ul of dH20, covered with 15ul of CHILLOUT liquid wax. Samples were denatured in the INVADER biplex by incubation at 95C for 5min., followed by incubation at 63C and fluorescence measured on a Cytofluor 4000 at various timepoints.

Using the following criteria to accurately make genotyping calls (FOZ_FAM+FOZ_RED-2 > 0.6), only 2 of the 10 INVADER assay calls can be made after 10 minutes of incubation at 63C, and only 5 of the 10 calls could'be made following an additional 50 min of incubation at 63C (60 min.). At the 60 min time point, the variation between the detectable FOZ values is over 100 fold between the strongest signal. Using the same INVADER assays directly against 100ng of human genomic DNA (where equimolar amounts of each target would be available), all reads could be made with in the dynamic range of the reader and variation in the FOZ values was approximately seven fold between the strongest and weakest of the assays. This suggests that the dramatic discrepancies in FOZ values seen between different amplicons in the same multiplex PCR reaction is a function of biased amplification, and not variability attributable to INVADER assay. Under these conditions, FOZ values generated by different INVADER assays are directly comparable to one another and can reliably be used as indicators of the efficiency of amplification.

**Estimation of amplification factor of a given amplicon using FOZ values.** In order to estimate the amplification factor ($F$) of a given amplicon, the FOZ values of the INVADER assay can be used to estimate amplicon abundance. The FOZ of a given amplicon with unknown concentration at a given time (FOZm) can be directly compared to the FOZ of a known amount of target (e.g. 100 ng of genomic DNA = 30,000 copies of a single gene) at a defined point in time ($FOZ_{204}$, 240 min) and used to calculate the number of copies of the unknown amplicon. In equation 1, $FOZm$ represents the sum of RED_FOZ and FAM_FOZ of an unknown concentration of target incubated in an INVADER assay for a given amount of time $(m)$. $FOZ_{240}$ represents an empirically determined value of RED_FOZ (using INVADER assay 41646), using for a known number of copies of target (e.g. 100ng of hgDNA $\cong$ 30,000 copies) at 240 minutes.

$$F = ((FOZ_m - 1) * 500/(FOZ_{240} - 1)) * (240/m)^{\wedge}2 \text{ (equation 1a)}$$

Although equation 1a is used to determine the linear relationship between primer concentration and amplification factor $F$, equation 1a' is used in the calculation of the amplification factor $F$ for the 10-plex PCR (both with equimolar amounts of primer and optimized concentrations of primer), with the value $D$ representing the dilution factor of the PCR reaction. In the case of a 1:3 dilution of the 50 ul multiplex PCR reaction. $D$=0.3333.

$$F = ((FOZ_m - 2) * 500/(FOZ_{240} - 1) * D) * (240/m)^{\wedge}2 \text{ (equation 1a')}$$

Although equations 1a and 1a' will be used in the description of the 10-plex multiplex PCR, a more correct adaptation of this equation was used in the optimization of primer concentrations in the 107-plex PCR. In this case, $FOZ_{240=}$the average of FAM_$FOZ_{240}$+RED_$FOZ_{240}$ over the entire INVADER MAP plate using hgDNA as target ($FOZ_{240=}$3.42) and the dilution factor $D$ is set to 0.125.

$$F = ((FOZ_m - 2) * 500/(FOZ_{240} - 2) * D) * (240/m)^{\wedge}2 \text{ (equation 1b)}$$

It should be noted that in order for the estimation of amplification factor F to be more accurate, FOZ values should be within the dynamic range of the instrument on which the reading are taken. In the case of the Cytofluor 4000 used in this study, the dynamic range was between about 1.5 and about 12 FOZ.

**Section 3. Linear Relationship between Amplification Factor and Primer Concentration.**

In order to determine the relationship between primer concentration and amplification factor (F), four distinct uniplex PCR reactions were run at using primers 1117-70-17 and 1117-70-18 at concentrations of 0.01uM, 0.012 uM, 0.014 uM, 0.020 uM respectively. The four independent PCR reactions were carried out under the following conditions; 100mM KCl, 3mM MgCl, 10mM Tris pH 8.0, 200uM dNTPs using 10ng of hgDNA as template. Incubation was carried out at (94C/30 sec., 50C/20 sec.) for 30 cycles. Following PCR, reactions were diluted 1:10 with water and run under standard conditions using INVADER Assay FRET Detection Plates, 96 well genomic biplex, 100ng CLEAVASE VIII enzyme. Each 15ul reaction was set up as follows; 1ul of 1:10 diluted PCR reaction, 3ul of the PPI mix SNP#47932, 5ul 22.5mM MgCl2, 6ul of water, 15 ul of CHILLOUT liquid wax. The entire plate was incubated at 95C for 5min, and then at 63C for 60 min at which point a single read was taken on a Cytofluor 4000 fluorescent plate reader. For each of the four different primer concentrations (0.01uM, 0.012 uM, 0.014 uM, 0.020 uM) the amplification factor $F$ was calculated using equation 1a, with $FOZm$=the sum of FOZ_FAM and FOZ_RED at 60 minutes, $m$=60, and $FOZ_{240}$=1.7. In plotting the primer concentration of each reaction against the log of the amplification factor Log(F), a strong linear relationship was noted. Using the data points generated, the formula describing the linear relationship between amplification factor and primer concentration is described in equation 2:

$$Y=1.684X+2.6837 \text{ (equation 2a)}$$

Using equation 2, the amplification factor of a given amplicon Log(F)=Y could be manipulated in a predictable fashion using a known concentration of primer (X). In a converse manner, amplification bias observed under conditions of equimolar primer concentrations in multiplex PCR, could be measured as the "apparent" primer concentration (X) based on the amplification factor *F*. In multiplex PCR, values of "apparent" primer concentration among different amplicons can be used to estimate the amount of primer of each amplicon required to equalize amplification of different loci:

$$X=(Y-2.6837)/1.68 \text{ (equation 2b)}$$

**Section 4. Calculation of Apparent Primer Concentrations from a Balanced Multiplex-Mix.**

As described in a previous section, primer concentration can directly influence the amplification factor of given amplicon. Under conditions of equimolar amounts of primers, *FOZm* readings can be used to calculate the "apparent" primer concentration of each amplicon using equation 2. Replacing Y in equation 2 with log(F) of a given amplification factor and solving for X, gives an "apparent" primer concentration based on the relative abundance of a given amplicon in a multiplex reaction. Using equation 2 to calculate the "apparent" primer concentration of all primers (provided in equimolar concentration) in a multiplex reaction, provides a means of normalizing primer sets against each other. In order to derive the relative amounts of each primer that should be added to an "Optimized" multiplex primer mix R, each of the "apparent" primer concentrations should be divided into the maximum apparent primer concentration ($X_{max}$), such that the strongest amplicon is set to a value of 1 and the remaining amplicons to values equal or greater than 1

$$R[n]=Xmax/X[n] \text{ (equation 3)}$$

Using the values of R[n] as an arbitrary value of relative primer concentration, the values of R[n] are multiplied by a constant primer concentration to provide working concentrations for each primer in a given multiplex reaction. In the example shown, the amplicon corresponding to SNP assay 41646 has an R[n] value equal to 1. All of the R[n] values were multiplied by 0.01uM (the original starting primer concentration in the equimolar multiplex PCR reaction) such that lowest primer concentration is R[n] of 41646 which is set to 1, or 0.01uM. The remaining primer sets were also proportionally increased as shown. The results of multiplex PCR with the "optimized" primer mix are described below.

**Section 5 Using optimized primer concentrations in multiplex PCR, variation in FOZ's among 10 INVADER assays are greatly reduced.**

Multiplex PCR was carried out using 10-plex PCR using varying amounts of primer based on the determined volumes (X[max] was SNP41646, setting 1x=0.01uM/primer). Multiplex PCR was carried out under conditions identical to those used in with equimolar primer mix; 100mMKCl, 3mMMgCl, 10mM Tris pH8.0, 200uM dNTPs, 2.5U taq, and 10ng of hgDNA template in a 50ul reaction. The reaction was incubated for (94C/30sec, 50C/44sec.) for 30 cycles. After incubation, the multiplex PCR reaction was diluted 1:10 with water and subjected to INVADER analysis. Using INVADER Assay FRET Detection Plates, (96 well genomic biplex, 100ng CLEAVSE VIII enzyme), reactions were assembled as 15ul reactions as follows; 1ul of the 1:10 dilution of the PCR reaction, 3ul of the appropriate PPI mix, 5ul of 22.5 mM MgCl2, 6ul of dH20. An additional 15ul of CHILL OUT was added to each well, followed by incubation at 95C for 5min. Plates were incubated at 63C and fluorescence measured on a Cytofluor 4000 at 10 min.

Using the following criteria to accurately make genotyping calls (FOZ_FAM+FOZ_RED-2 > 0.6), all 10 of 10 (100%) INVADER calls can be made after 10 minutes of incubation at 63C. In addition, the values of FAM+RED-2 (an indicator of overall signal generation, directly related to amplification factor (see equation 2)) varied by less than seven fold between the the lowest signal and the highest.

**Design of 101-plex PCR using the Software Application**

Using the TWT Oligo Order Entry Database, 144 sequences of less than 200 nucleotides in length were obtained, with SNPs annotated using brackets to indicate the SNP position for each sequence (e.g. NNNNNNN[N$_{(wt)}$/N$_{(mt)}$] NNNNNNNN). In order to expand sequence data flanking the SNP of interest, sequences were expanded to approximately 1kB in length (500 nts flanking each side of the SNP) using BLAST analysis. Of the 144 starting sequences, 16 could not expanded by BLAST, resulting in a final set of 128 sequences expanded to approximately 1kB length. These expanded sequences were provided to the user in Excel format with the following information for each sequence; (1) TWT Number, (2) Short Name Identifier, and (3) sequence. The Excel file was converted to a comma delimited format and used as the input file for Primer Designer INVADER CREATOR v1.3.3. software (this version of the program does not screen for FRET reactivity of the primers, nor does it allow the user to specify the maximum length of the primer). INVADER CREATOR Primer Designer v1.3.3., was run using default conditions (e.g. minimum primer size of 12, maximum of 30), with the exception of $Tm_{low}$ which was set to 60C. The output file contained 128 primer sets (256 primers), four of which were thrown out due to excessively long primer sequences (SNP # 47854, 47889, 54874, 67396), leaving 124 primers sets (248 primers) available for synthesis. The remaining primers were synthesized using standard procedures at the 200nmol scale and purified by desalting. After synthesis failures, 107 primer sets were available for assembly of an equimolar 107-plex primer mix (214 primers). Of the 107 primer sets available for amplification, only 101 were present on the INVADER MAP plate to evaluate amplification factor.

Multiplex PCR was carried out using 101-plex PCR using equimolar amounts of primer (0.025uM/primer) under the following conditions;100mMKCl, 3mM MgCl, 10mM Tris pH8.0, 200uM dNTPs, and 10ng of human genomic DNA (hgDNA) template in a 50ul reaction. After denaturation at 95C for 10min, 2.5 units of Taq was added and the reaction incubated for (94C/30sec, 50C/44sec.) for 50 cycles. After incubation, the multiplex PCR reaction was diluted 1:24 with water and subjected to INVADER assay analysis using INVADER MAP detection platform. Each INVADER MAP assay was run as a 6ul reaction as follows; 3ul of the 1:24 dilution of the PCR reaction (total dilution 1:8 equaling $D$=0.125), 3ul of 15 mM MgCl2 covered with covered with 6ul of CHILLOUT. Samples were denatured in the INVADER MAP plate by incubation at 95C for 5min., followed by incubation at 63C and fluorescence measured on a Cytofluor 4000 (384 well reader) at various timepoints over 160 minutes. Analysis of the FOZ values calculated at 10, 20, 40, 80, 160 min. shows that correct calls (compared to genomic calls of the same DNA sample) could be made for 94 of the 101 amplicons detectable by the INVADER MAP platform. This provides proof that the INVADER CREATOR Primer Designer software can create primer sets which function in highly multiplex PCR.

In using the FOZ values obtained throughout the 160 min. time course, amplification factor F and R[n] were calculated for each of the 101 amplicons. R[nmax] was set at 1.6, which although Low end corrections were made for amplicons which failed to provide sufficient FOZm signal at 160 min., assigning an arbitrary value of 12 for R[n]. High end corrections for amplicons whose FOZm values at the 10 min. read, an R[n] value of 1 was arbitrarily assigned. Optimized primer concentrations of the 101-plex were calculated using the basic principles outlined in the 10-plex example and equation 1b, with an R[n] of 1 corresponding to 0.025uM primer. Multiplex PCR was under the following conditions; 100mMKCl, 3mM MgCl, 10mM Tris pH8.0, 200uM dNTPs, and 10ng of human genomic DNA (hgDNA) template in a 50ul reaction. After denaturation at 95C for 10min, 2.5 units of Taq was added and the reaction incubated for (94C/30sec, 50C/44sec.) for 50 cycles. After incubation, the multiplex PCR reaction was diluted 1:24 with water and subjected to INVADER analysis using INVADER MAP detection platform. Each INVADER MAP assay was run as a 6ul reaction as follows; 3ul of the 1:24 dilution of the PCR reaction (total dilution 1:8 equaling $D$=0.125), 3ul of 15 mM MgCl2 covered with covered with 6ul of CHILLOUT. Samples were denatured in the INVADER MAP plate by incubation at 95C for 5min., followed by incubation at 63C and fluorescence measured on a Cytofluor 4000 (384 well reader) at various timepoints over 160 minutes. Analysis of the FOZ values was carried out at 10, 20, and 40 min. and compared to calls made directly against the genomic DNA. Under equimolar primer concentration, multiplex PCR results in only 50 correct calls at the 10 min time point, where under optimized primer concentrations multiplex PCR results in 71 correct calls, resulting in a gain of 21 (42%) new calls. Although all 101 calls could not be made at the 10 min timepoint, 94 calls could be made at the 40 min. timepoint suggesting the amplification efficiency of the majority of amplicons had improved. Unlike the 10-plex optimization that only required a single round of optimization, multiple rounds of optimization may be required for more complex multiplexing reactions to balance the amplification of all loci.

### USE OF THE INVADER ASSAY TO DETERMINE AMPLIFICATION

### FACTOR OF PCR

The INVADER assay can be used to monitor the progress of amplification during PCR reactions, *i.e.*, to determine the amplification factor F that reflects efficiency of amplification of a particular amplicon in a reaction. In particular, the INVADER assay can be used to determine the number of molecules present at any point of a PCR reaction by reference to a standard curve generated from quantified reference DNA molecules. The amplification factor F is measured as a ratio of PCR product concentration after amplification to initial target concentration. This example demonstrates the effect of varying primer concentration on the measured amplification factor.

PCR reactions were conducted for variable numbers of cycles in increments of 5, *i.e.*, 5, 10, 15, 20, 25, 30, so that the progress of the reaction could be assessed using the INVADER assay to measure accumulated product. The reactions were diluted serially to assure that the target amounts did not saturate the INVADER assay, *i.e.*, so that the measurements could be made in the linear range of the assay. INVADER assay standard curves were generated using a dilution series containing known amounts of the amplicon. This standard curve was used to extrapolate the number of amplified DNA fragments in PCR reactions after the indicated number of cycles. The ratio of the number of molecules after a given number of PCR cycles to the number present prior to amplification is used to derive the amplification factor, F, of each PCR reaction.

**PCR Reactions**

PCR reactions were set up using equimolar amounts of primers (*e.g.*, 0.02 $\mu$M or 0.1 $\mu$M primers, final concentration). Reactions at each primer concentration were set up in triplicate for each level of amplification tested, *i.e.*, 5, 10, 15, 20, 25, and 30 PCR cycles. One master mix sufficient for 6 standard PCR reactions (each in triplicate X 2 primer concentrations) plus 2 controls X 6 tests (5, 10, 15, 20, 25, or 30 cycles of PCR) plus enough for extra reactions to allow for overage.

Serial dilutions of PCR reaction products

In order to ensure that the amount of PCR product added as target to the INVADER assay reactions would not exceed the dynamic range of the real time assay on the PERSEPTIVE BIOSYSTEMS CYTOFLUOR 4000, the PCR reaction products were diluted prior to addition to the INVADER assays. An initial 20-fold dilution was made of each reaction, followed by subsequent five-fold serial dilutions.
To create standards, amplification products generated with the same primers used in the tests of different numbers of cycles were isolated from non-denaturing polyacrylimide gels using standard methods and quantified using the PICOGREEN assay. A working stock of 200 pM was created, and serial dilutions of these concentration standards were created in $dH_2O$ containing tRNA at 30 ng/$\mu$l to yield a series with final amplicon concentrations of 0.5, 1, 2.5, 6.25, 15.62, 39, and 100 fM.

**INVADER assay reactions**

Appropriate dilutions of each PCR reaction and the no target control were made in triplicate, and tested in standard, singlicate INVADER assay reactions. One master mix was made for all INVADER assay reactions. In all, there were 6 PCR cycle. conditions X 24 individual test assays [(1 test of triplicate dilutions X 2 primer conditions X 3 PCR replicates) = 18 + 6 no target controls]. In addition, there were 7 dilutions of the quantified amplicon standards and 1 no target control in the standard series. The standard series was analyzed in replicate on each of two plates, for an additional 32 INVADER assays. The total number of INVADER assays is 6 X 24 + 32 = 176. The master mix included coverage for 32 reactions.
INVADER assay master mix and comprised the following standard components:FRET buffer/Cleavase XI/Mg/PPI mix for 192 plus 16 wells.
The following oligonucleotides were included in the PPI mix.

0.25$\mu$M INVADER for assay 2 (GAAGCGGCGCCGGTTACCACCA)
2.5$\mu$M A Probe for assay 2 (CGCGCCGAGGTGGTTGAGCAATTCCAA)
2.5$\mu$M G Probe for assay 2 (ATGACGTGGCAGACCGGTTGAGCAATTCCA)

All wells were overlaid with 15$\mu$l mineral oil, incubated at 95 °C 5 min, then at 63 °C read at various intervals, eg. 20, 40, 80, or 160 min, depending on the level of signal generated. The reaction plate was read on a CytoFluor® Series 4000 Fluorescence Multi-Well Plate Reader. The settings used were: 485/20 nm excitation/bandwidth and 530/25 nm emission/bandwidth for F dye detection, and 560/20 nm excitation/bandwidth and 620/40 nm emission/bandwidth for R dye detection. The instrument gain was set for each dye so that the No Target Blank produced between 100 - 200 Absolute Fluorescence Units (AFUs).

**Results:**

These results indicate that the PCR reactions were exponential over the range of cycles tested. The use of different primer concentrations resulted in different slopes such that the slope generated from INVADER assay analysis of PCR reactions carried out with the higher primer concentration (0.1 $\mu$M) is steeper than that with-the lower (0.02 $\mu$M) concentration. In addition, the slope obtained using 0.1 $\mu$M approaches that anticipated for perfect doubling (0.301). The amplification factors from the PCR reactions at each primer concentration were obtained from the slopes:

For 0.1 $\mu$M primers, slope = 0.286; amplification factor: 1.93
For 0.02 $\mu$M primers, slope = 0.218; amplification factor: 1.65.

The lines do not appear to extend to the origin but rather intercept the X-axis between 0 and 5 cycles, perhaps reflective of errors in estimating the starting concentration of human genomic DNA.

Thus, these data show that primer concentration affects the extent of amplification during the PCR reaction. These data further demonstrate that the INVADER assay is an effective tool for monitoring amplification throughout the PCR reaction.

## DEPENDENCE OF AMPLIFICATION FACTOR ON PRIMER CONCENTRATION

This example demonstrates the correlation between amplification factor, F, and primer concentration, c. In this experiment, F was determined for 2 alleles from each of 6 SNPs amplified in monoplex PCR reactions, each at 4 different primer concentrations, hence 6 primer pairs X 2 genomic samples X 4 primer concentrations = 48 PCR reactions.

Whereas the effect of PCR cycle number was tested on a single amplified region, at two primer concentrations, in Example 3, in this example, all test PCR reactions were run for 20 cycles, but the effect of varying primer concentration was studied at 4 different concentration levels: 0.01 $\mu$M, 0.025 $\mu$M, 0.05 $\mu$M, 0.1 $\mu$M. Furthermore, this experiment examines differences in amplification of different genomic regions to investigate (a) whether different genomic regions are amplified to different extents (i.e. PCR bias) and (b) how amplification of different genomic regions depends on primer concentration.

As in Example 3, F was measured by generating a standard curve for each locus using a dilution series of purified, quantified reference amplicon preparations. In this case, 12 different reference amplicons were generated: one for each allele of the SNPs contained in the 6 genomic regions amplified by the primer pairs. Each reference amplicon concentration was tested in an INVADER assay, and a standard curve of fluorescence counts versus amplicon concentration was created. PCR reactions were also run on genomic DNA samples, the products diluted, and then tested in an INVADER assay to determine the extent of amplification, in terms of number of molecules, by comparison to the standard curve.

### a. Generation of standard curves using quantified reference amplicons

A total of 8 genomic DNA samples isolated from whole blood were screened in standard biplex INVADER assays to determine their genotypes at 24 SNPs in order to identify samples homozygous for the wild-type or variant allele at a total of 6 different loci.

Once these loci were identified, wild-type and variant genomic DNA samples were analyzed in separate PCR reactions with primers flanking the genomic region containing each SNP. At each SNP, one allele reported to FAM dye and one to RED.

Suitable genomic DNA preparations were then amplified in standard individual, monoplex PCR reactions to generate amplified fragments for use as PCR reference standards as described in Example 3.

Following PCR, amplified DNA was gel isolated using standard methods and previously quantified using the PICOGREEN assay. Serial dilutions of these concentration standards were created as follows:

Each purified amplicon was diluted to create a working stock at a concentration of 200 pM. These stocks were then serially diluted as follows. A working stock solution of each amplicon was prepared with a concentration of 1.25 pM in dH$_2$O containing tRNA at 30 ng/$\mu$l. The working stock was diluted in 96-well microtiter plates and then serially diluted to yield the following final concentrations in the INVADER assay: 1, 2.5, 6.25, 15.6, 39, 100, and 250 fM. One plate was prepared for the amplicons to be detected in the INVADER assay using probe oligonucleotides reporting to FAM dye and one plate for those to be tested with probe oligonucleotides reporting to RED dye. All amplicon dilutions were analyzed in duplicate.

Aliquots of 100 $\mu$l were transferred, in this layout, to 96 well MJ Research plates and denatured for 5 min at 95 °C prior to addition to INVADER assays.

### b. PCR amplification of genomic samples at different primer concentrations.

PCR reactions were set up for individual amplification of the 6 genomic regions described in the previous example on each of 2 alleles at 4 different primer concentrations, for a total of 48 PCR reactions. All PCRs were run for 20 cycles. The following primer concentrations were tested: 0.01 $\mu$M, 0.025 $\mu$M, 0.05 $\mu$M, and 0.1 $\mu$M. A master mix for all 48 reactions was prepared according to standard procedures, with the exception of the modified primer concentrations, plus overage for an additional 23 reactions (16 reactions were prepared but not used, and overage of 7 additional reactions was prepared).

**EP 1 567 540 B1**

**c. Dilution of PCR reactions**

Prior to analysis by the INVADER assay, it was necessary to dilute the products of the PCR reactions, as described in Examples 1 and 2. Serial dilutions of each of the 48 PCR reactions were made using one 96-well plate for each SNP. The left half of the plate contained the SNPs to be tested with probe oligonucleotides reporting to FAM; the right half, with probe oligonucleotides reporting to RED. The initial dilution was 1:20; asubsequent dilutions were 1:5 up to 1: 62,500.

**d. INVADER assay analysis of PCR dilutions and reference amplicons**

INVADER analysis was carried out on all dilutions of the products of each PCR reaction as well as the indicated dilutions of each quantified reference amplicon (to generate a standard curve for each amplicon) in standard biplex INVADER assays.

All wells were overlaid with 15 $\mu$l of mineral oil. Samples were heated to 95 °C for 5 min to denature and then incubated at 64°C. Fluorescence measurements were taken at 40 and 80 minutes in a CytoFluor® 4000 fluorescence plate reader (Applied Biosystems, Foster City, CA). The settings used were: 485/20 nm excitation/bandwidth and 530/25 nm emission/bandwidth for F dye detection, and 560/20 nm excitation/bandwidth and 620/40 nm emission/bandwidth for R dye detection. The instrument gain was set for each dye so that the No Target Blank produced between 100 - 200 Absolute Fluorescence Units (AFUs). The raw data is that generated by the device/instrument used to measure the assay performance (real-time or endpoint mode).

The results indicate that the dependence of *lnF* on *c* demonstrates different amplification rates for the 12 PCRs under the same reaction conditions, although the difference is much smaller within each pair of targets representing the same SNP. The amplification factor strongly depends on c at low primer concentrations with a trend to plateau at higher primer concentrations. This phenomenon can be explained in terms of the kinetics of primer annealing. At high primer concentrations, fast annealing kinetics ensures that primers are bound to all targets and maximum amplification rate is achieved, on the contrary, at low primer concentrations the primer annealing kinetics become a rate limiting step decreasing *F*.

This analysis suggests that plotting amplification factor as a function of primer concentration in $\ln\left(2 - F^{\frac{1}{n}}\right)$ *vs. c* coordinates should produce a straight line with a slope *-ka ta.* Re-plotting of the data in the $\ln\left(2 - F^{\frac{1}{n}}\right)$ *vs. c* coordinates demonstrates the expected linear dependence for low primer concentrations (low amplification factor) which deviates from the linearity at 0.1 $\mu$M primer concentration (*F* is $10^5$ or larger) due to lower than expected amplification factor. The $k_a t_a$, values can be calculated for each PCR using the following equation.

$$F = z^n = \left(2 - e^{-k_a c t_a}\right)^n$$

**INVADER ASSAY ANALYSIS OF 192-PLEX PCR REACTION**

This example describes the use of the INVADER assay to detect the products of a highly multiplexed PCR reaction designed to amplify 192 distinct loci in the human genome.

**Genomic DNA extraction**

Genomic DNA was isolated from 5 mls of whole blood and purified using the Autopure, manufactured by Gentra Systems, Inc. (Minneapolis, MN). The purified DNA was in 500 $\mu$l of dH$_2$O.

**Primer design**

Forward and reverse primer sets for the 192 loci were designed using Primer Designer, version 1.3.4 (See Primer Design section above, including Figure 10A). Target sequences used for INVADER designs, with no more than 500 bases flanking the relevant SNP site, were converted into a comma-delimited text file for use as an input file for PrimerDesigner. PrimerDesigner was run using default parameters, with the exception of oligo T$_m$, which was set at 60 °C.

57

**Primer synthesis**

Oligonucleotide primers were synthesized using standard procedures in a Polyplex (GeneMachines, San Carlos, CA). The scale was 0.2 µmole, desalted only (not purified) on NAP-10 and not dried down.

**PCR reactions**

Two master mixes were created. Master mix 1 contained primers to amplify loci 1-96; master mix 2, 97-192. The mixes were made according to standard procedures and contained standard components. All primers were present at a final concentration of 0.025 µM, with KCl at 100 mM, and MgCl at 3 mM. PCR cycling conditions were as follows in a MJ PTC-100 thermocycler (MJ Research, Waltham, MA): 95 °C for 15 min; 94°C for 30 sec; then 55°C 44 sec X 50 cycles

Following cycling, all 4 PCR reactions were combined and aliquots of 3 µl were distributed into a 384 deep-well plate using a CYBI-well 2000 automated pipetting station (CyBio AG, Jena, Germany). This instrument makes individual reagent additions to each well of a 384-well microplate. The reagents to be added are themselves arrayed in 384-well deep half plates.

**INVADER assay reactions**

INVADER assays were set up using the CYBI-well 2000. Aliquots of 3 µl of the genomic DNA target were added to the appropriate wells. No target controls were comprised of 3 µl of Te (10 mM Tris, pH 8.0, 0.1 mM EDTA). The reagents for use in the INVADER assays were standard PPI mixes, buffer, FRET oligonucleotides, and Cleavase VIII enzyme and were added individually to each well by the CYBI-well 2000.

Following the reagent additions, 6 µl of mineral oil were overlaid in each well. The plates were heated in a MJ PTC-200 DNA ENGINE thermocycler (MJ Research) to 95 °C for 5 minutes then cooled to the incubation temperature of 63 °C. Fluorescence was read after 20 minutes and 40 minutes using the Safire microplate reader (Tecan, Zurich, Switzerland) using the following settings. 495/5 nm excitation/bandwidth and 520/5 nm emission/bandwidth for F dye detection; and 600/5 nm emission/bandwidth, 575/5 nm excitation/bandwidth Z position, 5600µs; number of flashes, 10; lag time, 0; integration time, 40 µsec for R dye detection. Gain was set for F dye at 90 nm and R dye at 120. The raw data is that generated by the device/instrument used to measure the assay performance (real-time or endpoint mode).

Of the 192 reactions, genotype calls could be made for 157 after 20 minutes and 158 after 40 minutes, or a total of 82%. For 88 of the assays, genotyping results were available for comparison from data obtained previously using either monoplex PCR followed by INVADER analysis or INVADER results obtained directly from analysis of genomic DNA. For 69 results, no corroborating genotype results were available.

This example shows that it is possible to amplify more than 150 loci in a single multiplexed PCR reaction. This example further shows that the amount of each amplified fragment generated in such a multiplexed PCR reaction is sufficient to produce discernable genotype calls when used as a target in an INVADER assay. In addition, many of the amplicons generated in this multiplex PCR assay gave high signal, measured as FOZ, in the INVADER assay, while some gave such low signal that no genotype call could be made. Still others amplicons were present at such low levels, or not at all, that they failed to yield any signal in the INVADER assay.

**OPTIMIZATION OF PRIMER CONCENTRATION TO IMPROVE PERFORMANCE OF HIGHLY-MULTIPLEXED PCR REACTIONS**

Competition between individual reactions in multiplex PCR may aggravate amplification bias and cause an overall decrease in amplification factor compared with uniplex PCR. The dependence of amplification factor on primer concentration can be used to alleviate PCR bias. The variable levels of signal produced from the different loci amplified in the 192-plex PCR of the previous example, taken with the results from Example 3 that show the effect of primer concentration on amplification factor, further suggest that it may be possible to improve the percentage of PCR reactions that generate sufficient target for use in the INVADER assay by modulating primer concentrations.

For example, one particular sample analyzed in Example 5 yielded FOZ results, after a 40 minute incubation in the INVADER assay, of 29.54 FAM and 66.98 RED, while another sample gave FOZ results after 40 min of 1.09 and 1.22, respectively, prompting a determination that there was insufficient signal to generate a genotype call. Modulation of primer concentrations, down in the case of the first sample and up in the case of the second, should make it possible to bring the amplification factors of the two samples closer to the same value. It is envisioned that this sort of modulation maybe an iterative process, requiring more than one modification to bring the amplification factors sufficiently close to one another to enable most or all loci in a multiplex PCR reaction to be amplified with approximately equivalent efficiency.

**Claims**

1. A method for determining the presence or absence of mutations and polymorphisms at a plurality of loci in CFTR target nucleic acid comprising the 2789+5G>A allele, comprising:

   a) providing a sample comprising CFTR target nucleic acid;
   b) amplifying said CFTR target nucleic acid in a single reaction vessel with 17 cycles or fewer of a polymerase chain reaction to generate amplified target DNA, wherein said polymerase chain reaction contains primers pairs for the amplification of at least 20 different CFTR amplification targets suspected of containing mutant or polymorphic loci, and wherein the concentrations of the primers used in the polymerase chain reaction are modulated so that said at least 20 different CFTR amplification targets are amplified with approximately equivalent efficiency; and
   c) exposing said amplified target DNA to a plurality of detection assays, wherein said detection assays comprise invasive cleavage assays comprising first and second oligonucleotides configured to form invasive cleavage structures to detect a plurality of CFTR mutations and polymorphisms at loci in said amplified target nucleic acid, under conditions such that the presence or absence of said CFTR mutations and polymorphisms at said loci is determined.

2. The method of Claim 1, wherein said at least 20 different CFTR amplification targets comprise thirty or more different CFTR amplification targets.

3. The method of Claim 1, wherein said amplifying and exposing are conducted simultaneously.

**Patentansprüche**

1. Verfahren zur Bestimmung des Vorhandenseins oder des Fehlens von Mutationen und Polymorphismen an einer Vielzahl von Loci in CFTR Ziel-Nukleinsäure umfassend das 2789+5G>A Allel, umfassend:

   a) Bereitstellen einer Probe umfassend CFTR Ziel-Nukleinsäure;
   b) Amplifizieren der CFTR Ziel-Nukleinsäure in einem einzigen Reaktionsgefäß mit 17 Zyklen oder weniger einer Polymerase-Kettenreaktion, um amplifizierte Ziel-DNS zu generieren, worin die Polymerase-Kettenreaktion Primerpaare für die Amplifikation von mindestens 20 verschiedenen CFTR Amplifikationszielen enthält, von denen vermutet wird, dass sie mutierte oder polymorphe Loci enthalten, und worin die Konzentrationen der in der Polymerase-Kettenreaktion verwendeten Primer moduliert werden, sodass die mindestens 20 verschiedenen CFTR Amplifikationsziele mit ungefähr äquivalenter Effizienz amplifiziert werden; und
   c) Exponieren der amplifizierten Ziel-DNS an eine Vielzahl von Detektions-Assays, worin die Detektions-Assays invasive Spaltungs-Assays umfassen, die erste und zweite Oligonukleotide umfassen, die so konfiguriert sind, dass sie invasive Spaltungsstrukturen bilden, um eine Vielzahl von CFTR Mutationen und Polymorphismen an Loci in der amplifizierten Ziel-Nukleinsäure zu detektieren, und zwar unter Bedingungen, unter denen die Anwesenheit oder Abwesenheit der CFTR Mutationen und Polymorphismen an den Loci bestimmt wird.

2. Verfahren nach Anspruch 1, worin die mindestens 20 verschiedenen CFTR Amplifikationsziele mindestens dreißig oder mehr verschiedene CFTR Amplifikationsziele umfassen.

3. Verfahren nach Anspruch 1, worin das Amplifizieren und Exponieren gleichzeitig durchgeführt werden.

**Revendications**

1. Procédé pour déterminer la présence ou l'absence de mutations et de polymorphismes au niveau d'une pluralité de loci dans un acide nucléique cible de CFTR comprenant l'allèle 2789+5G>A, consistant à :

   a) mettre à disposition un échantillon comprenant un acide nucléique cible de CFTR ;
   b) amplifier ledit acide nucléique cible de CFTR dans un seul récipient de réaction avec 17 cycles ou moins d'une réaction en chaîne par polymérase afin de générer un ADN cible amplifié, où ladite réaction en chaîne par polymérase contient des paires d'amorces pour l'amplification d'au moins 20 cibles d'amplification de CFTR différentes suspectées de contenir des loci mutants ou polymorphiques, et où les concentrations des amorces utilisées dans la réaction en chaîne par polymérase sont modulées de sorte que lesdites au moins 20 cibles d'amplification de CFTR différentes soient amplifiées avec une efficacité approximativement équivalente ; et

c) exposer ledit ADN cible amplifié à une pluralité d'essais de détection, où lesdits essais de détection comprennent des essais de clivage invasif comprenant un premier et un second oligonucléotides configurés pour former des structures de clivage invasif afin de détecter une pluralité de mutations et de polymorphismes de CFTR au niveau de loci dans ledit acide nucléique cible amplifié, dans des conditions telles que la présence ou l'absence desdites mutations et desdits polymorphismes de CFTR au niveau desdits loci soit déterminée.

2. Procédé selon la revendication 1, dans lequel lesdites au moins 20 cibles d'amplification de CFTR différentes comprennent trente cibles d'amplification de CFTR différentes ou davantage.

3. Procédé selon la revendication 1, dans lequel lesdites amplification et exposition sont réalisées simultanément.

**Fig. 1**

# Figure 2

| SEQ ID NO | Mutation | Exemplary Pool | 3' blocking group | Oligo type (Arm) | Sequence (5'-3') | $\varepsilon_{260}$ M⁻¹ cm⁻¹ |
|---|---|---|---|---|---|---|
| SEQ ID NO: 296 | 2789+5G>A | 1 | none | invader | TTTGGTTGTGCTGTGGCTCCTTGGAAAGTGAT | 330800 |
| SEQ ID NO: 297 | 2789+5G>A | 1 | hex | probe/DM | CGCGCCGAGGATATTCCATGTCCTATTGTG | 306500 |
| SEQ ID NO: 298 | 2789+5G>A | 1 | none | synthetic target | CAATCTACACAATAGGACATGGAATATTCACTTTCCAAGGAGCCACAGCACAACCAAA | 667000 |
| SEQ ID NO: 299 | R1162X | 1 | none | invader | GTTTACCTTCTGTTGGCATGTCAATGAACTTAAAGACTCT | 428000 |
| SEQ ID NO: 300 | R1162X | 1 | hex | probe/DM | CGCGCCGAGGAGCTCACAGATCGC | 253000 |
| SEQ ID NO: 301 | R1162X | 1 | none | synthetic target | TCAGATGCGATCTGTGAGCTGAGTCTTTAAGTTCATTGACATGCCAACAGAAGGTAAAC | 659000 |
| SEQ ID NO: 302 | R347P | 4 | none | invader | CAGGGAAATTGCCGAGTGACCGCCATGT | 306600 |
| SEQ ID NO: 303 | R347P | 4 | hex | probe/ER24 | ACGGACGCGGAGGGCAGAACAATGCAG | 318200 |
| SEQ ID NO: 304 | R347P | 4 | none | synthetic target | CTCATTCTGCATTGTTCTGCCCATGGCGGTCACTCGGCAATTTCCCTGGG | 488000 |
| SEQ ID NO: 305 | 1898+1G>A | 1 | none | invader | GACTCTCCTTTTGGATACCTAGATGTTTTAACAGAAAAAGAAATATTTGAAAGT | 619900 |
| SEQ ID NO: 306 | 1898+1G>A | 1 | hex | probe/DM | CGCGCCGAGGATATGTTCTTTGAATACCTTACTTAT | 386500 |
| SEQ ID NO: 307 | 1898+1G>A | 1 | none | synthetic target | ATAAGTAAGGTATTCAAAGAACATATCTTTCAAATATTTCTTTTTCTGTTAAAACATCTAGGTATCCAAAAGGAGAGTC | 904800 |
| SEQ ID NO: 308 | 2184delA | 4 | none | invader | CCCCAAACTCTCCAGTCTGTTAAAAGATTATTTTTTC | 393000 |
| SEQ ID NO: 309 | 2184delA | 4 | hex | probe/DM | CGCGCCGAGGGTTTCTGTCCAGGAGACA | 305200 |
| SEQ ID NO: 310 | delI507 | 1 | none | invader | GCTTTGATGACGCTTCTGTATCTATATTCATCATAGGAAACACCAAT | 509300 |
| SEQ ID NO: 311 | delI507 | 1 | hex | probe/DM | CGCGCCGAGGAGATATTTTCTTTAATGGTGCC | 345200 |
| SEQ ID NO: 312 | delI507 | 1 | none | synthetic target | GCCTGGCACCATTAAAGAAAATATCTTTGGTGTTTCCTATGATGAATATAGATACAGAAGCGTCATCAAAGCATGCC | 866600 |
| SEQ ID NO: 313 | G85E | 4 | none | invader | GCCCTTCGGCGATGTTTTTCTGGAGATTTATGTTCTATGT | 409100 |
| SEQ ID NO: 314 | G85E | 4 | hex | probe/ER24 | ACGGACGCGGAGAAATCTTTTTATATTTAGGGGTAAG | 431700 |
| SEQ ID NO: 315 | G85E | 4 | none | synthetic target | AGATCCTTACCCCTAAATATAAAAAGATTTCATAGAACATAAATCTCCAGAAAAAACATCGCCGAAGGGCATTA | 869300 |
| SEQ ID NO: 316 | R117H | 3 | none | invader | AATCATAGCTTCCTATGACCCGGATAACAAGGAGGAACT | 443800 |
| SEQ ID NO: 317 | R117H | 3 | hex | probe/DM | CGCGCCGAGGACTCTATCGCGATTTATCT | 304200 |
| SEQ ID NO: 318 | R117H | 3 | none | synthetic target | ATGCCTAGATAAATCGCGATAGAGTGTTCCTCCTTGTTATCCGGGTCATAGGAAGCTATGATT | 681700 |
| SEQ ID NO: 319 | R560T | 1 | none | invader | CATGAATGACATTTACAGCAAATGCTTGCTAGACCAATAATTAGTTATTCACT | 595000 |
| SEQ ID NO: 320 | R560T | 1 | hex | probe/ER24 | ACGGACGCGGAGGTTGCTAAAGAAATTCTTGCT | 378100 |
| SEQ ID NO: 321 | R560T | 1 | none | synthetic target | CAACGAGCAAGAATTTCTTTAGCAACGTGAATAACTAATTATTGGTCTAGCAAGCATTTGCTGTAAATGTCATTCATGTAAAA | 945400 |
| SEQ ID NO: 322 | 3120+1G>A | 2 | none | invader | GCAATTTTGGATGACCTTCTGCCTCTTACCCATATTTGACTTCATCCAGT | 496000 |
| SEQ ID NO: 323 | 3120+1G>A | 2 | hex | probe/DM | CGCGCCGAGGATATGTAAAAATAAGTACCGTTAA | 397500 |
| SEQ ID NO: 324 | 3120+1G>A | 2 | none | synthetic target | AGACATACTTAACGGTACTTATTTTTACATATCTGGATGAAGTCAAATATGGTAAGAGGCAGAAGGTCATCCAAAATTGCTATATC | 984000 |
| SEQ ID NO: 325 | 3659delC | 2 | none | invader | GAGAGTTGGCCATTCTTGTATGGTTTGGTTGACTTT | 372300 |
| SEQ ID NO: 326 | 3659delC | 2 | hex | probe/DM | CGCGCCGAGGGTAGGTTTACCTTCTGTTGG | 302800 |
| SEQ ID NO: 327 | 3659delC | 2 | none | synthetic target | CATGCCAACAGAAGGTAAACCTACAAGTCAACCAAACCATACAAGAATGGCCAACTCTC | 679800 |
| SEQ ID NO: 328 | A455E | 1 | none | invader | CCTGAAAGATATTAATTTCAAGATAGAAAGAGGACAGTTGTTGGT | 531000 |
| SEQ ID NO: 329 | A455E | 1 | hex | probe/ER24 | ACGGACGCGGAGGGTTGCTGGATCCA | 298100 |
| SEQ ID NO: 330 | A455E | 1 | none | synthetic target | CCAGTGGATCCAGCAACCTCCAACAACTGTCCTCTTTCTATCTTGAAATTAATATCTTTCAGG | 661000 |
| SEQ ID NO: 331 | 1078delT | 2 | none | invader | AGTGCATAGGGAAGCACAGATAAAAACACCACAT | 413500 |
| SEQ ID NO: 332 | 1078delT | 2 | hex | probe/DM | CGCGCCGAGGAGAACCCTGAGAAGAAGAA | 355400 |
| SEQ ID NO: 333 | 1078delT | 2 | none | synthetic target | AGCCTTCTTCTTCTCAGGGTTCTTGTGGTGTTTTTATCTGTGCTTCCCTATGCACT | 533300 |
| SEQ ID NO: 334 | G551D | 2 | none | invader | GCAGAGAAAGACAATATAGTTCTTGGAGAAGGTGGAATCACACTGAGTGGAGT | 628200 |
| SEQ ID NO: 335 | G551D | 2 | hex | probe/DM | CGCGCCGAGGATCAACGAGCAAGAATTTCT | 343800 |
| SEQ ID NO: 336 | G551D | 2 | none | synthetic target | CTTGCTAAAGAAATTCTTGCTCGTTGATCTCCACTCAGTGTGATTCCACCTTCTCCAAGAACTATATTGTCTTTCTCTGCAAACTT | 883100 |
| SEQ ID NO: 337 | I148T | 1 | none | invader | AAATCAAACTAAACATAGCTATTCTCATCTGCATTCCAT | 432400 |
| SEQ ID NO: 338 | I148T | 1 | hex | probe/ER24 | ACGGACGCGGAGGTGTGATGAAGGCCAAA | 350200 |
| SEQ ID NO: 339 | I148T | 1 | none | synthetic target | CCATTTTTGACCTTCATCACACTGGAATGCAGATGAGAATAGCTATGTTTAGTTTGATTT | 643100 |
| SEQ ID NO: 340 | N1303K | 2 | none | invader | CCATATTTCTTGATCACTCCACTGTTCATAGGGATCCAAT | 414700 |
| SEQ ID NO: 341 | N1303K | 2 | hex | probe/DM | CGCGCCGAGGCTTTTTCTAAATGTTCCAGAAAAA | 391200 |
| SEQ ID NO: 342 | N1303K | 2 | none | synthetic target | ATTTATTTTTCTGGAACATTTAGAAAAAAGTTGGATCCCTATGAACAGTGGAGTGATCAAGAAATATGGAAAG | 867100 |
| SEQ ID NO: 343 | 711+1G>T | 2 | none | invader | GCCTTTCCAGTTGTATAATTTATAACAATAGTGCCTAAAAGATTAAATCAATAGGTACATT |  |
| SEQ ID NO: 344 | 711+1G>T | 2 | hex | probe/DM | CGCGCCGAGGAATTCATCAAATTTGTTCAGGT |  |
| SEQ ID NO: 345 | 711+1G>T | 2 | none | synthetic target | ACCTGAACAAATTTGATGAATTATGTACCTATTGATTTAATCTTTTAGGCACTATTGTTATAAATTATACAACTGGAAAGGC | 927000 |
| SEQ ID NO: 346 | 1717-1G>A | 3 | none | invader | GCCTTTCAAATTCAGATTGAGCATACTAAAAGTGACTCTCTAATTTTCTATTTTTGGTAATAT | 685000 |
| SEQ ID NO: 347 | 1717-1G>A | 3 | hex | probe/DM | CGCGCCGAGGAGCATCTCCAAGTTTGC | 294500 |
| SEQ ID NO: 348 | 1717-1G>A | 3 | none | synthetic target | CTCTGCAAACTTGGAGATGTCTTATTACCAAAAATAGAAAATTAGAGAGTCACTTTTAGTATGCTCAATCTGAATTTGAAAGGCACATC | 1010000 |
| SEQ ID NO: 349 | W1282X | 3 | none | invader | GCTCACCTGTGGTATCACTCCAAAGGCTTTCCTA | 345000 |
| SEQ ID NO: 350 | W1282X | 3 | hex | probe/DM | CGCGCCGAGGTCACTGTTGCAAAGTTATTG | 327800 |
| SEQ ID NO: 351 | W1282X | 3 | none | synthetic target | GATTCAATAACTTTGCAACAGTGAAGGAAAGCCTTTGGAGTGATACCACAGGTGAGCAA | 683000 |
| SEQ ID NO: 352 | 3849+10kbC>T | 2 | none | invader | CAAGAGTCTTCCATCTGTTGCAGTATTAAAATGGA | 390000 |
| SEQ ID NO: 353 | 3849+10kbC>T | 2 | hex | probe/DM | CGCGCCGAGGTGAGTAAGACACCCTGAAA | 327400 |
| SEQ ID NO: 354 | 3849+10kbC>T | 2 | none | synthetic target | TTCCTTTCAGGGTGTTCACTCACCATTTTAATACTGCAACAGATGGAAGACTCTTG | 601000 |
| SEQ ID NO: 355 | R553X | 4 | none | invader | CATTTACAGCAAATGCTTGCTAGACCAATAATTAGTTATTCACCTTGCTAAAGAAATTCTTGCTG |  |
| SEQ ID NO: 356 | R553X | 4 | hex | probe/DM | CGCGCCGAGGCATTGACCTCCACTCAGT |  |

EP 1 567 540 B1

62

| SEQ ID NO | | | | | | |
|---|---|---|---|---|---|---|
| SEQ ID NO: 357 | R553X | 4 | none | synthetic target | ACTGAGTGGAGGTCAATGAGCAAGAATTTCTTTAGCAAGGTGAATAACTAATTATTGGTCTAGCAAGCATTTGCTGTAAATG | |
| SEQ ID NO: 358 | G542X | 4 | none | invader | TCCAAGTTTGCAGAGAAAGACAATATAGTTCTTTC | |
| SEQ ID NO: 359 | G542X | 4 | hex | probe/DM | CGCGCCGAGGGAGAAGGTGGAATCACA | |
| SEQ ID NO: 360 | G542X | 4 | none | synthetic target | TGTGATTCCACCTTCTCAAAGAACTATATTGTCTTTCTCTGCAAACTTGGA | |
| SEQ ID NO: 361 | 621+1G>T | 3 | none | invader | CCTTCATCACATTGGAATGCAGATGAGAATAGCTATGTTTAGTTTGATTTATAAGAAGC | 664000 |
| SEQ ID NO: 362 | 621+1G>T | 3 | hex | probe/DM | CGCGCCGAGGTTAATACTTCCTTGCACAGG | 311900 |
| SEQ ID NO: 363 | 621+1G>T | 3 | none | synthetic target | GGGGCCTGTGCAAGGAAGTATTAACTTCTTATAAATCAAACTAAACATAGCTATTCTCATCTGCATTCCAATGTGATGAAGGCCAA | 965000 |
| SEQ ID NO: 364 | R334W | 2 | none | invader | CGCAGAACAATGCAGAATGAGATGGTGGTGAATATTTTCCT | 487000 |
| SEQ ID NO: 365 | R334W | 2 | hex | probe/DM | CGCGCCGAGGAGAGGATGATTCCTTTGATTA | 336800 |
| SEQ ID NO: 366 | R334W | 2 | none | synthetic target | TGCACTAATCAAAGGAATCATCCTCTGGAAAATATTCACCACCATCTCATTCTGCATTGTTCTGCG | 703000 |
| SEQ ID NO: 367 | Internal control | all | none | Invader | tgtacttcatgctgtctacactaagagagaatgagagacacaca | 503500 |
| SEQ ID NO: 368 | Internal control | all | hex | Probe/SNP4b | tccgcgcgtcctgaagaagcaccaatcatg | 321200 |
| SEQ ID NO: 369 | Internal control | all | none | Synthetic Target | tttcatgattggtgcttcttcagtgtgtctctcattctctcttagtgtagacagcatgaagtacattt | 698200 |
| SEQ ID NO: 370 | FRET cassette | all | Hex | DM/FAM FRET | Y-tct-X-agc-cgg-ttt-tcc-ggc-tga-gac-ctc-ggc-gcg-hex | |
| SEQ ID NO: 371 | FRET cassette | 1, 2, 5 | Hex | ER24/FAM FRET | Y-tct-X-agc-cgg-ttt-tcc-ggc-tga-gac-tcc-gcg-tcc-gt-hex | |
| SEQ ID NO: 372 | FRET cassette | all | Hex | SNP4b/Red FRET | Y-tct-X-tcg-gcc-ttt-tgg-ccg-aga-gag-gac-gcg-cgg-a-hex | |
| SEQ ID NO: 373 | delF508 | delF508 | none | Invader | TGATGACGCTTCTGTATCTATATTCATCATAGGAAACACA | 441500 |
| SEQ ID NO: 374 | delF508 | delF508 | Hex | WT Probe | CGCGCCGAGGCAAAGATGATATTTTCTTTAATGGT | 382200 |
| SEQ ID NO: 375 | delF508 | delF508 | Hex | Mut Probe | AGCTCGTCCGACACAATAATATTTTCTTTAATGGTGCCA | 418100 |
| SEQ ID NO: 376 | delF508 | delF508 | Hex | DM/FAM FRET | Y-tct-X-agc-cgg-ttt-tcc-ggc-tga-gac-gcg-hex | 347150 |
| SEQ ID NO: 377 | delF508 | delF508 | Hex | Wingra/Red FRET | Y-tct-X-tcg-gcc-ttt-tgg-ccg-aga-gat-gtc-gga-cga-gct-hex | 390400 |
| SEQ ID NO: 378 | delF508 | delF508 | none | WT Target | TGCCTGGCACCATTAAAGAAAATATCATCTTTGGTGTTTCCTATGATGAATATAGATACAGAAGCGTCATCAAA | 837500 |
| SEQ ID NO: 379 | delF508 | delF508 | none | Mut Target | ATGCCTGGCACCATTAAAGAAAATATCATTGGTGTTTCCTATGATGAATATAGATACAGAAGCGTCATCAAA | 828100 |
| SEQ ID NO: 380 | 2184delA | 2184delA | none | Invader | CTTCCTTTTTTCCCCAAACTCTCCAGTCTGTTTAAAAGATTGTTTA | |
| SEQ ID NO: 381 | 2184delA | 2184delA | hex | MUT probe/DM | CGCGCCGAGGTTTGTTTCTGTCCAGGAG | |
| SEQ ID NO: 382 | 2184delA | 2184delA | hex | WT probe/ER24 | ACGGACGCGGAGTTTGTTTCTGTCCAGGAG | |
| SEQ ID NO: 383 | | | | DM/FAM FRET | Y-tct-X-agc-cgg-ttt-tcc-ggc-tga-gac-ctc-ggc-gcg-hex | |
| SEQ ID NO: 384 | | | | ER24/RED FRET | Y-tct-X-agc-cgg-ttt-tcc-ggc-tga-gac-tcc-gcg-tcc-gt-hex | |
| SEQ ID NO: 385 | 3199del6 | 3199del6 | none | WT synthetic target | AAAATATGCTCTCAACATAATAAAAGCCACTATCACTGGCACTGTTGCAACAAAGATGTAGGGTTGTAA | |
| SEQ ID NO: 386 | 3199del6 | | none | MUT synthetic target | AAAATATGCTCTCAACATAATAAAAGCCACTGGCACTGTTGCAACAAAGATGTAGGGTTGTAA | |
| SEQ ID NO: 387 | 3199del6 | | none | Invader | TGTCGCAGTTTTACAACCCTACATCTTTGTTGCAACAGTGCCT | |
| SEQ ID NO: 388 | 3199del6 | | hex | WT probe/ ER24 | ACGGACGCGGAGAGTGATAGTGGCTTTTATTATGTT | |
| SEQ ID NO: 389 | 3199del6 | | hex | MUT probe/ ER38 | AGGCCACGGACGAGTGGCTTTTATTATGTTGAGAG | |
| SEQ ID NO: 390 | | | Hex | ER24/FAM | Y-tct-X-agc-cgg-ttt-tcc-ggc-tga-gac-tcc-gcg-tcc-gt-hex | |
| SEQ ID NO: 391 | | | Hex | ER38/RED | 5'-Y-TCT-X-TCG GCC TTT TGG CCG AGA GAC GTC CGT GGC CT-hexanediol -3' | |
| SEQ ID NO: 392 | 711+1G>T | 2 | hex | probe/ER24 | ACGGACGCGGAGAATTCATCAAATTTGTTCAGG | |
| SEQ ID NO: 393 | 3849+10kb | 2 | hex | probe/ER24 | ACGGACGCGGAGTGAGTAAGACACCCTGAAA | |

X = Quencher = Z28
Y = Dye = FAM for DM and ER24 and Y = Z35 (or Redmond Red) for SNP4b
X = Quencher = Z28
Y = Dye = FAM for DM and Y = Z35 (or Redmond Red) for Wingra

Y = Dye = FAM for DM and Y = Z35 (or Redmond Red) for ER24
Y = Dye = FAM for ER24 and Y = Z35 (or Redmond Red) for ER38

FIGURE 3

| SEQ ID NO | Mutation | Oligo Name | Oligo type | Sequence (5'-3') | ε260 M-1 cm-1 | Length |
|---|---|---|---|---|---|---|
| SEQ ID NO:1 | A455E | 1297-83-03 | Invader | CCTGAAAGATATTAATTTCAAGATAGAAAGAGGACAGTTGTTGGT | 465900 | 45 |
| SEQ ID NO:2 | A455E | 1657-96-01 | WT ER38 | AGGGCCACGGACGCGGTTGCTGGATC-hex | 235400 | 29 |
| SEQ ID NO:3 | A455E | 1297-31-05 | MT ER24 | ACGGACGCGGAGAGGTTGCTGGATCCA-hex | 266300 | 31 |
| SEQ ID NO:4 | A455E | 1618-54-14 | WT Syn Target (ST) | CCAGTGGATCCAGCAACCGCCAACAACTGTCCTCTTTCTATCTTGAAATTAATATCTTTCAGGT | 600200 | 64 |
| SEQ ID NO:5 | A455E | 1618-54-18 | Mut Syn Target (ST) | CCAGTGGATCCAGCAACCTCCAACAACTGTCCTCTTTCTATCTTGAAATTAATATCTTTCAGGT | 598800 | 64 |
| SEQ ID NO:6 | A455E | 23-204 | FAM FRET - WT | FAM-tct-X-agc-cgg-ttt-tcc-ggc-tga-gac-gtc-cgt-ggc-ct-hex | | |
| SEQ ID NO:7 | A455E | 23-755 | Red FRETw/FAM Stem-MT | Z35-tct-X-agc-cgg-ttt-tcc-ggc-tga-gac-tcc-gcg-tcc-gt-hex | | |
| SEQ ID NO:8 | 3659delC | 1618-60-10 | Invader | AGTTCATTGACATGCCAACAGAAGGTAAACCTAT | 343500 | 34 |
| SEQ ID NO:9 | 3659delC | 1618-60-12 | WT ER24 | ACGGACGCGGAGCCAAGTCAACCAAACCA-hex | 290200 | 33 |
| SEQ ID NO:10 | 3659delC | 1618-60-17 | MT ER38 | AGGGCCACGGACGCAAGTCAACCAAACCATAC-hex | 307600 | 35 |
| SEQ ID NO:11 | 3659delC | 1618-60-14 | WT ST | TCTTGTATGGTTTGGTTGACTTGGTAGGTTTACCTTCTGTTGGCATGTCAATGAACT | 531400 | 57 |
| SEQ ID NO:12 | 3659delC | 1618-60-18 | MT ST | TCTTGTATGGTTTGGTTGACTTGTAGGTTTACCTTCTGTTGGCATGTCAATGAACT | 521300 | 56 |
| SEQ ID NO:13 | 3659delC | 23-210 | FAM FRET - WT | FAM-tct-X-agc-cgg-ttt-tcc-ggc-tga-gac-tcc-gcg-tcc-gt-hex | | |
| SEQ ID NO:14 | 3659delC | 23-211 | Red FRET - MT | Z35-tct-X-tcg-gcc-ttt-tgg-ccg-aga-gac-gtc-cgt-ggc-ct-hex | | |
| SEQ ID NO:15 | G85E | 1614-57-10 | Invader | GCCCTTCGGCGATGTTTTTTCTGGAGATTTATGTTCTATGT | 374700 | 41 |
| SEQ ID NO:16 | G85E | 1614-57-13 | WT ER24 | ACGGACGCGGAGGAATCTTTTTATATTTAGGGGTAAG-hex | 372800 | 41 |
| SEQ ID NO:17 | G85E | 1614-57-18 | MT ER38 | AGGGCCACGGACGAAATCTTTTTATATTTAGGGGTAAGG-hex | 381500 | 42 |
| SEQ ID NO:18 | G85E | 1614-57-12 | WT ST | AGATCCTTACCCCTAAATATAAAAAGATTCCATAGAACATAAATCTCCAGAAAAAACATCGCCGAAGGGCATT | 742300 | 73 |
| SEQ ID NO:19 | G85E | 1614-57-16 | MT ST | AGATCCTTACCCCTAAATATAAAAAGATTTCATAGAACATAAATCTCCAGAAAAAACATCGCCGAAGGGCATT | 743200 | 73 |
| SEQ ID NO:20 | G85E | 1055-48-09 | FAM FRET-WT | FAM-tct-X-agc-cgg-ttt-tcc-ggc-tga-gac-tcc-gcg-tcc-gt-hex | | |
| SEQ ID NO:21 | G85E | 1230-33-01 | Red FRETw/FAM Stem-MT | Z35-tct-X-agc-cgg-ttt-tcc-ggc-tga-gac-gtc-cgt-ggc-ct-hex | | |
| SEQ ID NO:22 | N1303K | 16145404 | WT Probe ER24 | ACGGACGCGGAGGTTTTTTCTAAATGTTCCAGAAAAA-hex | 369300 | 41 |
| SEQ ID NO:23 | N1303K | 16145406 | MT Probe DM | CGCGCCGAGGCTTTTTTCTAAATGTTCCAGAAAA-hex | 334500 | 39 |
| SEQ ID NO:24 | N1303K | 16145401 | Invader | TTCTTGATCACTCCACTGTTCATAGGGATCCAAT | 308600 | 34 |
| SEQ ID NO:25 | N1303K | 16145403 | WT ST | TTATTTTTTTCTGGAACATTTAGAAAAAACTTGGATCCCTATGAACAGTGGAGTGATCAAGAAATT | 650400 | 65 |
| SEQ ID NO:26 | N1303K | 16145407 | MT ST | TTATTTTTTTCTGGAACATTTAGAAAAAAGTTGGATCCCTATGAACAGTGGAGTGATCAAGAAATT | 654900 | 65 |
| SEQ ID NO:27 | N1303K | 23-428 | DM FAM FRET MT | FAM-tct-X-agc-cgg-ttt-tcc-ggc-tga-gac-ctc-ggc-gcg-hex | | 53 |
| SEQ ID NO:28 | N1303K | 23-394 | ER24 RED FRET WT | Z35-tct-X-tcg-gcc-ttt-tgg-ccg-aga-gac-tcc-gcg-tcc-gt-hex | | 56 |
| SEQ ID NO:29 | D1270N | 1645-18-10 | Invader | TCAGCTTTTTTGAGACTACTGAACACTGAAGGAGAAATCCAGATCT | 451000 | 46 |
| SEQ ID NO:30 | D1270N | 1665-93-01 | WT DM | CGCGCCGAGGGATGGTGTGTCTTGGGA-hex | 257300 | 31 |
| SEQ ID NO:31 | D1270N | 1645-18-17 | MT Probe (ER24) | ACGGACGCGGAGAATGGTGTGTCTTGGG-hex | 275600 | 32 |
| SEQ ID NO:32 | D1270N | 1645-18-12 | WT ST | GAATCCCAAGACACACCATCGATCTGGATTTCTCCTTCAGTGTTCAGTAGTCTCAAAAAAGCTGATAT | 659200 | 68 |
| SEQ ID NO:33 | D1270N | 1645-18-16 | MT ST | GAATCCCAAGACACACCATTGATCTGGATTTCTCCTTCAGTGTTCAGTAGTCTCAAAAAAGCTGATAT | 659500 | 68 |
| SEQ ID NO:34 | D1270N | 23-746 | DM Red FRETw/FAM stem- WT | Z35-tct-X-agc-cgg-ttt-tcc-ggc-tga-gac-ctc-gcg-gcg-hex | | |
| SEQ ID NO:35 | D1270N | 23-210 | ER24 FAM FRET - MT | FAM-tct-X-agc-cgg-ttt-tcc-ggc-tga-gac-tcc-gcg-tcc-gt-hex | | |
| SEQ ID NO:36 | R560T | 1614-55-01 | invader | GACATTTACAGCAAATGCTTGCTAGACCAATAATTAGTTATTCACT | 452500 | 46 |
| SEQ ID NO:37 | R560T | 1614-55-04 | Wt probe (ER24) | ACGGACGCGGAGCTTGCTAAAGAAATTCTTGCT-hex | 308700 | 37 |
| SEQ ID NO:38 | R560T | 1614-55-06 | Mt probe (DM) | CGCGCCGAGGGTTGCTAAAGAAATTCTTGCT-hex | 283300 | 35 |
| SEQ ID NO:39 | R560T | 1614-55-03 | WT ST | AACGAGCAAGAATTTCTTTAGCAAGGTGAATAACTAATTATTGGTCTAGCAAGCATTTGCTGTAAATGTCT | 706600 | 71 |
| SEQ ID NO:40 | R560T | 1614-55-07 | MT ST | AACGAGCAAGAATTTCTTTAGCAACGTGAATAACTAATTATTGGTCTAGCAAGCATTTGCTGTAAATGTCT | 703300 | 71 |
| SEQ ID NO:41 | R560T | 23-210 | ER24 FAM FRET – WT | FAM-tct-X-agc-cgg-ttt-tcc-ggc-tga-gac-tcc-gcg-tcc-gt-hex | | |
| SEQ ID NO:42 | R560T | 23-205 | DM Red FRET– MT | Z35-tct-X-tcg-gcc-ttt-tgg-ccg-aga-gac-ctc-ggc-gcg-hex | | |
| SEQ ID NO:43 | 621+1G>T | 1618-48-10 | Invader | CCTTCATCACATTGGAATGCAGATGAGAATAGCTATGTTTAGTTTGATTTATAAGAAGC | 588400 | 59 |
| SEQ ID NO:44 | 621+1G>T | 1665-71-02 | WT ER24 | ACGGACGCGGAGGTAATACTTCCTTGCAC-hex | 277800 | 33 |
| SEQ ID NO:45 | 621+1G>T | 1618-48-15 | MT Probe (DM) | CGCGCCGAGGTTAATACTTCCTTGCACAGG-hex | 267700 | 35 |
| SEQ ID NO:46 | 621+1G>T | 1618-48-14 | WT ST | GGGGCCTGTGCAAGGAAGTATTACCTTCTTATAAATCAAACTAAACATAGCTATTCTCATCTGCATTCCAATGTGATGAAGG | 825700 | 85 |
| SEQ ID NO:47 | 621+1G>T | 1618-48-18 | MT ST | GGGGCCTGTGCAAGGAAGTATTAACTTCTTATAAATCAAACTAAACATAGCTATTCTCATCTGCATTCCAATGTGATGAAGG | 830500 | 85 |
| SEQ ID NO:48 | 621+1G>T | 23-755 | ER24 Red FRET w/Fam stem– WT | Z35-tct-X-agc-cgg-ttt-tcc-ggc-tga-gac-tcc-gcg-tcc-gt-hex | | |
| SEQ ID NO:49 | 621+1G>T | 23-428 | DM FAM FRET– MT | FAM-tct-X-agc-cgg-ttt-tcc-ggc-tga-gac-ctc-ggc-gcg-hex | | |
| SEQ ID NO:50 | 1717-1G>A | 1548-76-07 | Invader | GATGTGCCTTTCAAATTCAGATTGAGCATACTAAAAGTGACTCTCTAATTTTCTATTTTTGGTAATC | 644800 | 67 |
| SEQ ID NO:51 | 1717-1G>A | 1657-96-02 | WT probe (DM) | CGCGCCGAGGAGGACATCTGCAAGTTTG-hex | 266500 | 32 |
| SEQ ID NO:52 | 1717-1G>A | 1657-40-02 | MT probe (ER24) | ACGGACGCGGAGAAGACATGTCCAAGTTTG-hex | 298500 | 34 |
| SEQ ID NO:53 | 1717-1G>A | 1618-56-14 | WT ST | CTCTGCAAACTTGGAGATGTCCTATTACCAAAAATAGAAAATTAGAGAGTCACTTTTAGTATGCTCAATCTGAATTTGAAAGG | 863600 | 87 |
| SEQ ID NO:54 | 1717-1G>A | 1618-56-18 | MT ST | CTCTGCAAACTTGGAGATGTCTTATTACCAAAAATAGAAAATTAGAGAGTCACTTTTAGTATGCTCAATCTGAATTTGAAAGG | 864500 | 87 |

FIGURE 3

| SEQ ID NO | Mutation | Oligo Name | Oligo type | Sequence (5'-3') | ε260 M-1 cm-1 | Length |
|---|---|---|---|---|---|---|
| SEQ ID NO:55 | 1717-1G>A | 23-746 | DM Red FRET w/FAM stem– WT | Z35-tct-X-agc-cgg-ttt-tcc-ggc-tga-gac-ctc-ggc-gcg-hex | | |
| SEQ ID NO:56 | 1717-1G>A | 23-210 | ER24 FAM FRET– MT | FAM-tct-X-agc-cgg-ttt-tcc-ggc-tga-gac-tcc-gcg-tcc-gt-hex | | |
| SEQ ID NO:57 | 1078delT | 1614-60-01 | Invader | CCTTTGATTAGTGCATAGGGAAGCACAGATAAAAACACCACAT | 434600 | 43 |
| SEQ ID NO:58 | 1078delT | 1614-60-02 | WT Probe DM | CGCGCCGAGGAAGAACCCTGAGAAGAAGA-hex | 298900 | 29 |
| SEQ ID NO:59 | 1078delT | 1614-60-08 | MT Probe ER24 | ACGGACGCGGAGAGAACCCTGAGAAGAAGAA-hex | 329000 | 31 |
| SEQ ID NO:60 | 1078delT | 1614-60-03 | WT ST | AGCCTTCTTCTTCTCAGGGTTCTTTGTGGTGTTTTTATCTGTGCTTCCCTATGCACTAATCAAAGGAA | 623300 | 68 |
| SEQ ID NO:61 | 1078delT | 1614-60-07 | MT ST | AGCCTTCTTCTTCTCAGGGTTCTTGTGGTGTTTTTATCTGTGCTTCCCTATGCACTAATCAAAGGAA | 615200 | 67 |
| SEQ ID NO:62 | 1078delT | 23-428 | DM FAM FRET- WT | FAM-tct-X-agc-cgg-ttt-tcc-ggc-tga-gac-ctc-ggc-gcg-hex | | |
| SEQ ID NO:63 | 1078delT | 23-755 | ER24 Red FRET w/FAM Stem- MT | Z35-tct-X-agc-cgg-ttt-tcc-ggc-tga-gac-tcc-gcg-tcc-gt-hex | | |
| SEQ ID NO:64 | R347P | 1618-62-01 | Invader | CAGGGAAATTGCCGAGTGACCGCCATGT | 271100 | 28 |
| SEQ ID NO:65 | R347P | 1657-40-04 | WT Probe (DM) | CGCGCCGAGGCGCAGAACAATGCAGAAT-hex | 275800 | 32 |
| SEQ ID NO:66 | R347P | 1696-93-05 | MT ER24 | ACGGACGCGGAGGGCAGAACAATGCA-hex | 266700 | 30 |
| SEQ ID NO:67 | R347P | 1618-62-05 | WT ST | CTCATTCTGCATTGTTCTGCGCATGGCGGTCACTCGGCAATTTCCCTGGGT | 452500 | 51 |
| SEQ ID NO:68 | R347P | 1618-62-09 | MT ST | CTCATTCTGCATTGTTCTGCCCATGGCGGTCACTCGGCAATTTCCCTGGGT | 450200 | 51 |
| SEQ ID NO:69 | R347P | 23-394 | ER24 Red FRET – MT | Z35-tct-X-tcg-gcc-ttt-tgg-ccg-aga-gac-tcc-gcg-tcc-gt-hex | | |
| SEQ ID NO:70 | R347P | 23-428 | DM FAM FRET– WT | FAM-tct-X-agc-cgg-ttt-tcc-ggc-tga-gac-ctc-ggc-gcg-hex | | |
| SEQ ID NO:71 | 2184delA | 1532-85-01 | Mt probe (DM) | CGCGCCGAGGTTTGTTTCTGTCCAGGAG-hex | 257300 | 32 |
| SEQ ID NO:72 | 2184delA | 1582-25-02 | Wt probe (Er24) | ACGGACGCGGAGTTTGTTTCTGTCCAGGAG-hex | 295500 | 35 |
| SEQ ID NO:73 | 2184delA | 1532-85-04 | invader | CTTCCTTTTTTCCCCAAACTCTCCAGTCTGTTTAAAAGATTGTTTA | 418200 | 46 |
| SEQ ID NO:74 | 2184delA | 1660-88-17 | plasmid Het target | | | |
| SEQ ID NO:75 | 2184delA | 23-210 | ER24 FAM FRET-Wt | FAM-tct-X-agc-cgg-ttt-tcc-ggc-tga-gac-tcc-gcg-tcc-gt-hex | | |
| SEQ ID NO:76 | 2184delA | 23-746 | DM Red FRET w/FAM stem-Mt | Z35-tct-X-agc-cgg-ttt-tcc-ggc-tga-gac-ctc-ggc-gcg-hex | | |
| SEQ ID NO:77 | V520F | 16186602 | WT Probe (DM) | CGCGCCGAGGCGCTTCTGTATCTATATTCATCA-hex | 304500 | 33 |
| SEQ ID NO:78 | V520F | 16186607 | MT Probe (ER24) | ACGGACGCGGAGAGCTTCTGTATCTATATTCATCAT-hex | 348600 | 36 |
| SEQ ID NO:79 | V520F | 16186601 | invader | CACATAGTTTCTTACCTCTTCTAGTTGGCATGCTTTGATGAT | 386600 | 42 |
| SEQ ID NO:80 | V520F | 16186605 | WT Synthetic Target | TCCTATGATGAATATAGATACAGAAGCGTCATCAAAGCATGCCAACTAGAAGAGGTAAGAAACTATGTGAAT | 747300 | 72 |
| SEQ ID NO:81 | V520F | 16186609 | MT Synthetic Target | TCCTATGATGAATATAGATACAGAAGCTTCATCAAAGCATGCCAACTAGAAGAGGTAAGAAACTATGTGAAT | 744100 | 72 |
| SEQ ID NO:82 | V520F | 23-428 | DM FAM FRETWT | FAM-tct-X-agc-cgg-ttt-tcc-ggc-tga-gac-ctc-ggc-gcg-hex | | |
| SEQ ID NO:83 | V520F | 23-394 | ER24 RED FRET MT | Z35-tct-X-tcg-gcc-ttt-tgg-ccg-aga-gac-tcc-gcg-tcc-gt-hex | | |
| SEQ ID NO:84 | R347H | 1618-68-10 | Invader | GGAAAATATTCACCACCATCTCATTCTGCATTGTTCTGCT | 370700 | 40 |
| SEQ ID NO:85 | R347H | 1618-68-12 | WT Probe (ER24) | ACGGACGCGGAGGCATGGCGGTCAC-hex | 242300 | 29 |
| SEQ ID NO:86 | R347H | 1665-93-04 | MT ER38 | AGGCCACGGACGACATGGCGGTCAC-hex | 242300 | 29 |
| SEQ ID NO:87 | R347H | 1618-68-14 | WT ST | GCCGAGTGACCGCCATGCGCAGAACAATGCAGAATGAGATGGTGGTGAATATTTTCCT | 643800 | 58 |
| SEQ ID NO:88 | R347H | 1618-68-18 | MT ST | GCCGAGTGACCGCCATGTGCAGAACAATGCAGAATGAGATGGTGGTGAATATTTTCCT | 645100 | 58 |
| SEQ ID NO:89 | R347H | 23-210 | ER24 FAM FRET – WT | FAM-tct-X-agc-cgg-ttt-tcc-ggc-tga-gac-tcc-gcg-tcc-gt-hex | | |
| SEQ ID NO:90 | R347H | 23-752 | ER38 Red FRET w/FAM stem – MT | Z35-tct-X-agc-cgg-ttt-tcc-ggc-tga-gac-gtc-cgt-ggc-ct-hex | | |
| SEQ ID NO:91 | 2183AA>G | 1645-26-10 | invader AS | GAGATGCTCCTGTCTCCTGGACACAAACAAAAT | 335200 | 34 |
| SEQ ID NO:92 | 2183AA>G | 1667-19-08 | Wt ER24 | ACGGACGCGGAGAACAATCTTTTAAACAGACTG-hex | 331900 | 37 |
| SEQ ID NO:93 | 2183AA>G | 1667-19-10 | Mt DM | CGCGCCGAGGGCAATCTTTTAAACAGACTG-hex | 286500 | 34 |
| SEQ ID NO:94 | 2183AA>G | 1645-26-12 | WT ST | CCCCAAACTCTCCAGTCTGTTTAAAAGATTGTTTTTTTGTTTCTGTCCAGGAGACAGGAGCATCTCCTTCT | 654900 | 71 |
| SEQ ID NO:95 | 2183AA>G | 1645-26-16 | MT ST | CCCCAAACTCTCCAGTCTGTTTAAAAGATTGCTTTTTGTTTCTGTCCAGGAGACAGGAGCATCTCCTTCT | 644100 | 70 |
| SEQ ID NO:96 | 2183AA>G | 23-755 | ER24 Red FRET w/FAM stem – WT | Z35-tct-X-agc-cgg-ttt-tcc-ggc-tga-gac-tcc-gcg-tcc-gt-hex | | |
| SEQ ID NO:97 | 2183AA>G | 23-428 | DM FAMFRET – MT | FAM-tct-X-agc-cgg-ttt-tcc-ggc-tga-gac-ctc-ggc-gcg-hex | | |
| SEQ ID NO:98 | R334W | 1297-83-04 | Invader | CGCAGAACAATGCAGAATGAGATGGTGGTGAATATTTTCCT | 409900 | 41 |
| SEQ ID NO:99 | R334W | 1618-64-02 | WT DM | CGCGCCGAGGGGAGGATGATTCCTTTGAT-hex | 267600 | 34 |
| SEQ ID NO:100 | R334W | 1618-64-07 | MT ER24 | ACGGACGCGGAGAGGATGATTCCTTTGATTA-hex | 316900 | 37 |
| SEQ ID NO:101 | R334W | 1618-64-05 | WT ST | GCACTAATCAAAGGAATCATCCTCCGGAAAATATTCACCACCATCTCATTCTGCATTGTTCTGCGT | 624200 | 66 |
| SEQ ID NO:102 | R334W | 1618-64-09 | MT ST | GCACTAATCAAAGGAATCATCCTCTGGAAAATATTCACCACCATCTCATTCTGCATTGTTCTGCGT | 624500 | 66 |
| SEQ ID NO:103 | R334W | 23-746 | DM Red FRETw/ Fam WT | Z35-tct-X-agc-cgg-ttt-tcc-ggc-tga-gac-ctc-ggc-gcg-hex | | |
| SEQ ID NO:104 | R334W | 23-210 | ER24 FAM FRET MT | FAM-tct-X-agc-cgg-ttt-tcc-ggc-tga-gac-tcc-gcg-tcc-gt-hex | | |
| SEQ ID NO:105 | R117H | 1614-48-10 | Invader | AGAATCATAGCTTCCTATGACCCGGATAACAAGGAGGAACT | 414400 | 41 |
| SEQ ID NO:106 | R117H | 1614-48-11 | WT DM | CGCGCCGAGGGCTCTATCGCGATTTATCTA-hex | 277600 | 34 |
| SEQ ID NO:107 | R117H | 1614-48-18 | MT ER38 | AGGCCACGGACGACTCTATCGCGATTTATCTAG-hex | 317300 | 37 |
| SEQ ID NO:108 | R117H | 1614-48-12 | WT ST | ATGCCTAGATAAATCGCGATAGAGCGTTCCTCCTTGTTATCCGGGTCATAGGAAGCTATGATTCTTCT | 651200 | 68 |

FIGURE 3

| SEQ ID NO | Mutation | Oligo Name | Oligo type | Sequence (5'-3') | ε260 M-1 cm-1 | Length |
|---|---|---|---|---|---|---|
| SEQ ID NO:109 | R117H | 1614-48-16 | MT ST | ATGCCTAGATAAATCGCGATAGAGTGTTCCTCCTTGTTATCCGGGTCATAGGAAGCTATGATTCTTCT | 653300 | 68 |
| SEQ ID NO:110 | R117H | 23-428 | DM FAM FRET WT | FAM-tct-X-agc-cgg-ttt-tcc-ggc-tga-gac-ctc-ggc-gcg-hex | | |
| SEQ ID NO:111 | R117H | 23-211 | ER38 RED FRET MT | Z35-tct-X-tcg-gcc-ttt-tgg-ccg-aga-gac-gtc-cgt-ggc-ct-hex | | |
| SEQ ID NO:112 | 2789+5G>A | 1297-83-06 | Invader | TTTGGTTGTGCTGTGGCTCCTTGGAAAGTGAT | 296200 | 32 |
| SEQ ID NO:113 | 2789+5G>A | 1618-46-13 | WT ER38 | AGGCCACGGACGGTATTCCATGTCCTATTGTG-hex | 292600 | 37 |
| SEQ ID NO:114 | 2789+5G>A | 1618-46-16 | MT ER24 | ACGGACGCGGAGATATTCCATGTCCTATTGTG-hex | 298400 | 37 |
| SEQ ID NO:115 | 2789+5G>A | 1618-46-14 | WT ST | TCTACACAATAGGACATGGAATACTCACTTTCCAAGGAGCCACAGCACAACCAAAT | 554800 | 56 |
| SEQ ID NO:116 | 2789+5G>A | 1618-46-18 | MT ST | TCTACACAATAGGACATGGAATATTCACTTTCCAAGGAGCCACAGCACAACCAAAT | 556700 | 56 |
| SEQ ID NO:117 | 2789+5G>A | 23-211 | ER38 RED FRET WT | Z35-tct-X-tcg-gcc-ttt-tgg-ccg-aga-gac-gtc-cgt-ggc-ct-hex | | |
| SEQ ID NO:118 | 2789+5G>A | 23-210 | ER24 FAM FRET MT | FAM-tct-X-agc-cgg-ttt-tcc-ggc-tga-gac-tcc-gcg-tcc-gt-hex | | |
| SEQ ID NO:119 | 394delTT | 1665-32-10 | Invader | CCTTCGGCGATGTTTTTCTGGAGATTTATGTTCTATGGAATT | 400100 | 43 |
| SEQ ID NO:120 | 394delTT | 1714-28-02 | WT (ER38) | AGGCCACGGACGCTTTTATATTTAGGGGTAAGGATCTC-hex | 371400 | 42 |
| SEQ ID NO:121 | 394delTT | 1680-16-07 | MT (ER24) | ACGGACGCGGAGCTTTATATTTAGGGGTAAGGATCTC-hex | 366000 | 41 |
| SEQ ID NO:122 | 394delTT | 1614-65-12 | WT ST | ACAAATGAGATCCTTACCCCTAAATATAAAAAGATTCCATAGAACATAAATCTCCAGAAAAAACATCGCCGAAGGGC | 788000 | 77 |
| SEQ ID NO:123 | 394delTT | 1614-65-16 | MT ST | ACAAATGAGATCCTTACCCCTAAATATAAAGATTCCATAGAACATAAATCTCCAGAAAAAACATCGCCGAAGGGC | 764000 | 75 |
| SEQ ID NO:124 | 394delTT | 23-211 | ER38 Red FRET – WT | Z35-tct-X-tcg-gcc-ttt-tgg-ccg-aga-gac-gtc-cgt-ggc-ct-hex | | |
| SEQ ID NO:125 | 394delTT | 23-210 | ER24 FAM FRET – MT | FAM-tct-X-agc-cgg-ttt-tcc-ggc-tga-gac-tcc-gcg-tcc-gt-hex | | |
| SEQ ID NO:126 | 3849+10kbC>T | 1618-44-01 | Invader | TGCATTGTACCATGAATAGAACATTTCCTTTCAGGGTGTCTTACTCT | 492000 | 47 |
| SEQ ID NO:127 | 3849+10kbC>T | 1757-50-02 | WT DM | CGCCGCCGAGGGCCATTTTAATAGTGCAACAGA-hex | 308400 | 36 |
| SEQ ID NO:128 | 3849+10kbC>T | 1618-44-07 | Mt probe (ER24) | ACGGACGCGGAGACCATTTTAATACTGCAACAG-hex | 372800 | 37 |
| SEQ ID NO:129 | 3849+10kbC>T | 1618-44-05 | WT ST | CCATCTGTTGCAGTATTAAAATGCCGAGTAAGACACCCTGAAAGGAAATGTTCTATTCATGGTACAATGCAT | 715200 | 72 |
| SEQ ID NO:130 | 3849+10kbC>T | 1618-44-09 | MT ST | CCATCTGTTGCAGTATTAAAATGCTGAGTAAGACACCCTGAAAGGAAATGTTCTATTCATGGTACAATGCAT | 717300 | 72 |
| SEQ ID NO:131 | 3849+10kbC>T | 23-205 | DM Red FRET – WT | Z35-tct-X-tcg-gcc-ttt-tgg-ccg-aga-gac-ctc-ggc-gcg-hex | | |
| SEQ ID NO:132 | 3849+10kbC>T | 23-210 | ER24 FAM FRET – MT | FAM-tct-X-agc-cgg-ttt-tcc-ggc-tga-gac-tcc-gcg-tcc-gt-hex | | |
| SEQ ID NO:133 | W1282X | 1618-42-01 | Invader | GCTCACCTGTGGTATCACTCCAAGGCTTTCCTA | 311700 | 34 |
| SEQ ID NO:134 | W1282X | 1618-42-03 | WT ER24 | ACGGACGCGGAGCCACTGTTGCAAAGTTATT-hex | 300300 | 35 |
| SEQ ID NO:135 | W1282X | 1618-42-06 | MT DM | CGCGCCGAGGTCACTGTTGCAAAGTTATTG-hex | 283200 | 34 |
| SEQ ID NO:136 | W1282X | 1618-42-05 | WT ST | GATTCAATAACTTTGCAACAGTGGAGGAAAGCCTTTGGAGTGATACCACAGGTGAGCAAT | 602000 | 60 |
| SEQ ID NO:137 | W1282X | 1618-42-09 | MT ST | GATTCAATAACTTTGCAACAGTGAAGGAAAGCCTTTGGAGTGATACCACAGGTGAGCAAT | 603900 | 60 |
| SEQ ID NO:138 | W1282X | 23-210 | ER 24 FAM FRET WT | FAM-tct-X-agc-cgg-ttt-tcc-ggc-tga-gac-ctc-ggc-tcc-gt-hex | | |
| SEQ ID NO:139 | W1282X | 23-746 | DM Red FRET w/Fam stem MT | Z35-tct-X-agc-cgg-ttt-tcc-ggc-tga-gac-ctc-ggc-gcg-hex | | |
| SEQ ID NO:140 | G542X | 1614-49-10 | Invader | AATAGGACATCTCCAAGTTTGCAGAGAAAGACAATATAGTTCTTC | 457000 | 45 |
| SEQ ID NO:141 | G542X | 1614-49-14 | WT probe (ER38) | AGGCCACGGACGGGAGAAGGTGGAATCAC | 296800 | 29 |
| SEQ ID NO:142 | G542X | 1614-49-15 | MT probe (DM) | CGCGCCGAGGTGAGAAGGTGGAATCACA | 282400 | 28 |
| SEQ ID NO:143 | G542X | 1614-49-12 | WT ST | TCAGTGTGATTCCACCTTCTCCAAGAACTATATTGTCTTTCTCTGCAAACTTGGAGATGTCCTATTT | 622000 | 67 |
| SEQ ID NO:144 | G542X | 1614-49-16 | MT ST | TCAGTGTGATTCCACCTTCTCAAGAACTATATTGTCTTTCTCTGCAAACTTGGAGATGTCCTATTT | 626800 | 67 |
| SEQ ID NO:145 | G542X | 23-752 | ER38-Red FRET w/FAM Stem–WT | Z35-tct-X-agc-cgg-ttt-tcc-ggc-tga-gac-gtc-cgt-ggc-ct-hex | | |
| SEQ ID NO:146 | G542X | 23-428 | DM-FAM FRET–MT | FAM-tct-X-agc-cgg-ttt-tcc-ggc-tga-gac-ctc-ggc-gcg-hex | | |
| SEQ ID NO:147 | 3120+1G>A | 1618-58-10 | Invader | GCAATTTTGGATGACCTTCTGCCTCTTACCATATTTGACTTCATCCAGT | 496000 | 49 |
| SEQ ID NO:148 | 3120+1G>A | 1745-66-03 | WT ER38 | AGGCCACGGACGGTATGTAAAAATAAGTACCGTT-hex | 348100 | 38 |
| SEQ ID NO:149 | 3120+1G>A | 1618-58-15 | Mt probe (DM) | CGCGCCGAGGATATGTAAAAATAAGTACCGTTAA-hex | 350500 | 38 |
| SEQ ID NO:150 | 3120+1G>A | 1618-58-14 | WT ST | ATACTTAACGGTACTTATTTTTACATACCTGGATGAAGTCAAATATGGTAAGAGGCAGAAGGTCATCCAAAATTGCTAT | 796400 | 79 |
| SEQ ID NO:151 | 3120+1G>A | 1618-58-18 | MT ST | ATACTTAACGGTACTTATTTTTACATATCTGGATGAAGTCAAATATGGTAAGAGGCAGAAGGTCATCCAAAATTGCTAT | 798300 | 79 |
| SEQ ID NO:152 | 3120+1G>A | 23-752 | ER38 Red w/FAM stem – WT | Z35-tct-X-agc-cgg-ttt-tcc-ggc-tga-gac-gtc-cgt-ggc-ct-hex | | |
| SEQ ID NO:153 | 3120+1G>A | 23-428 | DM FAM FRET – MT | FAM-tct-X-agc-cgg-ttt-tcc-ggc-tga-gac-ctc-ggc-gcg-hex | | |
| SEQ ID NO:154 | I148T | 1614-50-10 | Invader | CCTACACCCAGCCATTTTTGGCCTTCATCACAA | 298400 | 33 |
| SEQ ID NO:155 | I148T | 1614-50-11 | WT probe (DM) | CGCGCCGAGGTTGGAATGCAGATGAGAATA-hex | 304400 | 30 |
| SEQ ID NO:156 | I148T | 1614-50-17 | MT probe (ER24) | ACGGACGCGGAGCTGGAATGCAGATGAGAA-hex | 309700 | 30 |
| SEQ ID NO:157 | I148T | 1614-50-12 | WT ST | TAGCTATTCTCATCTGCATTCCAATGTGATGAAGGCCAAAAATGGCTGGGTGTAGGAGT | 576700 | 59 |
| SEQ ID NO:158 | I148T | 1614-50-16 | MT ST | TAGCTATTCTCATCTGCATTCCAGTGTGATGAAGGCCAAAAATGGCTGGGTGTAGGAGT | 575400 | 59 |
| SEQ ID NO:159 | I148T | 23-428 | DM FAM FRET –WT | FAM-tct-X-agc-cgg-ttt-tcc-ggc-tga-gac-ctc-ggc-gcg-hex | | |
| SEQ ID NO:160 | I148T | 23-755 | ER24 Red FRET w/FAM stem–MT | Z35-tct-X-agc-cgg-ttt-tcc-ggc-tga-gac-tcc-gcg-tcc-gt-hex | | |
| SEQ ID NO:161 | 711+1G>T | 1453-08-06 | Invader (+2S) | GCCTTTCCAGTTGTATAATTTATAACAATAGTGCCTAAAAGATTAAATCAATAGGTACATT | 615700 | 61 |
| SEQ ID NO:162 | 711+1G>T | 1791-06-03 | Wt DM | CGCGCCGAGGACTTCATCAAATTGTTCAG-hex | 282800 | 34 |

EP 1 567 540 B1

FIGURE 3

| SEQ ID NO | Mutation | Oligo Name | Oligo type | Sequence (5'-3') | ε260 M-1 cm-1 | Length |
|---|---|---|---|---|---|---|
| SEQ ID NO:163 | 711+1G>T | 1791-06-02 | Mt ER38 | AGGCCACGGACGAATTCATCAAATTTGTTCAGG-hex | 325500 | 37 |
| SEQ ID NO:164 | 711+1G>T | 1614-56-03 | Wt ST | AACAACCTGAACAAATTTGATGAAGTATGTACCTATTGATTTAATCTTTTAGGCACTATTGTTATAAATTATACAACTGGAAAC | 882700 | 88 |
| SEQ ID NO:165 | 711+1G>T | 1614-56-07 | Mt ST | AACAACCTGAACAAATTTGATGAATTATGTACCTATTGATTTAATCTTTTAGGCACTATTGTTATAAATTATACAACTGGAAAG | 880100 | 88 |
| SEQ ID NO:166 | 711+1G>T | 23-204 | ER38 FAM FRET–MT | FAM-tct-X-agc-cgg-ttt-tcc-ggc-tga-gac-gtc-cgt-ggc-ct-hex | | |
| SEQ ID NO:167 | 711+1G>T | 23-746 | DM Red FRET w/FAMstem–WT | Z35-tct-X-agc-cgg-ttt-tcc-ggc-tga-gac-ctc-ggc-gcg-hex | | |
| SEQ ID NO:168 | S549N | 1614-62-01 | Invader | TCACCTTGCTAAAGAAATTCTTGCTCGTTGACCTCCAA | 352200 | 38 |
| SEQ ID NO:169 | S549N | 1614-62-04 | WT probe ( ER24) | ACGGACGCGGAGCTCAGTGTGATTCCACC-hex | 275200 | 33 |
| SEQ ID NO:170 | S549N | 1614-62-08 | MT probe (DM) | CGCGCCGAGGTTCAGTGTGATTCCACC-hex | 247800 | 31 |
| SEQ ID NO:171 | S549N | 1614-62-03 | WT ST | AGAAGGTGGAATCACACTGAGTGGAGGTCAACGAGCAAGAATTTCTTTAGCAAGGTGAATT | 622500 | 61 |
| SEQ ID NO:172 | S549N | 1614-62-07 | MT ST | AGAAGGTGGAATCACACTGAATGGAGGTCAACGAGCAAGAATTTCTTTAGCAAGGTGAATT | 623800 | 61 |
| SEQ ID NO:173 | S549N | 23-755 | ER24 Red FRET w/FAM stem – WT | Z35-tct-X-agc-cgg-ttt-tcc-ggc-tga-gac-tcc-gcg-tcc-gt-hex | | |
| SEQ ID NO:174 | S549N | 23-428 | DM FAM FRET – MT | FAM-tct-X-agc-cgg-ttt-tcc-ggc-tga-gac-ctc-ggc-gcg-hex | | |
| SEQ ID NO:175 | D1152H | 1645-17-10 | Invader | TGAGTACATTGCAGTGGGCTGTAAACTCCAGCATAT | 351900 | 36 |
| SEQ ID NO:176 | D1152H | 1733-73-02 | WT probe (ER24) | ACGGACGCGGAGGATGTGGACTTGGTA-hex | 303500 | 34 |
| SEQ ID NO:177 | D1152H | 1733-73-03 | Mt probe (ER38) | AGGCCACGGACGCATGTGGATAGCTTGGTA-hex | 296300 | 34 |
| SEQ ID NO:178 | D1152H | 1645-17-12 | WT ST | AGACTTACCAAGCTATCCACATCTATGCTGGAGTTTACAGCCCACTGCAATGTACTCATGT | 581000 | 61 |
| SEQ ID NO:179 | D1152H | 1645-17-16 | MT ST | AGACTTACCAAGCTATCCACATGTATGCTGGAGTTTACAGCCCACTGCAATGTACTCATGT | 584500 | 61 |
| SEQ ID NO:180 | D1152H | 23-210 | ER24 FAM FRET – WT | FAM-tct-X-agc-cgg-ttt-tcc-ggc-tga-gac-tcc-gcg-tcc-gt-hex | | |
| SEQ ID NO:181 | D1152H | 23-752 | ER38 Red FRET w/FAM stem – MT | Z35-tct-X-agc-cgg-ttt-tcc-ggc-tga-gac-gtc-cgt-ggc-ct-hex | | |
| SEQ ID NO:182 | 3905insT | 1614-72-12 | Invader | GATCTGGATTTCTCCTTCAGTGTTCAGTAGTCTCAT | 334000 | |
| SEQ ID NO:183 | 3905insT | 1667-91-02 | WT probe (ER38) | AGGCCACGGACGAAAAAGCTGATAACAAAGTACT | 352100 | |
| SEQ ID NO:184 | 3905insT | 1614-72-07 | MT probe (DM) | CGCGCCGAGGAAAAAAGCTGATAACAAAGTACT | 337300 | |
| SEQ ID NO:185 | 3905insT | 1645-07-01 | WT ST | CAGGGAAGAGTACTTTGTTATCAGCTTTTTTGAGACTACTGAACACTGAAGGAGAAATCCAGATCGATGG | 698200 | |
| SEQ ID NO:186 | 3905insT | 1645-07-02 | MT ST | CAGGGAAGAGTACTTTGTTATCAGCTTTTTTGAGACTACTGAACACTGAAGGAGAAATCCAGATCGATGG | 706300 | |
| SEQ ID NO:187 | 3905insT | 23-428 | DM FAM FRET–MT | FAM-tct-X-agc-cgg-ttt-tcc-ggc-tga-gac-ctc-ggc-gcg-hex | | |
| SEQ ID NO:188 | 3905insT | 23-752 | ER38 Red w/FAM stem–WT | Z35-tct-X-agc-cgg-ttt-tcc-ggc-tga-gac-gtc-cgt-ggc-ct-hex | | |
| SEQ ID NO:189 | Y1092X C>G | 1645-14-10 | Invader | CCACAAAGCTCTGAATTTACATACTGCCAACTGGTTCTTGTAT | 404700 | |
| SEQ ID NO:190 | Y1092X C>G | 1733-75-01 | WT probe DM | CGCGCCGAGGCCTGTCAACACTGC | 214800 | |
| SEQ ID NO:191 | Y1092X C>G | 1645-14-17 | MT probe ER24 | ACGGACGCGGAGGCTGTCAACACTGCG | 258400 | |
| SEQ ID NO:192 | Y1092X C>G | 1645-14-12 | WT ST | CCAGCGCAGTGTTGACAGGTACAAGAACCAGTTGGCAGTATGTAAATTCAGAGCTTTGTGGAAT | 636100 | |
| SEQ ID NO:193 | Y1092X C>G | 1645-14-16 | MT ST | CCAGCGCAGTGTTGACAGCTACAAGAACCAGTTGGCAGTATGTAAATTCAGAGCTTTGTGGAAT | 631400 | |
| SEQ ID NO:194 | Y1092X C>G | 23-428 | DM FAM FRET-WT | FAM-tct-X-agc-cgg-ttt-tcc-ggc-tga-gac-ctc-ggc-gcg-hex | | |
| SEQ ID NO:195 | Y1092X C>G | 23-394 | ER24 Red FRET-MT | Z35-tct-X-tcg-gcc-ttt-tgg-ccg-aga-gac-tcc-gcg-tcc-gt-hex | | |
| SEQ ID NO:196 | 3849+4A>G | 1645-13-10 | Invader AS | ACATTTCCTTCTCAATAAGTCCTGGCCAGAGGGTGT | 340400 | |
| SEQ ID NO:197 | 3849+4A>G | 1645-13-14 | Wt ER38 | AGGCCACGGACGAGATTTGAACACTGCTTG | 291200 | |
| SEQ ID NO:198 | 3849+4A>G | 1645-13-15 | Mt DM | CGCGCCGAGGGGATTTGAACACTGCTTG | 261300 | |
| SEQ ID NO:199 | 3849+4A>G | 1645-13-12 | Wt ST | AAAGCAAGCAGTGTTCAAATCTCAGCCTCTGGCCAGGACTTATTGAGAAGGAAATGTTCT | 587100 | |
| SEQ ID NO:200 | 3849+4A>G | 1645-13-16 | Mt ST | AAAGCAAGCAGTGTTCAAATCCCAGCCTCTGGCCAGGACTTATTGAGAAGGAAATGTTCT | 586200 | |
| SEQ ID NO:201 | 3849+4A>G | 23-205 | DM Red FRET | Z35-tct-X-tcg-gcc-ttt-tgg-ccg-aga-gac-ctc-ggc-gcg-hex | | |
| SEQ ID NO:202 | 3849+4A>G | 23-204 | ER38 FAM FRET | FAM-tct-X-agc-cgg-ttt-tcc-ggc-tga-gac-gtc-cgt-ggc-ct-hex | | |
| SEQ ID NO:203 | 3876delA | 1614-61-01 | Invader S | CCTTCAGTGTTCAGTAGTCTCAAAAAAGCTGATAACAAAGTACTCTTCCT | 483100 | |
| SEQ ID NO:204 | 3876delA | 1614-61-02 | Wt DM | CGCGCCGAGGCTGATCCAGTTCTTCCCV | 237300 | |
| SEQ ID NO:205 | 3876delA | 1614-61-09 | Mt ER38 | AGGCCACGGACGCGATCCAGTTCTTCCCV | 256600 | |
| SEQ ID NO:206 | 3876delA | 1614-61-03 | WT ST | CTCTTGGGAAGAACTGGATCAGGGAAGAGTACTTTGTTATCAGCTTTTTTGAGACTACTGAACACTGAAGGAG | 719600 | |
| SEQ ID NO:207 | 3876delA | 1614-61-07 | MT ST | CTCTTGGGAAGAACTGGATCGGGAAGAGTACTTTGTTATCAGCTTTTTTGAGACTACTGAACACTGAAGGAG | 706800 | |
| SEQ ID NO:208 | 3876delA | 23-428 | DM FAM FRET WT | FAM-tct-X-agc-cgg-ttt-tcc-ggc-tga-gac-ctc-ggc-gcg-hex | | |
| SEQ ID NO:209 | 3876delA | 23-752 | ER38 Red w/FAM stem MT | Z35-tct-X-agc-cgg-ttt-tcc-ggc-tga-gac-gtc-cgt-ggc-ct-hex | | |
| SEQ ID NO:210 | Q493X | 1645-12-01 | Invader | TGGTGCCAGGCATAATCCAGGAAAACTT | 275700 | |
| SEQ ID NO:211 | Q493X | 1767-83-03 | WT ER24 | ACGGACGCGGAGGAGAACAGAATGAAATTCT | 322300 | |
| SEQ ID NO:212 | Q493X | 1645-12-09 | MT ER38 | AGGCCACGGACGAAGAACAGAATGAAATTCTTCC | 343700 | |
| SEQ ID NO:213 | Q493X | 1645-12-03 | WT ST | CAGTGGAAGAATTTCATTCTGTTCTCAGTTTTCCTGGATTATGCCTGGCACCATTT | 518600 | |
| SEQ ID NO:214 | Q493X | 1645-12-07 | MT ST | CAGTGGAAGAATTTCATTCTGTTCTTAGTTTTCCTGGATTATGCCTGGCACCATTT | 520100 | |
| SEQ ID NO:215 | Q493X | 23-210 | ER24 FAM FRET – WT | FAM-tct-X-agc-cgg-ttt-tcc-ggc-tga-gac-tcc-gcg-tcc-gt-hex | | |
| SEQ ID NO:216 | Q493X | 23-752 | ER38 Red FRETw/FAM stem – MT | Z35-tct-X-agc-cgg-ttt-tcc-ggc-tga-gac-gtc-cgt-ggc-ct-hex | | |

FIGURE 3

| SEQ ID NO | Mutation | Oligo Name | Oligo type | Sequence (5'-3') | ε260 M-1 cm-1 | Length |
|---|---|---|---|---|---|---|
| SEQ ID NO:217 | G551D | 1614-52-10 | Invader AS | TGGAGAAGGTGGAATCACACTGAGTGGAGT | 308600 | |
| SEQ ID NO:218 | G551D | 1711-01-01 | Wt DM | CGCGCCGAGGGTCAACGAGCAAGAATTTV | 274400 | |
| SEQ ID NO:219 | G551D | 1711-01-06 | Mt ER38 | AGGCCACGGACGATCAACGAGCAAGAATTTCV | 311200 | |
| SEQ ID NO:220 | G551D | 1614-52-12 | Wt ST | CTAAAGAAATTCTTGCTCGTTGACCTCCACTCAGTGTGATTCCACCTTCTCCAAGT | 514900 | |
| SEQ ID NO:221 | G551D | 1614-52-16 | Mt ST | CTAAAGAAATTCTTGCTCGTTGATCTCCACTCAGTGTGATTCCACCTTCTCCAAGT | 516800 | |
| SEQ ID NO:222 | G551D | 23-746 | DM Red FRET w/FAM stem WT | Z35-tct-X-agc-cgg-ttt-tcc-ggc-tga-gac-ctc-ggc-gcg-hex | | |
| SEQ ID NO:223 | G551D | 23-204 | ER38 FAM FRET | FAM-tct-X-agc-cgg-ttt-tcc-ggc-tga-gac-gtc-cgt-ggc-ct-hex | | |
| SEQ ID NO:224 | R553X | 1645-72-01 | Invader +2S | GCTAGACCAATAATTAGTTATTCACCTTGCTAAAGAAATTCTTGCTG | 456700 | |
| SEQ ID NO:225 | R553X | 1667-08-02 | Wt ER38 | AGGCCACGGACGCGTTGACCTCCACTCA | 260300 | |
| SEQ ID NO:226 | R553X | 1453-07-01 | Mt DM | CGCGCCGAGGCATTGACCTCCACTCAGT | 253700 | |
| SEQ ID NO:227 | R553X | 1614-53-03 | Wt ST | TCACACTGAGTGGAGGTCAACGAGCAAGAATTTCTTTAGCAAGGTGAATAACTAATTATTGGTCTAGCAAGCT | 727300 | |
| SEQ ID NO:228 | R553X | 1614-53-07 | Mt ST | TCACACTGAGTGGAGGTCAATGAGCAAGAATTTCTTTAGCAAGGTGAATAACTAATTATTGGTCTAGCAAGCT | 728600 | |
| SEQ ID NO:229 | R553X | 23-428 | DM FAM FRET MT | FAM-tct-X-agc-cgg-ttt-tcc-ggc-tga-gac-ctc-ggc-gcg-hex | | |
| SEQ ID NO:230 | R553X | 23-752 | ER38 Red FRET w/FAM stem WT | Z35-tct-X-agc-cgg-ttt-tcc-ggc-tga-gac-gtc-cgt-ggc-ct-hex | | |
| SEQ ID NO:231 | R1162X | 1618-52-01 | Invader | GTTTACCTTCTGTTGGCATGTOAATGAACTTAAAGACTCT | 424200 | |
| SEQ ID NO:232 | R1162X | 1618-52-03 | WT probe (ER24) | ACGGACGCGGAGGGCTCACAGATCGCV | 284000 | |
| SEQ ID NO:233 | R1162X | 1618-52-08 | Mt probe (ER38) | AGGCCACGGACGAGCTCACAGATCGCV | 283800 | |
| SEQ ID NO:234 | R1162X | 1618-52-05 | WT ST | AGATGCGATCTGTGAGCCGAGTCTTTAAGTTCATTGACATGCCAACAGAAGGTAAACT | 574600 | |
| SEQ ID NO:235 | R1162X | 1618-52-09 | MT ST | AGATGCGATCTGTGAGCTGAGTCTTTAAGTTCATTGACATGCCAACAGAAGGTAAACT | 574900 | |
| SEQ ID NO:236 | R1162X | 23-204 | ER38 FAM FRET— MT | FAM-tct-X-agc-cgg-ttt-tcc-ggc-tga-gac-gtc-cgt-ggc-ct-hex | | |
| SEQ ID NO:237 | R1162X | 23-755 | ER24 Red FRETw/FAM — WT | Z35-tct-X-agc-cgg-ttt-tcc-ggc-tga-gac-tcc-gcg-tcc-gt-hex | | |
| SEQ ID NO:238 | S549R (A>C) | 1614-63-01 | Invader | CACCTTGCTAAAGAAATTCTTGCTCGTTGACCTCCACC | 344400 | |
| SEQ ID NO:239 | S549R (A>C) | 1614-63-02 | WT probe (DM) | CGCGCCGAGGTCAGTGTGATTCCACCTV | 247300 | |
| SEQ ID NO:240 | S549R (A>C) | 1733-78-02 | MT probe (ER38) | AGGCCACGGACGGCAGTGTGATTCCAC | 259500 | |
| SEQ ID NO:241 | S549R (A>C) | 1614-63-03 | WT ST | GAGAAGGTGGAATCACACTGAGTGGAGGTCAACGAGCAAGAATTTCTTTAGCAAGGTGAAT | 624200 | |
| SEQ ID NO:242 | S549R (A>C) | 1614-63-07 | MT ST | GAGAAGGTGGAATCACACTGCGTGGAGGTCAACGAGCAAGAATTTCTTTAGCAAGGTGAAT | 617600 | |
| SEQ ID NO:243 | S549R (A>C) | 23-428 | DM FAM FRET— WT | FAM-tct-X-agc-cgg-ttt-tcc-ggc-tga-gac-ctc-ggc-gcg-hex | | |
| SEQ ID NO:244 | S549R (A>C) | 23-211 | ER38 Red FRET— MT | Z35-tct-X-tcg-gcc-ttt-tgg-ccg-aga-gac-gtc-cgt-ggc-ct-hex | | |
| SEQ ID NO:245 | F508C | 1618-70-10 | Invader AS | CAGTTTCCTGGATTATGCCTGGCACCATTAAAGAAAATATCATCTC | 450800 | |
| SEQ ID NO:246 | F508C | 1791-37-01 | Wt DM | CGCGCCGAGGTTGGTGTTTCCTATGATGAAT | 291900 | |
| SEQ ID NO:247 | F508C | 1826-07-05 | Mt ER38 | AGGCCACGGACGGTGGTGTTTCCTATGATG | 287600 | |
| SEQ ID NO:248 | F508C | 1618-70-14 | WT ST | TATATTCATCATAGGAAACACCAAAGATGATATTTTCTTTAATGGTGCCAGGCATAATCCAGGAAAACTGAGT | 736000 | |
| SEQ ID NO:249 | F508C | 1618-70-18 | MT ST | TATATTCATCATAGGAAACACCACAGATGATATTTTCTTTAATGGTGCCAGGCATAATCCAGGAAAACTGAGT | 731600 | |
| SEQ ID NO:250 | F508C | 23-428 | DM FAM FRET-WT | FAM-tct-X-agc-cgg-ttt-tcc-ggc-tga-gac-ctc-ggc-gcg-hex | | |
| SEQ ID NO:251 | F508C | 23-211 | ER38 Red FRET Mt | Z35-tct-X-tcg-gcc-ttt-tgg-ccg-aga-gac-gtc-cgt-ggc-ct-hex | | |
| SEQ ID NO:252 | Y1092X C>A | 1645-14-10 | Invader | CCACAAAGCTCTGAATTTACATACTGCCAACTGGTTCTTGTAT | 404700 | |
| SEQ ID NO:253 | Y1092X C>A | 1645-14-11 | WT probe -DM | CGCGCCGAGGCCTGTCAACACTGCG | 225400 | |
| SEQ ID NO:254 | Y1092X C>A | 1645-15-08 | MT probe -ER38 | AGGCCACGGACGACTGTCAAGACTGCG | 258400 | |
| SEQ ID NO:255 | Y1092X C>A | 1645-14-12 | WT ST | CCAGCGCAGTGTTGACAGGTACAAGAACCAGTTGGCAGTATGTAAATTCAGAGCTTTGTGGAAT | 636100 | |
| SEQ ID NO:256 | Y1092X C>A | 1645-15-06 | MT ST | CCAGCGCAGTGTTGACAGTTACAAGAACCAGTTGGCAGTATGTAAATTCAGAGCTTTGTGGAAT | 634100 | |
| SEQ ID NO:257 | Y1092X C>A | 23-428 | DM FAM FRET–WT | FAM-tct-X-agc-cgg-ttt-tcc-ggc-tga-gac-ctc-ggc-gcg-hex | | |
| SEQ ID NO:258 | Y1092X C>A | 23-752 | ER38 Red FRETw/FAM–MT | Z35-tct-X-agc-cgg-ttt-tcc-ggc-tga-gac-gtc-cgt-ggc-ct-hex | | |
| SEQ ID NO:259 | S549R (T>G) | 1614-64-01 | Invader | AGTTATTCACCTTGCTAAAGAAATTCTTGCTCGTTGACCTCCT | 397300 | |
| SEQ ID NO:260 | S549R(T>G) | 1776-64-06 | WT ER38 | AGGCCACGGACGACTCAGTGTGATTCCACC | 285300 | |
| SEQ ID NO:261 | S549R (T>G) | 1614-64-06 | MT probe (DM) | CGCGCCGAGGCCTCAGTGTGATTCCACV | 245100 | |
| SEQ ID NO:262 | S549R (T>G) | 1614-64-03 | WT ST | GAAGGTGGAATCACACTGAGTGGAGGTCAACGAGCAAGAATTTCTTTAGCAAGGTGAATAACTAAT | 675300 | |
| SEQ ID NO:263 | S549R (T>G) | 1614-64-07 | MT ST | GAAGGTGGAATCACACTGAGGGGAGGTCAACGAGCAAGAATTTCTTTAGCAAGGTGAATAACTAAT | 676700 | |
| SEQ ID NO:264 | S549R (T>G) | 23-211 | ER38 Red FRET— WT | Z35-tct-X-tcg-gcc-ttt-tgg-ccg-aga-gac-gtc-cgt-ggc-ct-hex | | |
| SEQ ID NO:265 | S549R (T>G) | 23-428 | DM FAM FRET— MT | FAM-tct-X-agc-cgg-ttt-tcc-ggc-tga-gac-ctc-ggc-gcg-hex | | |
| SEQ ID NO:266 | del I507 | 1614-58-01 | Invader S | CATGCTTTGATGACGCTTCTGTATCTATATTCATCATAGGAAACACCAAAT | 493900 | |
| SEQ ID NO:267 | del I507 | 1645-56-01 | Wt ER24 | ACGGACGCGGAGGATGATATTTCTTTAATGGTG | 335900 | |
| SEQ ID NO:268 | del I507 | 1645-56-04 | Mt ER38 | AGGCCACGGACGGATATTTTCTTTAATGGTGCCI | 314500 | |
| SEQ ID NO:269 | del I507 | 1614-58-03 | Wt ST | ATGCCTGGCACCATTAAAGAAAATATCATCTTTGGTGTTTCCTATGATGAATATAGATACAGAAGCGTCATCAAAGCATGCC | 814200 | |
| SEQ ID NO:270 | del I507 | 1614-58-07 | Mt ST | ATGCCTGGCACCATTAAAGAAAATATCTTTGGTGTTTCCTATGATGAATATAGATACAGAAGCGTCATCAAAGCATGCC | 785500 | |

EP 1 567 540 B1

68

EP 1 567 540 B1

FIGURE 3

| SEQ ID NO | Mutation | Oligo Name | Oligo type | Sequence (5'-3') | ε260 M-1 cm-1 | Length |
|---|---|---|---|---|---|---|
| EQ ID NO:271 | dell507 | 23-204 | ER38 FAM FRET MT | FAM-tct-X-agc-cgg-ttt-tcc-ggc-tga-gac-gtc-cgt-ggc-ct-hex | | |
| EQ ID NO:272 | dell507 | 23-755 | ER24 Redw/FAM FRET WT | Z35-tct-X-agc-cgg-ttt-tcc-ggc-tga-gac-tcc-gcg-tcc-gt-hex | | |
| EQ ID NO:273 | 5T Variant (5T) | 16924810 | Probe (DM) | CGCGCCGAGGGTTTTTAACAGGGATTTGGG | 286900 | |
| EQ ID NO:274 | 5T Variant (7T) | 16925804 | Probe (DM) | CGCGCCGAGGGTTTTTTTAACAGGGATTTGG | 293000 | |
| EQ ID NO:275 | 5T Variant (9T) | 16929202 | Probe (DM) | CGCGCCGAGGG9TTTTTTTTACCAGGGATTTGGGGA | 338300 | |
| EQ ID NO:276 | 5T Variant | 16027813 | Invader | CTCATCTTTTATTTTTGATGTGTGTGTGTGTGTGTA | 353300 | |
| EQ ID NO:277 | 5T Variant | 16027814 | Invader | CTCATCTTTTATTTTTGATGTGTGTGTGTGTGTGTGTA | 372100 | |
| EQ ID NO:278 | 5T Variant | 16027815 | Invader | CTCATCTTTTATTTTTGATGTGTGTGTGTGTGTGTGTGTA | 390900 | |
| EQ ID NO:279 | 5T Variant | 16027816 | Invader | CTCATCTTTTATTTTTGATGTGTGTGTGTGTGTGTGTGTGTA | 409700 | |
| EQ ID NO:280 | 5T Variant | 16608401 | Invader | CTCATCTTTTATTTTTGATGTGTGTGTGTGTGTGTA | 334500 | |
| EQ ID NO:281 | 5T Variant | 23-428 | DM FAM FRET | FAM-tct-X-agc-cgg-ttt-tcc-ggc-tga-gac-ctc-ggc-gcg-hex | | |
| EQ ID NO:282 | Y122X | 1809-46-01 | Invader | GTGTCCTCACAATAAAGAGAAGGCATAAGCCTATGCCTAA | 403500 | |
| EQ ID NO:283 | Y122X | 1827-34-03 | WT ER24 | ACGGACGCGGAGGATAAATCGCGATAGAGC | 309000 | |
| EQ ID NO:284 | Y122X | 1827-34-12 | MT ER38 | AGGCCACGGACGGTTAAATCGCGATAGAG | 295100 | |
| EQ ID NO:285 | Y122X | 1645-16-03 | WT ST | GGAACGCTCTATCGCGATTTATCTAGGCATAGGCTTATGCCTTCTCTTTATTGTGAGGACACTGT | 609400 | |
| EQ ID NO:286 | Y122X | 1645-16-07 | MT ST | GGAACGCTCTATCGCGATTTAACTAGGCATAGGCTTATGCCTTCTCTTTATTGTGAGGACACTGT | 612300 | |
| EQ ID NO:287 | Y122X | 23-394 | ER24 Red FRET—WT | Z35-tct-X-tcg-gcc-ttt-tgg-ccg-aga-gac-tcc-gcg-tcc-gt-hex | | |
| EQ ID NO:288 | Y122X | 23-204 | ER38 FAM FRET — MT | FAM-tct-X-agc-cgg-ttt-tcc-ggc-tga-gac-gtc-cgt-ggc-ct-hex | | |
| EQ ID NO:289 | 1898+1 G>A | 1353-02-02 | Invader | GACTCTCCTTTTGGATACCTAGATGTTTTAACAGAAAAAGAAATATTTGAAAGT | | |
| EQ ID NO:290 | 1898+1 G>A | 1801-65-03 | WT ER24 | ACGGACGCGGAGGTATGTTCTTTGAATACCTTACT | | |
| EQ ID NO:291 | 1898+1 G>A | 1801-65-08 | MT DM | CGCGCCGAGGATATGTTCTTTGAATACCTTACTT | | |
| SEQ ID NO:292 | 1898+1 G>A | 1614-59-12 | WT ST | AGCATTATAAGTAAGGTATTCAAAGAACATACCTTTCAAATATTTCTTTTTCTGTTAAAACATCTAGGTATCCAAAAGGAGT | | |
| SEQ ID NO:293 | 1898+1 G>A | 1614-59-18 | MT ST | AGCATTATAAGTAAGGTATTCAAAGAACATATCTTTCAAATATTTCTTTTTCTGTTAAAACATCTAGGTATCCAAAAGGAGT | | |
| SEQ ID NO:294 | 1898+1 G>A | 23-210 | ER24 FAM FRET- WT | FAM-tct-X-agc-cgg-ttt-tcc-ggc-tga-gac-tcc-gcg-tcc-gt-hex | | |
| SEQ ID NO:295 | 1898+1 G>A | 23-746 | DM Red FRET w/FAM- MT | Z35-tct-X-agc-cgg-ttt-tcc-ggc-tga-gac-ctc-ggc-gcg-hex | | |

X = Quencher = Z28
9= 2' O-methyl modified base

FIGURE 4

| SEQ ID NO | Allele | Part No | | | |
|---|---|---|---|---|---|
| SEQ ID NO:417 | R117H | 1614-48-17 | Mt Probe (ER24) | ACGGACGCGGAGACTCTATCGCGATTTATCTAGV | |
| SEQ ID NO:418 | R117H | 1614-48-10 | invader | AGAATCATAGCTTCCTATGACCCGGATAACAAGGAGGAACT | |
| SEQ ID NO:419 | W1282X | 1618-42-04 | Wt probe (ER38) | AGGCCACGGACGCCACTGTTGCAAAGTTATTV | |
| SEQ ID NO:420 | 711+1G>T | 1791-06-04 | Wt ER24 | ACGGACGCGGAGACTTCATCAAATTTGTTCAG | |
| SEQ ID NO:421 | 711+1G>T | 1510-82-03 | Mt ER24 | ACGGACGCGGAGAATTCATCAAATTTGTTCAGG | |
| SEQ ID NO:422 | 711+1G>T | 1614-56-05 | Wt probe (ER38) | AGGCCACGGACGCTTCATCAAATTTGTTCAGGTV | |
| SEQ ID NO:423 | 711+1G>T | 1614-56-06 | Mt probe (DM) | CGCGCCGAGGATTCATCAAATTTGTTCAGGTV | |
| SEQ ID NO:424 | 711+1G>T | 1614-56-08 | Mt Probe (ER24) | ACGGACGCGGAGATTCATCAAATTTGTTCAGGTV | |
| SEQ ID NO:425 | 711+1G>T | 1614-56-01 | invader | CCTTTCCAGTTGTATAATTTATAACAATAGTGCCTAAAAGATT AAATCAATAGGTACATAT | |
| SEQ ID NO:426 | 394delTT | | invader | CCCTTCGGCGATGTTTTTTCTGGAGATTTATGTTCTATGGAA Tt | |
| SEQ ID NO:427 | 394delTT | | WT probe | AGGCCACGGACGCTTTTTATATTTAGGGGTAAGGATCTHex | |
| SEQ ID NO:428 | 394delTT | | MT probe | AAGCACGCAGCACCTTTATATTTAGGGGTAAGGATCTCHex | |
| SEQ ID NO:429 | delI507 | 1353-01-01 | Invader S | GCTTTGATGACGCTTCTGTATCTATATTCATCATAGGAAACAC CAAT | 454500 |
| SEQ ID NO:430 | delI507 | 1556-23-09 | Wt DM | CGCGCCGAGGAGATGATATTTTCTTTAATGGTG | 319000 |
| SEQ ID NO:431 | delI507 | 1826-61-01 | Wt DM | CGCGCCGAGGAGATGATATTTTCTTTAATGGT | 308700 |
| SEQ ID NO:432 | delI507 | 1556-23-10 | WT ER24 | ACGGACGCGGAGAGATGATATTTTCTTTAATGGTG | 349100 |
| SEQ ID NO:433 | delI507 | 1826-61-02 | WT ER24 | ACGGACGCGGAGAGATGATATTTTCTTTAATGGT | 338800 |
| SEQ ID NO:434 | delI507 | 1556-23-11 | WT ER38 | AGGCCACGGACGAGATGATATTTTCTTTAATGGTG | 345800 |
| SEQ ID NO:435 | delI507 | 1826-61-03 | WT ER38 | AGGCCACGGACGAGATGATATTTTCTTTAATGGT | 335500 |
| SEQ ID NO:436 | delI507 | 1353-01-03 | Mt DM | CGCGCCGAGGAGATATTTTCTTTAATGGTGCC | 300900 |
| SEQ ID NO:437 | delI507 | 1826-61-04 | Mt DM | CGCGCCGAGGAGATATTTTCTTTAATGGTGC | 293700 |
| SEQ ID NO:438 | delI507 | 1353-55-01 | Mt ER24 | ACGGACGCGGAGAGATATTTTCTTTAATGGTGCCA | 344800 |
| SEQ ID NO:439 | delI507 | 1826-61-05 | Mt ER24 | ACGGACGCGGAGAGATATTTTCTTTAATGGTGCC | 331000 |
| SEQ ID NO:440 | delI507 | 1826-61-06 | Mt ER24 | ACGGACGCGGAGAGATATTTTCTTTAATGGTGC | 323800 |
| SEQ ID NO:441 | delI507 | 1353-55-02 | Mt ER38 | AGGCCACGGACGAGATATTTTCTTTAATGGTGCCA | 341500 |
| SEQ ID NO:442 | delI507 | 1826-61-07 | Mt ER38 | AGGCCACGGACGAGATATTTTCTTTAATGGTGCC | 327700 |
| SEQ ID NO:443 | delI507 | 1826-61-08 | Mt ER38 | AGGCCACGGACGAGATATTTTCTTTAATGGTGC | 320500 |
| SEQ ID NO:444 | 5T Variant (5T) | 18312001 | Probe (ER24) | ACGGACGCGGAGGTTTTTAACAGGGATTGGG | |
| SEQ ID NO:445 | 5T Variant (5T) | 18312002 | Probe (ER38) | AGGCCACGGACGGTTTTTAACAGGGATTGGG | |
| SEQ ID NO:446 | 5T Variant (5T) | 16027817 | Probe (DM) | CGCGCCGAGGGTTTTTAACAGGGATTGGGG | |
| SEQ ID NO:447 | 5T Variant (5T) | 16027818 | Probe (ER24) | ACGGACGCGGAGGTTTTTAACAGGGATTGGGG | |
| SEQ ID NO:448 | 5T Variant (5T) | 16027819 | Probe (ER38) | AGGCCACGGACGGTTTTTAACAGGGATTGGGG | |

EP 1 567 540 B1

FIGURE 4

| SEQ ID NO:449 | 5T Variant (7T) | 18312003 | Probe (ER24) | ACGGACGCGGAGGTTTTTTTTAACAGGGATTTGG |
|---|---|---|---|---|
| SEQ ID NO:450 | 5T Variant (7T) | 18312004 | Probe (ER38) | AGGCCACGGACGGTTTTTTTTAACAGGGATTTGG |
| SEQ ID NO:451 | 5T Variant (7T) | 16924813 | Probe (DM) | CGCGCCGAGGGTTTTTTTTAACAGGGATTTGGG |
| SEQ ID NO:452 | 5T Variant (7T) | 16924814 | Probe (ER24) | ACGGACGCGGAGGTTTTTTTTAACAGGGATTTGGG |
| SEQ ID NO:453 | 5T Variant (7T) | 16924815 | Probe (ER38) | AGGCCACGGACGGTTTTTTTTAACAGGGATTTGGG |
| SEQ ID NO:454 | 5T Variant (7T) | 16027820 | Probe (DM) | CGCGCCGAGGGTTTTTTTTAACAGGGATTTGGGG |
| SEQ ID NO:455 | 5T Variant (7T) | 16027821 | Probe (ER24) | ACGGACGCGGAGGTTTTTTTTAACAGGGATTTGGGG |
| SEQ ID NO:456 | 5T Variant (7T) | 16027822 | Probe (ER38) | AGGCCACGGACGGTTTTTTTTAACAGGGATTTGGGG |
| SEQ ID NO:457 | 5T Variant (9T) | 18312005 | Probe (ER24) | ACGGACGCGGAGG9TTTTTTTTTACCAGGGATTTGGGGA* |
| SEQ ID NO:458 | 5T Variant (9T) | 18312006 | Probe (ER38) | AGGCCACGGACGG9TTTTTTTTTACCAGGGATTTGGGGA* |
| SEQ ID NO:459 | 5T Variant (9T) | 16929204 | Probe (DM) | CGCGCCGAGGG9TTTTTTTTTAACAGGGAATTGGGGA* |
| SEQ ID NO:460 | 5T Variant (9T) | 18312007 | Probe (ER24) | ACGGACGCGGAGG9TTTTTTTTTAACAGGGAATTGGGGA* |
| SEQ ID NO:461 | 5T Variant (9T) | 18312008 | Probe (ER38) | AGGCCACGGACGG9TTTTTTTTTAACAGGGAATTGGGGA* |
| SEQ ID NO:462 | 5T Variant (9T) | 17337201 | Probe (DM) | CGCGCCGAGGGT9TTTTTTTTACCAGGGATTTGGGGA* |
| SEQ ID NO:463 | 5T Variant (9T) | 18312009 | Probe (ER24) | ACGGACGCGGAGGT9TTTTTTTTACCAGGGATTTGGGGA* |
| SEQ ID NO:464 | 5T Variant (9T) | 18312010 | Probe (ER38) | AGGCCACGGACGGT9TTTTTTTTACCAGGGATTTGGGGA* |
| SEQ ID NO:465 | 5T Variant (9T) | 17337202 | Probe (DM) | CGCGCCGAGGGT9TTTTTTTTAACAGGGAATTGGGGA* |
| SEQ ID NO:466 | 5T Variant (9T) | 18312011 | Probe (ER24) | ACGGACGCGGAGGT9TTTTTTTTAACAGGGAATTGGGGA* |
| SEQ ID NO:467 | 5T Variant (9T) | 18312012 | Probe (ER38) | AGGCCACGGACGGT9TTTTTTTTAACAGGGAATTGGGGA* |
| SEQ ID NO:468 | 5T Variant (9T) | 16027823 | Probe (DM) | CGCGCCGAGGGTTTTTTTTTTAACAGGGATTTGGGG |
| SEQ ID NO:469 | 5T Variant (9T) | 16027824 | Probe (ER24) | ACGGACGCGGAGGTTTTTTTTTTAACAGGGATTTGGGG |
| SEQ ID NO:470 | 5T Variant (9T) | 16027825 | Probe (ER38) | AGGCCACGGACGGTTTTTTTTTTAACAGGGATTTGGGG |
| SEQ ID NO:471 | 5T Variant (9T) | 16925101 | Probe (DM) | CGCGCCGAGGGTTTATTTTTAACAGGGATTTGGGG* |
| SEQ ID NO:472 | 5T Variant (9T) | 16925102 | Probe (DM) | CGCGCCGAGGGTTTTATTTTAACAGGGATTTGGGG* |
| SEQ ID NO:473 | 5T Variant (9T) | 16925103 | Probe (DM) | CGCGCCGAGGGTTTTTATTTAACAGGGATTTGGGG* |
| SEQ ID NO:474 | 5T Variant (9T) | 16925104 | Probe (DM) | CGCGCCGAGGGTTTTTTATTAACAGGGATTTGGGG* |
| SEQ ID NO:475 | 5T Variant (9T) | 16925105 | Probe (DM) | CGCGCCGAGGGTTTTTTTATAACAGGGATTTGGGG* |
| SEQ ID NO:476 | 5T Variant (9T) | 16925106 | Probe (DM) | CGCGCCGAGGGTTTTTTTTAAACAGGGATTTGGGG* |
| SEQ ID NO:477 | 5T Variant (9T) | 16925107 | Probe (DM) | CGCGCCGAGGGTTTTTTTTTCACAGGGATTTGGGG* |
| SEQ ID NO:478 | 5T Variant (9T) | 16925108 | Probe (DM) | CGCGCCGAGGGTTTTTTTTTACCAGGGATTTGGGG* |
| SEQ ID NO:479 | 5T Variant (9T) | 16925109 | Probe (DM) | CGCGCCGAGGGTTTTTTTTTAAGAGGGATTTGGGG* |
| SEQ ID NO:480 | 5T Variant (9T) | 16925110 | Probe (DM) | CGCGCCGAGGGTTTTTTTTTAACCGGGATTTGGGG* |
| SEQ ID NO:481 | 5T Variant (9T) | 16925111 | Probe (DM) | CGCGCCGAGGGTTTTTTTTTAACAAGGATTTGGGG* |
| SEQ ID NO:482 | 5T Variant (9T) | 16925112 | Probe (DM) | CGCGCCGAGGGTTTTTTTTTAACAGAGATTTGGGG* |
| SEQ ID NO:483 | 5T Variant (9T) | 16925113 | Probe (DM) | CGCGCCGAGGGTTTTTTTTTAACAGGAATTTGGGG* |
| SEQ ID NO:484 | 5T Variant (9T) | 16925114 | Probe (DM) | CGCGCCGAGGGTTTTTTTTTAACAGGGCTTTGGGG* |
| SEQ ID NO:485 | 5T Variant (9T) | 16925115 | Probe (DM) | CGCGCCGAGGGTTTTTTTTTTAACAGGGAATTGGGG* |

EP 1 567 540 B1

FIGURE 4

EP 1 567 540 B1

| SEQ ID NO:486 | 5T Variant (9T) | 16925116 | Probe (DM) | CGCGCCGAGGGTTTTTTTTTTAACAGGGATATGGGG* |
| SEQ ID NO:487 | 5T Variant (9T) | 16925117 | Probe (DM) | CGCGCCGAGGGTTTTTTTTTTAACAGGGATTAGGGG*. |
| SEQ ID NO:488 | 5T Variant (9T) | 16925118 | Probe (DM) | CGCGCCGAGGGTTTTTTTTTTAACAGGGATTTAGGG* |
| SEQ ID NO:489 | 5T Variant (9T) | 16925119 | Probe (DM) | CGCGCCGAGGGTTTTTTTTTTAACAGGGATTTGAGG* |
| SEQ ID NO:490 | 5T Variant (9T) | 16925120 | Probe (DM) | CGCGCCGAGGGTTTTTTTTTTAACAGGGATTTGGAG* |
| SEQ ID NO:491 | 5T Variant (9T) | 16927101 | Probe (DM) | CGCGCCGAGGG9TTTTTTTTAACAGGGATTTGGGG* |
| SEQ ID NO:492 | 5T Variant (9T) | 16927102 | Probe (DM) | CGCGCCGAGGGT9TTTTTTTAACAGGGATTTGGGG* |
| SEQ ID NO:493 | 5T Variant (9T) | 16929201 | Probe (DM) | CGCGCCGAGGG9TTTTTTTTACCAGGGATTTGGGG* |
| SEQ ID NO:494 | 5T Variant (9T) | 16929203 | Probe (DM) | CGCGCCGAGGG9TTTTTTTTAACAGGGAATTGGGG* |

9 = 2' O-methyl modified base

FIGURE 5

| SEQ ID NO | | forward primer |
|---|---|---|
| SEQ ID NO: 394 | cftr exon 3 | TGGTCCCACTTTTTATTCTTTTGCAGA |
| SEQ ID NO: 395 | cftr exon 4 | AAGTCACCAAAGCAGTACAGCC |
| SEQ ID NO: 396 | cftr exon 5 | GCTGTCAAGCCGTGTTCTAGATAAA |
| SEQ ID NO: 397 | cftr exon 7 | CGGAAGGCAGCCTATGTGAGA |
| SEQ ID NO: 398 | cftr exon 9 | CATGGGCCATGTGCTTTTCAAAC |
| SEQ ID NO: 399 | cftr exon 9-1 | CATGGGCCATGTGCTTTTCAAAC |
| SEQ ID NO: 400 | cftr exon 9-2 | CTTCTTGGTACTCCTGTCCTGAAAGA |
| SEQ ID NO: 401 | cftr exon 10 | ATTATGGGAGAACTGGAGCCTTCA |
| SEQ ID NO: 402 | cftr exon 11 | GATTACATTAGAAGGAAGATGTGCCTTTCAA |
| SEQ ID NO: 403 | cftr exon 12 | TAAGGCAAATCATCTACACTAGATGACCA |
| SEQ ID NO: 404 | cftr exon 13 | TAACTGAGACCTTACACCGTTTCTCA |
| SEQ ID NO: 405 | cftr exon 14B | ATGGGAGGAATAGGTGAAGATGTTAGAA |
| SEQ ID NO: 406 | cftr exon 16 | TCTGAATGCGTCTACTGTGATCCA |
| SEQ ID NO: 407 | cftr exon 17A | CCTGCACAATGTGCACATGTACC |
| SEQ ID NO: 408 | cftr exon 17B | GGACTATGGACACTTCGTGCC |
| SEQ ID NO: 409 | cftr exon 18 | GGAGAAGGAAGAGTTGGTATTATCCTGAC |
| SEQ ID NO: 410 | cftr exon 19 | GCATCAAACTAATTGTGAAATTGTCTGCC |
| SEQ ID NO: 411 | cftr exon 19-1 | GCATCAAACTAATTGTGAAATTGTCTGCC |
| SEQ ID NO: 412 | cftr exon 19-2 | GAAGGTGGAAATGCCATATTAGAGAACA |
| SEQ ID NO: 413 | cftr exon 20 | GTACCTATATGTCACAGAAGTGATCCCA |
| SEQ ID NO: 414 | cftr exon 21 | GATTAGAAAAATGTTCACAAGGGACTCCA |
| SEQ ID NO: 415 | cftr 3849+10kb | CAGTTGACTTGTCATCTTGATTTCTGGA |
| SEQ ID NO: 416 | cftr exon 17A-2 | CCTCGACAATGTGCACATGTACC |

| | | reverse primer |
|---|---|---|
| SEQ ID NO: 495 | cftr exon 3 | ACCTATTCACCAGATTTCGTAGTCTTTTCA |
| SEQ ID NO: 496 | cftr exon 4 | TGTACCAGCTCACTACCTAATTTATGACA |
| SEQ ID NO: 497 | cftr exon 5 | GAGCTGAGCAAGACTTAACCACTAATTAC |
| SEQ ID NO: 498 | cftr exon 7 | GTGAACATTCCTAGTATTAGCTGGCAAC |
| SEQ ID NO: 499 | cftr exon 9 | CTCCAAAAATACCTTCCAGCACTACAAA |
| SEQ ID NO: 500 | cftr exon 9-1 | GAAATTACTGAAGAAGAGGCTGTCATCAC |
| SEQ ID NO: 501 | cftr exon 9-2 | CTCCAAAAATACCTTCCAGCACTACAAA |
| SEQ ID NO: 502 | cftr exon 10 | GACTAACCGATTGAATATGGAGCCAAA |
| SEQ ID NO: 503 | cftr exon 11 | CTTAAATGTGATTCTTAACCCACTAGCCA |
| SEQ ID NO: 504 | cftr exon 12 | GAGGTAAAATGCAATCTATGATGGGACA |
| SEQ ID NO: 505 | cftr exon 13 | TAAGGGAGTCTTTTGCACAATGGAAAA |
| SEQ ID NO: 506 | cftr exon 14B | ACCTCACCCAACTAATGGTCATCA |
| SEQ ID NO: 507 | cftr exon 16 | TAGACAGGACTTCAACCCTCAATCA |
| SEQ ID NO: 508 | cftr exon 17A | GAGTATCGGACATTCACTGTCATACC |
| SEQ ID NO: 509 | cftr exon 17B | AAGGTAACAGCAATGAAGAAGATGACAAA |
| SEQ ID NO: 510 | cftr exon 18 | TAATGACAGATACACAGTGACCCTCAA |
| SEQ ID NO: 511 | cftr exon 19 | GCTTCAGGCTACTGGGATTCAC |
| SEQ ID NO: 512 | cftr exon 19-1 | GTCATCTTTCTTCACGTGTGAATTCTCAA |
| SEQ ID NO: 513 | cftr exon 19-2 | GCTTCAGGCTACTGGGATTCAC |
| SEQ ID NO: 514 | cftr exon 20 | TTCTGGCTAAGTCCTTTTGCTCAC |
| SEQ ID NO: 515 | cftr exon 21 | CATTTCAGTTAGCAGCCTTACCTCA |
| SEQ ID NO: 516 | cftr 3849+10kb | TCCTCCCTGAGAATGTTGGATCAA |
| SEQ ID NO: 517 | Vs1 Int std F | TGATGGTGGTATGTTTTCAGGCTAGA |
| SEQ ID NO: 518 | Vs1 Int std R | GTTCTCCCCTGTCCCAGTTTTAAC |

## Fig. 6

**A**

| Mutation | Sample | IC ALLELE | MUT ALLELE | FOZ Ratio |
|---|---|---|---|---|
| 2789+5G>A | 26mix | 3.94 | 4.69 | 1.19 |
| R1162X | 29 | 3.42 | 2.18 | 0.62 |
| R347P | 15 | 3.38 | 4.60 | 1.36 |
| G85E | 21 | 3.62 | 2.55 | 0.70 |
| R560T | 9 | 3.30 | 2.47 | 0.75 |
| delI507 | 1 | 3.16 | 1.98 | 0.63 |
| 1898+1G>A | 111 A2/8 | 6.23 | 2.84 | 0.46 |
| R117H | 30 | 3.46 | 1.87 | 0.54 |
| delF508 homo MT | 3 | 3.44 | 1.14 | 0.33 |
| WT gDNA | 03-243 | 3.58 | 1.06 | 0.30 |

| Mutation | Sample | IC ALLELE | MUT ALLELE | FOZ Ratio |
|---|---|---|---|---|
| 2184delA plasmid/internal control syn. Target | plasmid/syn. Target | 4.67 | 3.65 | 0.78 |

**B**

| Mutation | Sample | IC ALLELE | MUT ALLELE | FOZ Ratio |
|---|---|---|---|---|
| A455E | 8 | 3.26 | 2.88 | 0.88 |
| 3659delC | 14 | 3.38 | 2.36 | 0.68 |
| N1303K | 16 | 3.92 | 2.11 | 0.54 |
| 3120+1G>A | 6 | 3.84 | 2.45 | 0.64 |
| G551D | 20 | 3.44 | 2.04 | 0.59 |
| WT gDNA | 03-243 | 3.74 | 1.00 | 0.27 |

| Mutation | Sample | IC ALLELE | MUT ALLELE | FOZ Ratio |
|---|---|---|---|---|
| I148T/Internal control | syn. target | 4.35 | 5.08 | 1.17 |
| 1078delT/Internal control | syn. target | 4.44 | 4.97 | 1.12 |

**C**

| Mutation | Sample | IC ALLELE | MUT ALLELE | FOZ Ratio |
|---|---|---|---|---|
| 711+1G>T | 2 | 3.95 | 2.82 | 0.71 |
| W1282X | 19 | 4.44 | 2.16 | 0.49 |
| 1717-1G>A | 28 | 4.87 | 2.19 | 0.45 |
| 3849+10kbC>T | 5 | 3.82 | 2.48 | 0.65 |
| WT gDNA | 03-243 | 4.67 | 1.10 | 0.24 |

**D**

| Mutation | Sample | IC ALLELE | MUT ALLELE | FOZ Ratio |
|---|---|---|---|---|
| 621+1G>T | 11 | 4.23 | 2.05 | 0.49 |
| G542X | 18 | 3.40 | 2.83 | 0.81 |
| R553X | 7 | 4.53 | 3.27 | 0.72 |
| R334W | 22 | 3.72 | 2.79 | 0.75 |
| WT gDNA | 03-243 | 4.18 | 1.14 | 0.27 |

FIGURE 7

## All Sample Extraction Methods

Bivariate Fit of FFOZ By RFOZ

FFOZ

10—

0—

0    RFOZ    10

G85E

Legend
Red = synthetic Mut
Blue = synthetic WT
Green = HET
Black = WT or NTB

EP 1 567 540 B1

## All Sample Extraction Methods

Bivariate Fit of FFOZ By RFOZ

# A455E

Legend
Red = synthetic Mut
Blue = synthetic WT
Green = HET
Black = WT or NTB

EP 1 567 540 B1

# All Sample Extraction Methods

Bivariate Fit of FFOZ By RFOZ

## 3659 del C

### Legend

Red = synthetic Mut
Blue = synthetic WT
Green = HET
Black = WT or NTB

# Figure 8

## Analysis of Characterized Samples _ Range Determination

Avg's _Ratio - "9T-IC" / "7T-IC"_ +/- 3 Stdev's

y-axis: 10.00, 9.00, 8.00, 7.00, 6.00, 5.00, 4.00, 3.00, 2.00, 1.00, 0.00

5t/7t - 7t/7t (n=38)    7t/9t (n=35)    5t/9t - 9t/9t (n=13)

Figure 9A

A455E, 20plex amplified genomics (2ng, 20 cycles), 40 min read

fam
red

fold over zero

delta F508, 20plex amplified genomics (2ng, 20 cycles), 40 min read

fam
red

fold over zero

# Figure 9B

**R117H, 2ul amp. punch in Inv rxn, 17 cycles**

EP 1 567 540 B1

Figure 9C

## Figure 9D

FIGURE 10

## REFERENCES CITED IN THE DESCRIPTION

**Patent documents cited in the description**

- US 6001588 A
- US 5407496 A
- US 5981178 A
- US 5776677 A
- WO 0121833 A
- EP 0677900 B1
- WO 0244994 A
- US 6291187 B
- US 6323009 B
- WO 0188190 A
- WO 0200934 A
- US 5198543 A
- US 5001050 A
- US 6251639 B
- US 6210884 B
- US 6183960 B
- US 6235502 B
- US 6410278 B
- US 32103902 A
- US 60511955 A
- US 6117634 A
- US 6197557 A
- WO 9839485 A
- US 6110684 A, Variagenics
- US 5958692 A
- US 5851770 A
- US 5849481 A, Chiron
- US 5710264 A
- US 5124246 A
- US 5624802 A
- US 5409818 A
- US 6150097 A
- US 6248229 B
- US 6221583 B
- US 6013170 A
- US 6063573 A
- US 5403711 A
- US 5011769 A
- US 5660988 A

- US 6121001 A
- US 6110677 A
- US 5914230 A
- US 5882867 A
- US 5792614 A
- US 5288609 A
- US 5614402 A
- US 5795763 A
- US 5843669 A
- US 6090 A
- WO 9823774 A
- WO 02070755 A2
- WO 0190337 A2
- US 6001983 A, S. Benner
- US 5846717 A
- US 5985557 A
- US 5994069 A
- US 6001567 A
- US 6090543 A
- US 6348314 B
- US 6458535 B
- WO 9727214 A
- WO 9842873 A
- US 6348314 A
- US 6458535 A
- US 6194149 B
- WO 9850403 A
- WO 0198537 A
- US 6258568 B
- US 5902724 A
- WO 9967641 A
- WO 0039587 A
- US 6514700 B
- US 6627400 B
- US 5952174 A
- US 5919626 A
- US 321039 A
- US 51195503 P

**Non-patent literature cited in the description**

- **Heim et al.** *Genetics in Medicine,* 2001, vol. 3 (3), 168-176
- **Raman et al.** *Pediatrics,* 2002, vol. 109 (1), E13
- **Dumur et al.** *Hum Genet,* 1996, vol. 97, 7-10

- **Grody, Cutting et al.** *Genetics in Medicine,* 2001, vol. 3 (2), 149-154
- **Grody ; Desnick.** *Genetics in Medicine,* 2001, vol. 3 (2), 87-90
- **Rosenstein ; Cutting.** *Journal of Pediatrics,* 1998, vol. 132 (4), 589-595

- **LeGrys.** *Laboratory Medicine,* 2002, vol. 33 (1), 55-57
- **Wilson et al.** *Journal of Pediatrics,* 1998, vol. 132 (4), 596-599
- **Gregg et al.** *Pediatrics,* 1997, vol. 99 (6), 819-824
- **Rock et al.** *Pediatrics,* 1990, vol. 85 (6), 1001-1007
- **Kerem et al.** *Science,* 1989, vol. 245, 1073-1080
- **Riordan et al.** *Science,* 1989, vol. 245, 1066-1073
- **Rommens et al.** *Science,* 1989, vol. 245, 1059-1065
- **Bobadilla et al.** *Human Mutation,* 2002, vol. 19, 575-606
- **Noone ; Knowles.** *Respiratory Research,* 2001, vol. 2 (6), 328-332
- **Rohlfs et al.** *Genetics in Medicine,* 2002, vol. 4 (5), 319-323
- **Kiesewetter et al.** *Nature Genetics,* 1993, vol. 5 (3), 274-278
- **Rohlfs, EM. et al.** *Genetics in Medicine,* 2002, vol. 4, 319-323
- Polymorphism identification and quantitative detection of genomic DNA by invasive cleavage of oligonucleotide probes. **LYAMICHEV V et al.** NATURE BIOTECHNOLOGY. NATURE PUBLISHING GROUP, March 1999, vol. 17, 292-296
- **Makowski, G.S. et al.** *Ann. Clin. Lab. Sci.,* 2003, vol. 33, 243-250
- **Barnay.** *Proc. Natl. Acad. Sci USA,* 1991, vol. 88, 189-93
- **Marmur ; Lane.** *Proc. Natl. Acad. Sci. USA,* 1960, vol. 46, 453
- **Doty et al.** *Proc. Natl. Acad. Sci. USA,* 1960, vol. 46, 461
- **Anderson ; Young.** *Quantitative Filter Hybridization, in Nucleic Acid Hybridization,* 1985
- **Allawi, H.T. ; SantaLucia, J., Jr.** Thermodynamics and NMR of internal G.T mismatches in DNA. *Biochemistry,* 1997, vol. 36, 10581-94
- **B.A. Schweitzer ; E.T. Kool.** *J. Org. Chem.,* 1994, vol. 59, 7238-7242
- **B.A. Schweitzer ; E.T. Kool.** *J. Am. Chem. Soc.,* 1995, vol. 117, 1863-1872
- **P. Kong et al.** *Nucleic Acids Res.,* 1989, vol. 17, 10373-10383
- **P. Kong et al.** *Nucleic Acids Res.,* 1992, vol. 20, 5149-5152
- **Lyamichev et al.** *Nat. Biotech.,* 1999, vol. 17, 292
- **Hall et al.** *PNAS, USA,* 2000, vol. 97, 8272
- **Makowski et al.** *Ann. Clin. Lab. Sci.,* 2003, vol. 33, 243-250
- **Luis P. Reynaldo ; Alexander V. Vologodskii ; Bruce P. Neri ; Victor I. Lyamichev.** *J. Mol. Biol.,* 2000, vol. 97, 511-520
- **Richards, C.S. et al.** *Genetics in Medicine,* 2002, vol. 4, 379-391
- **Strom, C.M. et al.** *Genetics in Medicine,* 2002, vol. 4, 289-296
- **Chamberlain et al.** *Nucleic Acids Res.,* 1988, vol. 16, 11141
- **Elnifro et al.** *Clinical Microbiology Reviews,* 2000, vol. 13, 559
- **Hidding ; Schmitt.** *Forensic Sci. Int.,* 2000, vol. 113, 47
- **Bauer et al.** *Int. J. Legal Med.,* 2002, vol. 116, 39
- **Ouhibi et al.** *Curr Womens Health Rep.,* 2001, vol. 1, 138
- **Rudi et al.** *Int J Food Microbiology,* 2002, vol. 78, 171
- **Zarlenga ; Higgins.** *Vet Parasitol,* 2001, vol. 101, 215
- **Kwok.** *Molecular Medicine Today,* 1999, vol. 5, 538-5435
- **Kwok.** *Pharmacogenomics,* 2000, vol. 1, 231
- **Risch ; Merikangas.** *Science,* 1996, vol. 273, 1516
- **Cargill et al.** *Nature Genetics,* 1999, vol. 22, 231
- **Halushka et al.** *Nature Genetics,* 1999, vol. 22, 239
- **Hagmann.** *Science,* 1999, vol. 285, 21
- **Polz ; Cavanaugh.** *Applied and Environmental Microbiology,* 1998, vol. 64, 3724
- **Walsh et al.** *PCR Methods and Applications,* 1992, vol. 1, 241
- **Lindblad-Toh et al.** *Nature Genet.,* 2000, vol. 4, 381
- **Allawi ; SantaLucia.** *Biochemistry,* 1997, vol. 36, 10581
- **SantaLucia.** *Proc Natl Acad Sci U S A,* 1998, vol. 95, 1460